# EUROPEAN PATENT SPECIFICATION

(11) **EP 0 232 966 B1**
(45) Date of publication and mention of the grant of the patent: **08.10.1997**
(21) Application number: 87300193.7
(22) Date of filing: 09.01.1987
(51) Int. Cl.: C07D 499/00

(54) **Process for the preparation of penem compounds and intermediates for this preparation**
Verfahren zur Herstellung von Penem Derivaten und Zwischenprodukte zu dieser Herstellung
Procédé de préparation de dérivés de pénème et intermédiaires pour cette préparation

(30) Priority: 17.01.1986 GB 8601119; 18.10.1986 GB 8625017
(43) Date of publication of application: 19.08.1987
(73) Proprietor: BEECHAM GROUP PLC, Brentford, Middlesex TW8 9EP (GB)
(72) Inventor: Coulton, Steven, Third Avenue Harlow Essex CM19 5AW (GB); Harbridge, John Barry, Third Avenue Harlow Essex CM19 5AW (GB); Osborne, Neal Frederick, Third Avenue Harlow Essex CM19 5AW (GB); Walker, Graham, Third Avenue Harlow Essex CM19 5AW (GB)
(74) Representative: Walker, Ralph Francis, Dr.

(56) References cited:
- EP-A- 0 003 960
- EP-A- 0 150 781
- EP-A- 0 188 247
- EP-A- 0 210 814
- DE-A- 2 343 497
- GB-A- 1 368 074
- GB-A- 2 144 743
- JOURNAL OF THE AMERICAN CHEMICAL SOCIETY, vol. 107, no. 22, 30th October 1985, pages 6398-6399, American Chemical Society; M. ALPEGIANI et al.: "Silver-assisted 1,2-cleavage of penicillins. A three-step synthesis of optically active penems"
- JOURNAL OF THE CHEMICAL SOCIETY, vol. 4, no. 12, 1979, pages 3175-3184; P.M. DENERLEY et al.: "Alkylation of penicillanates: Aspects of the chemistry of penicillanate sulphonium salts"

## Description

This invention relates to a process for preparing 6-(substituted methylene)-penems, and to intermediates in this process.

EP 0188247A discloses a process for preparing penem compounds from penam compounds.

EP 0 041 768 A, EP 0 120 613 A, EP 0 150 781, EP 0 154 132 A, EP 0210814 A, EP 0210065, WO 87/00525, EP 0 003 960 B1, GB 2 042 514 A, GB 2 042 515 A, EP 0 087 792 A, EP 0 115 308 A, GB 2 124 614 B, EP 0 150 984 A, EP 0087792 and EP 0115308 disclose 6-(substituted methylene)-penems and intermediates in their preparation.

The present invention now provides a process for the preparation of a compound of the formula (X): 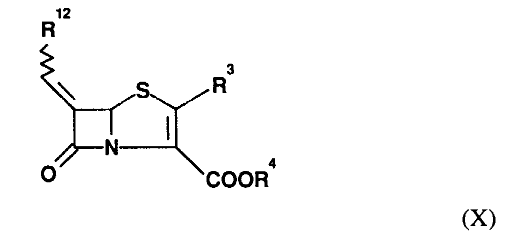 wherein:
R³ denotes a hydrogen atom or a group -R^{9A} or -SR^{9A} where R^{9A} denotes an unsubstituted or substituted (C₁₋₁₀) hydrocarbon or heterocyclyl group, or a group -OR^{9B} or -R^{9C} in which R^{9B} denotes an unsubstititued or substituted phenyl, naphthyl, thienyl, pyridyl, quinolyl or isoquinolyl, and R^{9C} denotes a nitrogen-containing heterocyclyl ring bonded through a ring-nitrogen atom;
R⁴ denotes a hydrogen atom, a carboxy-salt-forming ion, or a carboxy-ester-forming group;
R¹² denotes a hydrogen atom, an unsubstituted or substituted (C₁₋₁₀) hydrocarbon group, or an unsubstituted or substituted heterocyclyl group;
wherein the term "heterocyclyl" denotes an optionally substituted, 5 or 6 membered hetero-aromatic ring, or a fused bicyclic hetero-aromatic ring system having 5 or 6 atoms in each ring, or a non-aromatic heterocyclic ring having 3 to 7 ring atoms, and from 1 to 3 of the ring atoms being heteroatoms selected from nitrogen, oxygen and sulphur; characterised in that (a) a penicillin or 6-aminopenicillanic acid is converted into a compound of formula (IC): 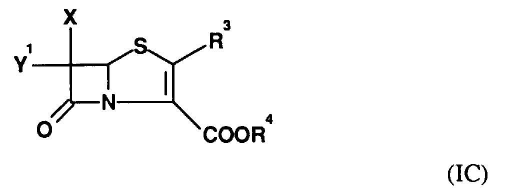 wherein R³ and R⁴ are as defined in formula (X), X denotes a halogen atom, and Y¹ denotes a hydrogen atom or a halogen atom, (b) the compound of formula (IC) is reacted with a compound of formula (XXV):

R¹² - CHO (XXV)

wherein R¹² is as defined above, to form a compound of formula (IB) 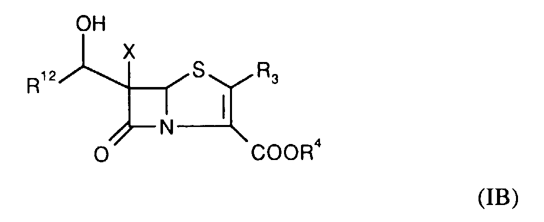 wherein R³, R⁴, R¹² and X are as defined above, and (c) the compound of formula (IB) is subjected to reductive elimination to eliminate X and hydroxy. The term 'hydrocarbon' as used herein includes groups having up to 18 carbon atoms, suitably up to 10 carbon atoms, conveniently up to 6 carbon atoms. Suitable hydrocarbon groups include (C₁₋₆)alkyl, (C₂₋₆)alkenyl, (C₂₋₆)alkynyl, (C₃₋₇)cycloalkyl, aryl,(C₃₋₇)cycloalkyl(C₁₋₆)alkyl, aryl(C₁₋₆)alkyl, (C₁₋₆)alkyl(C₃₋₇)cycloalkyl, and (C₁₋₆)alkylaryl.

Examples of suitable optional substituents for the above-mentioned hydrocarbon groups include, heterocylyl, amino, (C₁₋₆)alkanoylamino, (mono, di, or tri)-(C₁₋₆)alkylamino, hydroxy, (C₁₋₆)alkoxy, (C₁₋₆)alkoxy(C₁₋₆)alkoxy, aryloxy, mercapto, C₁₋₆)alkylthio, heterocyclylthio, arylthio, sulphamoyl, carbamoyl, substituted carbamoyl, amidino, guanidino, nitro, chloro, bromo, fluoro, carboxy and salts and esters thereof, (C₁₋₆)alkanoyloxy, arylcarbonyloxy, heterocyclylcarbonyloxy, acyl, and acyloxy groups.

Any alkyl group or moiety referred to herein may be straight or branched, unsubstituted or substituted, and may contain, for example, up to 12 carbon atoms, suitably up to 6 carbon atoms. In particular, the alkyl group or moiety may be an unsubstituted or substituted methyl, ethyl, n-propyl, iso-propyl, n-butyl, sec-butyl, isobutyl or tert-butyl group. Examples of suitable optional substitutents for any such alkyl group or moiety include the above-listed substitutents for hydrocarbon groups, and also the above-listed non-alkyl hydrocarbon groups, for example cycloalkyl and aryl groups.

The term 'aryl' as used herein includes phenyl and naphthyl, which may be unsubstituted or substituted by up to five, preferably up to three, groups selected from halogen, (C₁₋₆)alkyl, phenyl, (C₁₋₆)alkoxy, halo(C₁₋₆)alkyl, hydroxy, amino, nitro, carboxy, (C₁₋₆)alkoxycarbonyl, (C₁₋₆)alkoxycarbonyl(C₁₋₆)alkyl, (C₁₋₆)alkylcarbonyloxy, and (C₁₋₆)alkylcarbonyl groups, and also the other above-listed substituents for hydrocarbon groups, and the other above-listed non-aryl hydrocarbon groups.

Heterocyclyl groups as discussed herein may be unsubstituted or substituted by up to three groups. Suitable substituent groups include the above-mentioned hydrocarbon groups, as well as the optional substituents listed hereinbefore as suitable substituents for hydrocarbon groups.

The term 'halogen' as used herein includes in particular bromine, chlorine, iodine, and fluorine.

A halogen atom denoted by X, Y or Z may be a bromine, chlorine, iodine, or fluorine atom, advantageously a chlorine or iodine atom, and especially a bromine atom.

In one embodiment of the process of this invention a 6-halo or 6,6-dihalopenicillanic acid derivative of formula (IX): 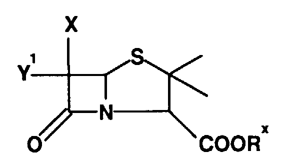 wherein X and Y¹ are as defined above, and R^{x} is a carboxy protecting group is converted into a compound of formula (IC), which is then converted into a compound of formula (X).

In a first group of compounds of the general formula IC, Y¹ denotes a halogen atom or, especially, a hydrogen atom.

Examples of such compounds include, in particular, those compounds in which Y¹ denotes a hydrogen atom and X denotes a bromine atom, in which case the bromine atom may advantageously be in the 6a-position (also referred to as the 6S-position), but may alternatively be in the 6b-position (also referred to as the 6R-position). Further examples of such compounds include those in which Y¹ denotes a hydrogen atom and X denotes a chlorine atom, which may advantageously be in the 6a-position, and also those in which both X and Y¹ denote bromine atoms.

A hydrocarbon group denoted by R¹² may suitably be an unsubstituted or substituted (C₁₋₁₀)hydrocarbon group, preferably an unsubstituted or substituted (C₁₋₆)alkyl group or an unsubstituted or substituted phenyl group, and especially an unsubstituted or substituted methyl, ethyl or phenyl group. Such groups are more fully described as the groups R¹ and R² in EP 0 041 768 A.

A heterocyclyl group R¹² may suitably be an aromatic heterocyclyl group, and in such cases the requirement for aromaticity will, of course, affect the choice of ring size, type and number of hetero-atoms, and type and number of ring substituents in manner well known to a person skilled in the art (see, for example, M.J.Cook et al, 'Aromaticity of heterocycles', Advances in heterocyclic chemistry, Academic Press, 1974, 17, 255ff; and A.R.Katritzky and J.M.Lagowski, 'Protopic tautomerism of heteroaromatic compounds', ibid, 1, 311, 339; 2, 1, 27; Supplement 1).

A heterocyclyl group denoted by R¹² may suitably be an unsubstituted or substituted five-membered or six-membered aromatic heterocyclyl group comprising one or more ring hetero-atoms selected from oxygen, nitrogen and sulphur, the remaining ring atoms being carbon atoms. The heterocyclyl group is advantageously bonded to the remainder of the molecule through a ring carbon atom.

More particularly, a heterocyclyl group R¹² may be an unsubstituted or substituted five-membered aromatic heterocyclyl group bonded through a carbon atom thereof and having one ring hetero-atom selected from oxygen, nitrogen and sulphur, and optionally additionally having from one to three nitrogen atoms. Such five-membered groups may be as more particularly defined and described in EP 0 120 613 A (wherein X¹, R^{a} and R^{b} in formula (B) above correspond, respectively, to X, R⁴ and R⁵ as defined in EP 0 120 613 A). Alternatively, such five-membered groups may be five-membered hetero-aromatic groups of the type defined and described in EP 0 154 132 A.

Suitable five-membered aromatic heterocyclyl rings R¹² include furans, thiophenes, pyrroles, pyrazoles, imidazoles, triazoles, tetrazoles, thiazoles, isothiazoles, oxazoles, isoxazoles, thiadiazoles, and oxadiazoles, each of which may be unsubstituted or substituted. (It is to be understood that, where appropriate, all isomeric forms of the above-mentioned aromatic heterocyclyl rings are included).

Particularly suitable five-membered aromatic heterocyclyl rings R¹² include furans, oxazoles, isoxazoles, pyrazoles, and triazoles.

Examples of individual five-membered aromatic heterocyclyl groups R¹² include furyl, isothiazolyl, isoxazolyl, methylthiazolyl, methyloxazolyl, dimethyloxazolyl, methyl-1,2,3-thiadiazolyl, methyl-1,2,4-oxadiazolyl, N-methylpyrazolyl, N-methylimidazolyl, N-methyl-1,2,3-triazolyl, N-methyl-1,2,4-triazolyl, and N-methyltetrazolyl groups.

A heterocyclyl group R¹² may furthermore be an unsubstituted or substituted six-membered aromatic heterocyclyl group bonded through a carbon atom thereof and having from one to three nitrogen atoms as ring hetero-atoms, as more fully defined and described in EP 0 150 731 A.

Suitable six-membered aromatic heterocyclyl rings R¹² include pyridine, pyrazine, pyrimidine, pyridazine, and triazines, each of which may be unsubstituted or substituted. (It is to be understood that, where appropriate, all isomeric forms of the above-mentioned aromatic heterocyclyl rings are included).

Examples of individual six-membered aromatic heterocyclyl groups R¹² include 3-pyridyl, 4-pyridyl, methoxypyridyl, pyrazinyl, 4-pyrimidinyl, 3-pyridazinyl, and dimethyltriazinyl groups.

A heterocyclyl group R¹² may also suitably be an unsubstituted or substituted fused bicyclic hetero-aromatic group bonded through a carbon atom thereof and having five or six atoms in each ring, wherein the expression 'fused bicyclic hetero-aromatic group' means a group comprising two fused rings together having aromatic character at least one of which rings contains a non-carbon atom (i.e. a hetero-atom) as a ring member and at least one of which rings contains a carbon-bonded free valency by which the group is bonded to the remainder of the molecule, as more fully defined and described in WO 87/00525.

Each ring of a fused bicyclic hetero-aromatic group R¹² may contain 5 or 6 ring atoms (including ring junction atoms - also referred to as bridgehead atoms - constituting members of both rings) and the hetero-aromatic group may consist of two fused rings both of the same size or it may consist of one 5-membered ring fused to one 6-membered ring.

A fused bicyclic hetero-aromatic group R¹² may contain one or more hetero-atoms selected from nitrogen, oxygen and sulphur atoms. Provided that at least one hetero-atom is present in at least one of the rings(which may be a 5-membered ring or a 6-membered ring),it is not necessary for both rings to contain a hetero-atom: one ring may contain no hetero-atoms or both rings may each contain one or more hetero-atoms depending on the type of hetero-atoms. Also, the ring through which the hetero-aromatic group is bonded to the remainder of the molecule should contain at least one non-bridgehead carbon atom for attachment of the bond. The maximum possible number of each type of hetero-atom and the various possible combinations of two or more different hetero-atoms is dictated by the ring structure and the requirement for aromaticity and will be apparent to the chemist. The hetero-aromatic group may contain one bridgehead nitrogen atom at the ring fusion without losing its aromaticity.

Examples of individual fused bicyclic hetero-aromatic groups R¹² inclde benzo[b]furyl, indolyl, N-substituted indolyl, and benzotriazolyl groups.

A heterocyclyl group R¹² may furthermore suitably be an unsubstituted or substituted non-aromatic heterocyclyl group bonded through a carbon atom thereof, as more fully defined in EP 0 210 065.

A non-aromatic heterocyclyl group R¹² will have a minimum of three ring atoms, and may suitably have from three to seven ring atoms, advantageously from three to six ring atoms. At least one of the ring atoms, advantageously from one to three of the ring atoms, will be hetero-atoms selected from nitrogen, oxygen and sulphur, the remaining ring atoms being carbon atoms. At least one of the ring atoms, advantageously at least two of the ring atoms will be carbon atoms. The ring may be saturated or unsaturated, provided that the unsaturation does not impart aromaticity. The heterocyclyl group is bonded to the remainder of the molecule through a ring carbon atom.

In the case of non-aromatic heterocyclyl groups R¹² containing unsaturation, such unsaturation may in general be present as a C=C double bond, C=N double bond or N=N double bond. In the case of such groups containing a sulphur atom as a ring hetero-atom, the sulphur atom may be in fully reduced form or may carry one or two oxygen atoms such that the ring is in the form of a sulphoxide or sulphone. In the case of such groups containing a nitrogen atom, the nitrogen atom may be unsubstituted and attached to a ring double oond, or the nitrogen atom may be substituted by a hydrogen atom or an organic group, or the nitrogen atom may be substituted by an oxygen atom such that the ring nitrogen is in the form of an N-oxide. A substituted nitrogen atom may, for example, be denoted by -N(Rⁿ)-, in which Rⁿ denotes a hydrogen atom, an oxygen atom, or an organic group, including, for example, an unsubstituted or substituted hydrocarbon group (for example, alkyl or aryl), an acyl group, a substituted hydroxy group (for example, acyloxy or alkoxy), a substituted amino group (for example, acylamino), or a nitrogen-protecting group (which may optionally be removed at any convenient stage.)

Examples of individual non-aromatic heterocyclyl groups R¹² include oxiranyl, azetidinyl, pyrrolidinyl, (di or tetra)hydro-oxazolyl or -isoxazoyl, dioxolanyl, and (di or tetra)hydropyranyl groups.

A heterocyclyl group R¹² may be unsubstituted or may be substituted by one or more substituents, each of which may be carried on a ring carbon atom or a ring nitrogen atom, provided of course that, where appropriate, the aromaticity of the ring is not destroyed.

Examples of suitable substituents which may be present in a heterocyclyl group R¹² include (C₁₋₆)alkanoyl, (C₁₋₆)alkanoyloxy, heterocyclyl, amino, (C₁₋₆)alkanoylamino, (mono or di)-(C₁₋₆)alkylamino, hydroxy, (C₁₋₆)alkoxy, sulpho, mercapto, (C₁₋₆)alkylthio, (C₁₋₆)alkylsulphinyl, (C₁₋₆)alkylsulphonyl, heterocyclylthio, arylthio, sulphamoyl, carbamoyl, amidino, guanidino, hydroxy, cyano, nitro, halogen, carboxy, carboxy salts, carboxy esters, arylcarbonyl, and heterocyclylcarbonyl groups, and also unsubstituted or substituted (C₁₋₆)alkyl, (C₂₋₆)alkenyl, (C₂₋₆)alkynyl, aryl, and aryl(C₁₋₆)alkyl groups.

Examples of suitable optional substituents for the above-mentioned (C₁₋₆)alkyl, (C₂₋₆)alkenyl, (C₂₋₆)alkynyl, aryl and aryl(C₁₋₆)alkyl substitutents include (C₁₋₆)alkanoyl, (C₁₋₆)alkanoyloxy, heterocyclyl, amino, (C₁₋₆)alkanoylamino, (mono or di)-(C₁₋₆)alkylamino, hydroxy, (C₁₋₆)alkylsulphinyl, (C₁₋₆)alkylsulphonyl, heterocyclylthio, arylthio, sulphamoyl, carbamoyl, amidino, guanidino, nitro, halogen, carboxy, carboxy salts, carboxy esters, arylcarbonyl and heterocyclylcarbonyl groups.

In addition to or instead of the above-mentioned monovalent substituents for the heterocyclyl group R¹², a non-aromatic heterocyclyl group R¹² or, in some cases a fused bicyclic hetero-aromatic group R¹², may optionally contain one or more divalent substituents, such as those of the formulae =O, =S, =N(Rⁿ), and =CR⁰¹R⁰², in which Rⁿ is defined as above, and each of R⁰¹ and R⁰², which may be identical or different, denotes a hydrogen atom, an unsubstituted or substituted hydrocarbon group, or an unsubstituted or substituted heterocyclyl group.

Moreover, a non-aromatic heterocyclyl group R¹² may be disubstituted on a single ring carbon atom by two of the above-mentioned monovalent substituents, which may be identical or different. Alternatively, it may be disubstituted on a single carbon atom by a ring in such a manner as to form a spirocyclic ring system. It may also contain a monovalent substituent (for example those listed above) on the carbon atom through which the group is bonded to the remainder of the molecule. Such a substituent is advantageously a small substituent, for example a methyl group.

When the group R¹² includes a carboxy salt or carboxy ester substituent, that substituent may a pharmaceutically acceptable salt or pharmaceutically acceptable ester, but, in the case of intermediates, such a substituent need not be pharmaceutically acceptable.

When the heterocyclyl group R¹² is or includes a basic moiety, the compound according to the invention may exist in zwitterionic form.

R³ may denote a hydrogen atom or a group of formula -R^{9A} or -SR^{9A} where R^{9A} denotes an unsubstituted or substituted (C₁₋₁₀)hydrocarbon or heterocyclyl group. Preferred groups R^{9A} include (C₁₋₁₀)alkyl and substituted (C₁₋₁₀)alkyl groups, wherein the substituent may be hydroxy, (C₁₋₆)alkoxy, (C₁₋₆)alkanoyloxy, halogen, mercapto, (C₁₋₆)alkylthio, heterocyclylthio, amino, (mono or di)-(C₁₋₆)alkylamino, (C₁₋₆)alkanoylamino, carboxy, or (C₁₋₆)alkoxycarbonyl.

Alternatively, R³ may uenote a yroup or the formula -OR^{9B} or -R^{9C}, in which R^{9B} denotes an unsubstituted or substituted phenyi, naphthyl, thienyl, pyridyl, quinolyl or isoquinolyl group, and R^{9C} denotes a nitrogen-containing heterocyclyl ring bonded through a ring-nitrogen atom. Examples of groups denoted oy R^{9B} and R^{9C}, and also examples of suitable substituents for the aryl or heterocyclyl rings denoted by R^{9B} are given in GB 2 102 798 A (Hoechst), EP 0 099 059 A (Hoechst) and EP 0 148 128 A.

Preferably R³ denotes a hydrogen atom or a group -R^{9A} or -R^{9C} as defined above.

Examples of organic groups R³ include methyl, ethyl, propyl, methylthio, ethylthio. methylsulphinyl, ethylsulphinyl, hydroxymethyl, methoxymethyl, ethoxymethyl, acetoxymethyl, (1 or 2)-acetoxyethyl, aminomethyl, 2-aminoethyl, acetamidomethyl, 2-acetamidoethyl, carboxymethyl, 2-hydroxyethylthio, methoxymethylthio, 2-methoxyethylthio, acetoxymethylthio, 2-aminoethylthio, acetamidomethylthio, 2-acetamidoetnylthio, acetamidomethylthio, 2-carboxyethylthio, aryl (especially phenyl), arylthio (especially phenylthio), furyl, pyrazolyl, dimethylpyrazolyl, imidazolyl, pyridyl, pyrimidyl, isoxazolyl, pyrimidylthio, tetrazolylthio, and pyridylthio groups.

In particular, R³ denotes a hydrogen atom.

The -COOR⁴ substituent in the 3-position of the penem of the general formulae (X), IB and (IC) may be a free carboxy substituent, a carboxy salt or a carboxy ester. The salts and esters may be pharmaceutically acceptable but, in the case of intermediate compounds, need not be pharmaceutically acceptable and may suitably be carboxy-protecting groups.

Pharmaceutically acceptable esters are suitably pharmaceutically acceptable in vivo hydrolysable esters (also referred to as 'metabolisable esters'), namely those esters which hydrolyse in the human body to produce the parent acid or its salt. Such esters may be identified by oral or intravenous administration to a test animal, and subsequent examination of the test animal's body fluids for the presence of the parent acid or a salt thereof.

Suitable in vivo hydrolysable ester groups include those of formulae (a), (b) and (c):

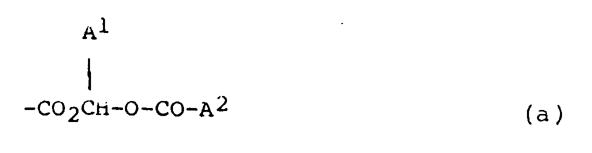

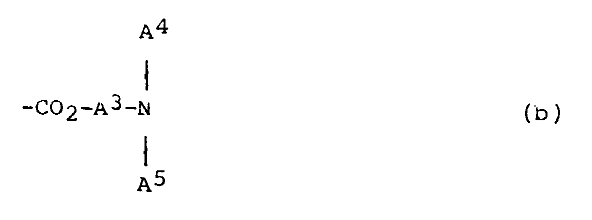

-CO₂CH₂-OA⁶ (c)

in which
A¹ denotes hydrogen, methyl, or phenyl;
A² denotes (C₁₋₆)alkyl, (C₁₋₆)alkoxy or phenyl; or
A¹ and A² together denote 1,2-phenylene, which may beunsubstituted or substituted by one or two methoxy groups;
A³ denotes (C₁₋₆)alkylene, which may be unsubstituted or substituted by a methyl or ethyl group;
each of A⁴ and A⁵ which may be identical or different, denotes (C₁₋₆)alkyl; and
A⁶ denotes (C₁₋₆)alkyl.

Examples of suitable in vivo hydrolysable ester groups include acetoxymethyl, pivaloyloxymethyl, α-acetoxyethyl, α-acetoxybenzyl, α-pivaloyloxyethyl, ethoxycarbonyloxymethyl, α-ethoxycarbonyloxyethyl, dimethylaminomethyl, diethylaminomethyl, phthalidyl and dimethoxyphthalidyl groups.

Suitable pharmaceutically acceptable salts of the 3-carboxylic acid group of the compound of formula (X) include metal salts, e.g. aluminium salts, alkali metal salts (e.g. sodium or potassium salts), alkaline earth metal salts (e.g. calcium or magnesium salts), ammonium salts, and substituted ammonium salts, for example those with lower alkylamines (e.g.triethylamine), hydroxy-lower alkylamines (e.g. 2-hydroxyethylamine), di(2-hydroxyethyl)amine or tri(2-hydroxyethyl)amine), cycloalkylamines (e.g. dicyclohexylamine), or with procaine, and also dibenzylamine, N,N-dibenzylethylenediamine, 1-ephenamine, N-ethylpiperidine, NN-benzyl-β-phenethylamine, dehydroabietylamine, N,N'-bishydroabietylethylene-diamine, -diamine, oases of the pyridine type (e.g. pyridine, collidine and quinoline), and other amines which have been or can be used to form salts with penicillins.

A carboxy-protecting group R⁴ is suitably a group that can readily be removed at a subsequent process stage.

Examples of suitable carboxy-protecting salt-forming groups R⁴ include inorganic salts, for example alkali metal atoms (e.g. lithium and sodium) and other metal atoms, and also organic salts, for example tertiary amino groups (e.g. tri-lower-alkylamino, N-ethylpiperadino, and dimethylipiperazino groups). A preferred carboxy-protecting salt-forming group R⁴ is the triethylamino group.

A carboxy-protecting ester-forming group R⁴ is advantageously one that can be removed under conventional conditions, such as hydrolysis or hydrogenolysis. Examples of suitable carboxy-protecting ester-forming groups R⁴ include benzyl, p-methoxybenzyl, 2,4,6-trimethylbenzyl, 3,5-di-t-butyl-4-hydroxy-benzyl, benzoylmethyl, p-nitrobenzyl, 4-pyridylmethyl, 2,2,2-trichloroethyl, 2,2,2-tribromoethyl, allyl, acetonyl, t-butyl, t-amyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, p-toluene-sulphonylethyl, 1-phenoxyethyl, and methoxymethyl groups, and also silyl, stannyl and phosphorus-containing groups, and oxime radicals of formula -N=CHR^{o} in which R^{o} denotes aryl or heterocyclyl, as well as the above-mentioned in vivo hydrolysable ester groups.

When desired, the free carboxyl group may be regenerated from any of the above esters by usual methods appropriate to the particular R⁴ group, for example, by hydrolysis (which may, for example, be acid-catalysed, base-catalysed, enzymically-catalysed or Lewis-acid-medicated), or by reduction (for example, by hydrogenation).

The removal of the ester moiety must of course be carried out under conditions in which the groups on the rest of the molecule are stable. In particular, in the case of compounds of the general formula I, the ester removal technique used should, of course, be such that it will not result in removal of the C-6 halogen atom(s).

The compounds of the general formula IC may exist in two optically active forms at the 5-position and it is to be understood that both such forms as well as racemic mixtures thereof are embraced by the present invention. Advantageously, the compounds are of the formula IA: 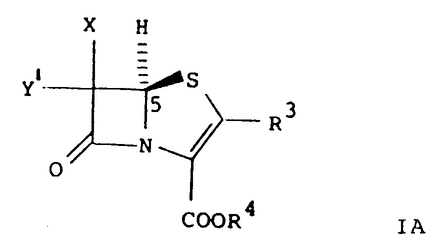 in which X, Y¹, R³ and R⁴ are defined as above.

It is to be understood that formulae given in this specification are not intended to indicate any particular geometric or optical orientation, except where the context obviously requires otherwise. Specifically, ordinary solid lines are not used to indicate orientation.

The compounds of the general formula IC may be prepared by a variety of routes using Known reaction steps.

Such routes will, in general, proceed through an azetidinone intermediate of the general formula IV: 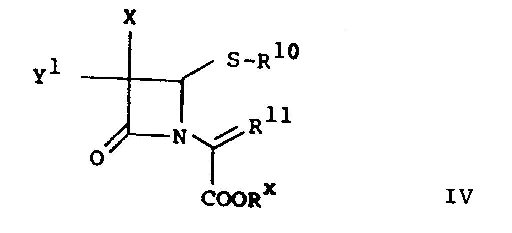 in which
X denotes a halogen atom;
Y¹ denotes a hydrogen atom or a halogen atom;
R^{x} denotes a carboxy-protecting group;
R¹⁰ denotes a hydrogen atom, an ammonium or substituted ammonium ion, an organothio group (especially a heterocyclylthio (preferably aromatic) group), a triphenylmethyl group (also referred to as a trityl group), or a group of one of the formulae

   -M/q V

   or 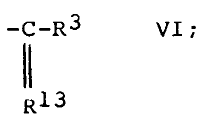
R¹¹ denotes a 1-methyl-ethylidene group (=C(CH₃)₂), a phosphoranylidene group (for example, a triarylphosphoranylidene group or a trialkoxyphosphoranylidene group), or an oxygen atom;
R¹³ denotes an oxygen or sulphur atom, or a group of the formula

   =CH-R¹⁴ VII;
R¹⁴ denotes a hydrogen atom or an organic group, including an organosilicon group (e.g. an organosilyl group, especially a trialkylsilyl group), an organometallic group (e.g. an organotin group, especially an alkyltin group), an unsubstituted or substituted hydrocarbon group, an unsubstituted or substituted heterocyclyl group, an alkoxycarbonyl group, and the groups listed hereinbefore as possible substituents for hydrocarbon groups;
R³ is defined as above, with the proviso that if R¹³ denotes a sulphur atom, R³ denotes a group of the formula -SR^{9A} as defined above;
M denotes a metal (for example, silver or mercury) atom or ion of valency or ionic charge q; and
q denotes the valency or ionic charge of the metal atom or ion M.

In particular, the compounds of the general formula IC: 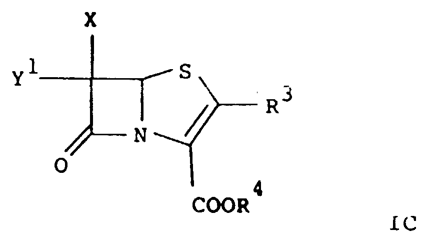 in which X, Y¹, R³ and R⁴ are defined as above,
may, for example, be prepared by cyclising an azetidinone intermediate of the general formula IVA 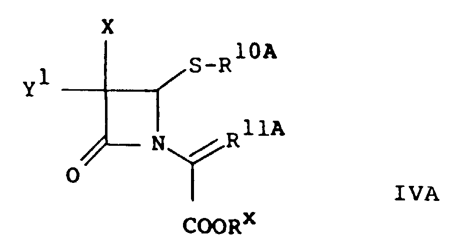 in which
R^{10A} denotes a group of the formula 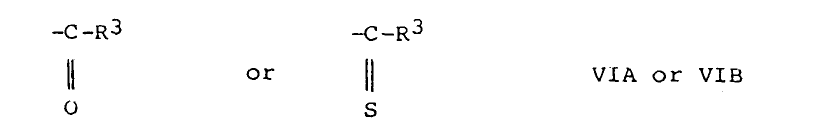
R^{11A} denotes a phosphoranylidene group or an oxygen atom; and
X, Y¹, R³ and R^{x} are defined as above.

The cyclisation reaction may generally be carried out in known manner, for example in solution in an organic solvent (e.g. ethyl acetate, toluene, or xylene) at a temperature of from -20°C to +150°C, optionally in the presence of a trivalent phosphorus compound (especially when R^{11A} is oxygen), as discussed in more detail in connection with Routes A to E below.

The compound of the general formula IVA may in general be prepared from other compounds of the general formula IV using known reaction steps, for example those reaction steps illustrated in Routes A to E below. In some cases, the compound of the general formula IVA may be formed in situ and may be cyclised without intermediate isolation or purification.

A compound of the general formula IC may be prepared by any of the routes outlined in the following reaction schemes, Routes A to E, all starting from a 6-halo- or 6,6-dihalo-penicillanic acid of the general formula VIII, which are known compounds: 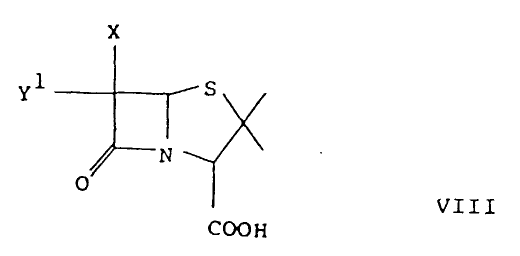 in which X and Y¹ are defined as above.

As a first step, the 3-carboxy group of the penicillanic acid of the general formula VIII may be protected to give a compound of the general formula IX: 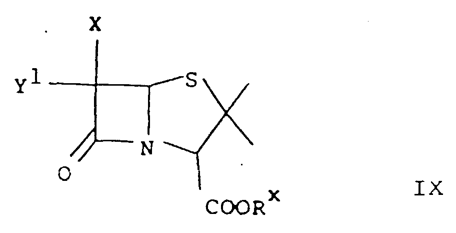 in which
X, Y¹ and R^{x} are defined as above.

According to the present invention, Route F illustrates the conversion of compounds of the general formula IC, into compounds of the general formula IB, and their subsequent conversion into compounds of the general formula X: 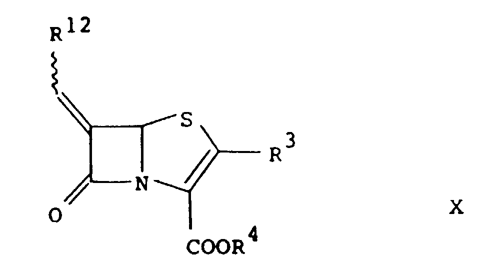 in which R³, R⁴ and R¹² are defined as above.

In the following reaction schemes, the symbols
R³, R¹², R¹⁴, R^{x}, M, X, Y¹, Z, and q are defined as above;
Hal denotes a halogen atom;
Het denotes an aromatic heterocyclyl group, for example a pyridyl or benzothiazoyl group;
Q denotes an acyl protecting group, for example a benzoyl or, especially, an acetyl group;
P^{o} denotes a phosphoranylidene group;
the letters and numbers (e.g. Al) denote the various reaction steps, corresponding to the various lettered reaction schemes, as discussed below; and
the roman numerals (with suffix letters in some cases) indicate the respective formulae.

The compounds of the formula 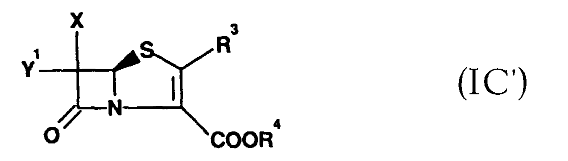 in which X and Y¹ are both bromine, R³ is hydrogen and R⁴ is hydrogen, ie 6, 6-dibromopenem-3-carboxylate, or a salt or ester thereof, is a novel compound and forms a further subject of the present invention.

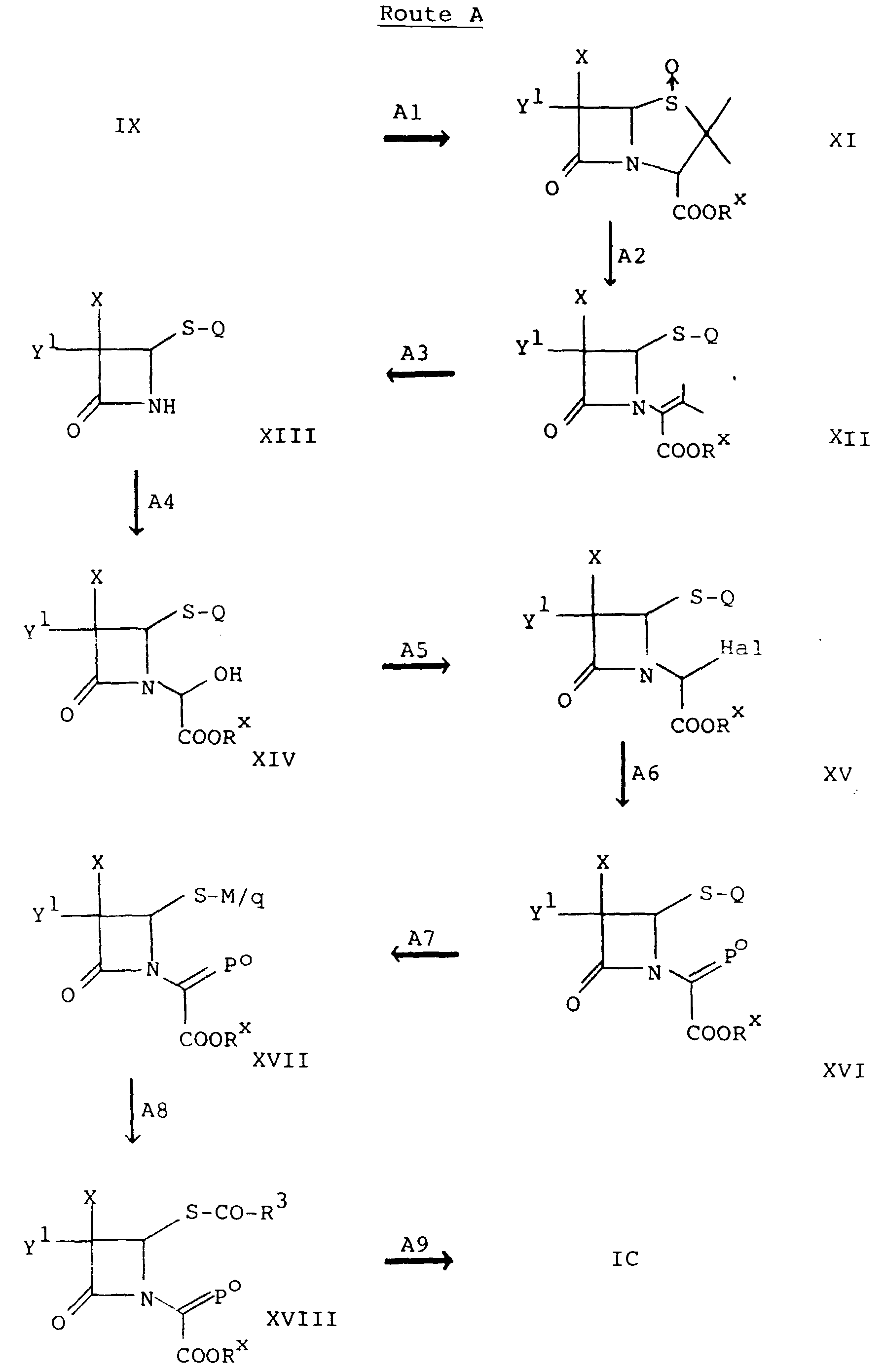

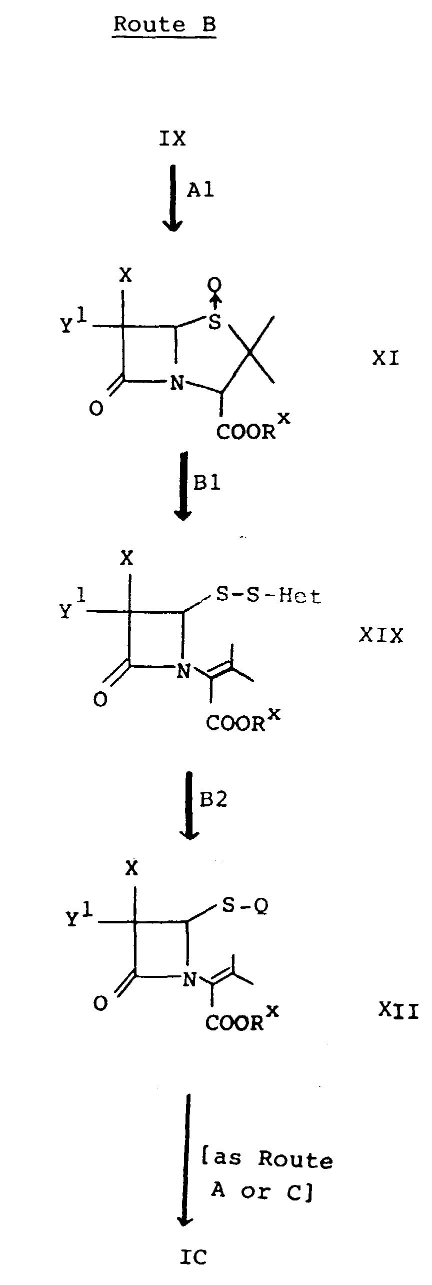

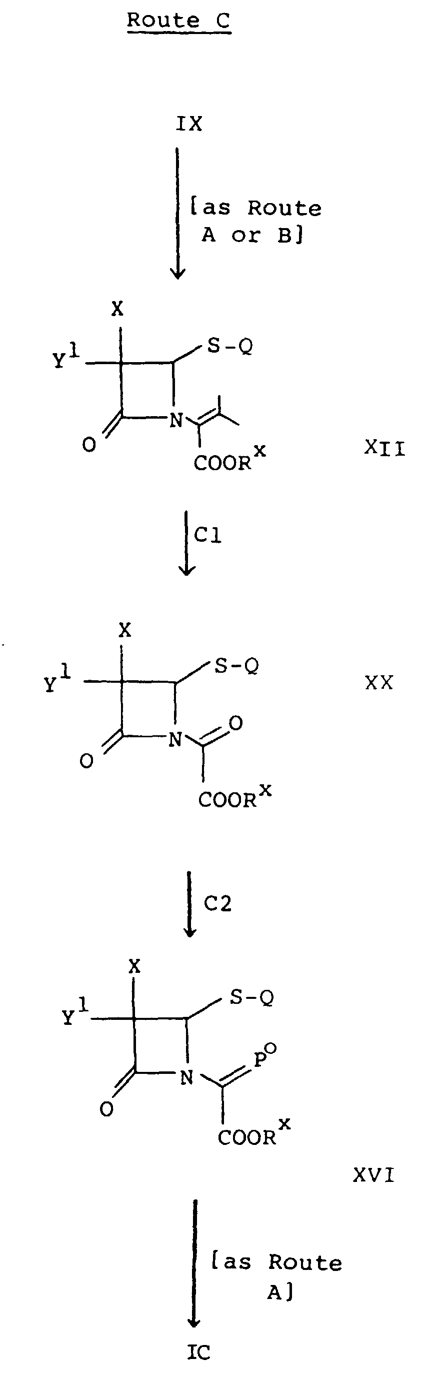

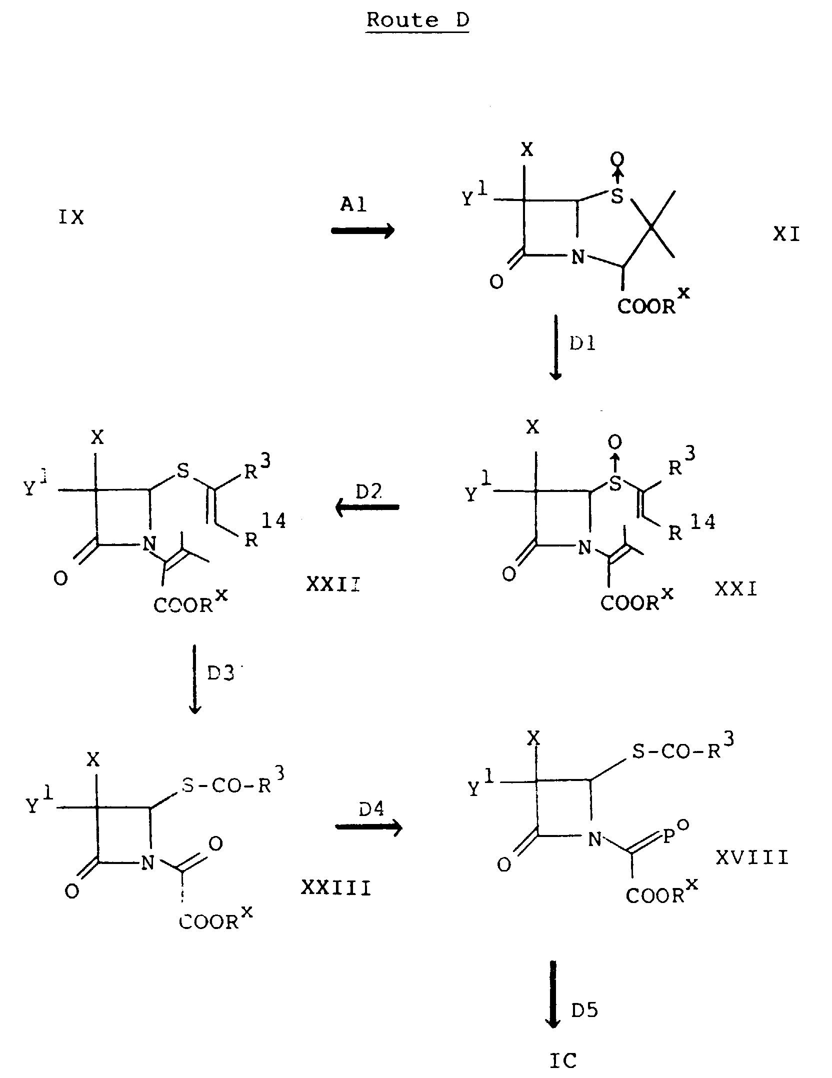

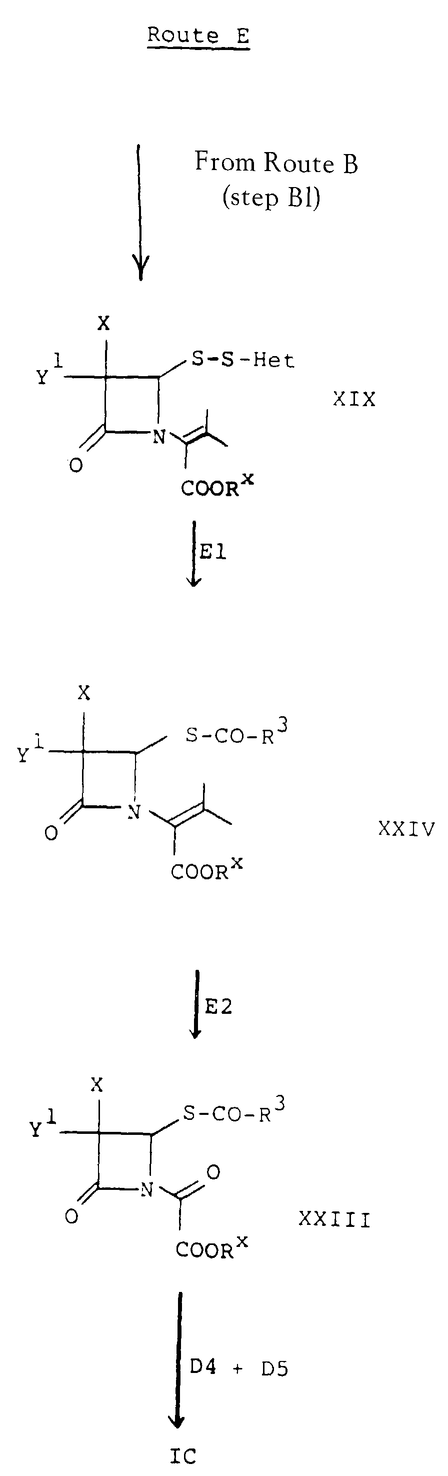

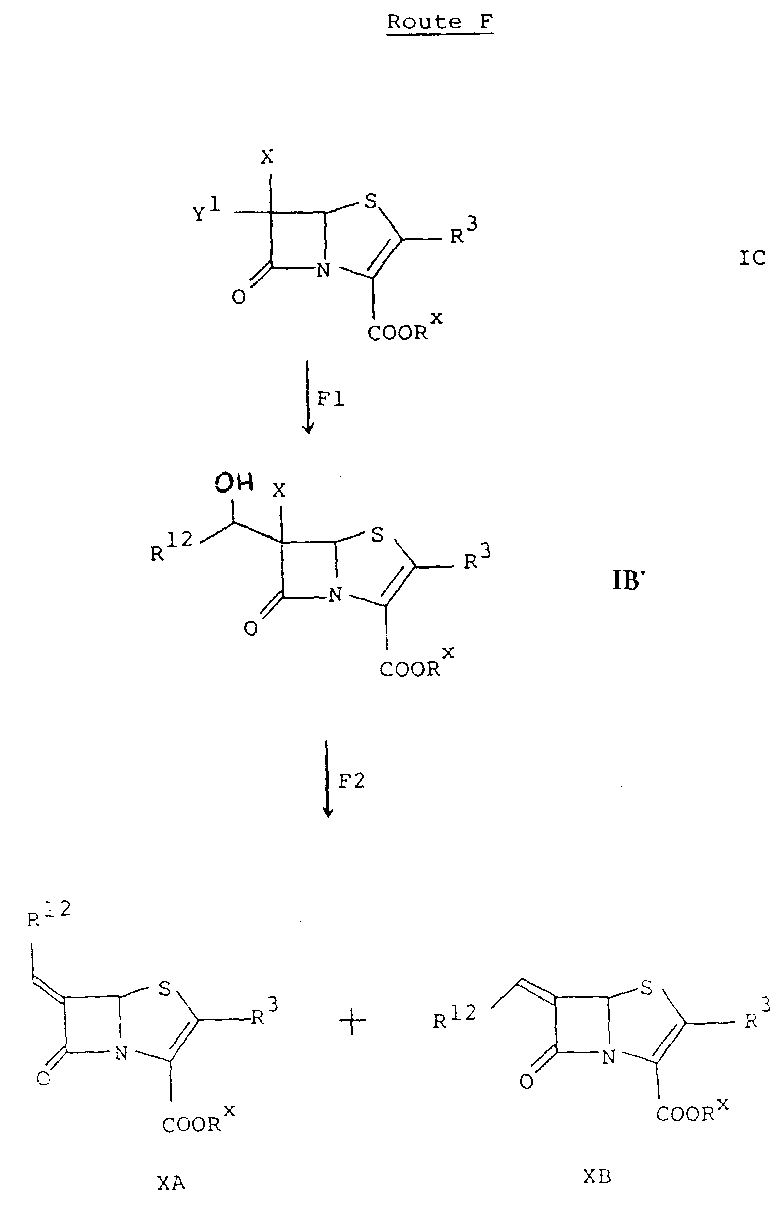

Several of the individual reaction steps outlined in Routes A to E may be carried out in known manner. Although such reaction steps may not have previously been described with respect to 6-halo- and 6,6-dihalo-penicillanic acids or 3-halo- and 3,3-dihalo-azetidin-2-ones, as the case may be, they have in several cases previously been described with respect to penicillanic acids and azetidinones containing various organic substituents at the 6- and 3-positions, respectively. The reagents and methodology known for use with such prior organo-substituted compounds may be applied analogously to the present halo-substituted compounds.

The various routes will now be discussed in more uetail, with examples of suitable reagents and conditions for each step. Where the reaction steps involve methods already known per se, in many cases only brief details of examples of suitable reagents and conditions will be given. The person skilled in the art will readily be able to effect the reactions from the information given herein and from information contained in the scientific literature describing analogous reactions.

As stated previously, the starting compound for each route is a protected 6-halo- or 6,6-dihalo-penicillanic acid of the general formula IX. Such a compound is already known and may conveniently be obtained from a penicillin or from 6-aminopenicillanic acid (6-APA) in known manner.

### Route A

Parts of Route A constitute conventional penem preparation methods and have been described in numerous publications, including, for example, EP 0 002 210 A (pages 13-14), EP 0 138 538 A (Scheme A), GB 2 042 514 A, GB 2 042 515 A, GB 2 111 496 A, GB 2 124 614 A, GB 2 144 126 A, GB 2 144 743 A, and EP 0 120 613 A, EP 0 150 781 A, and EP 0 154 132 A, but with organo substituents instead of the halo substitutents. The corresponding steps of the present route may be carried out analogously.

Steps A1 to A3 may conveniently be carried out using compounds in which the carboxy-protecting group R^{x} is a methyl group, and Steps A4 to AS may conveniently be carried out using, for example a p-methoxybenzyl group as the carboxy-protecting group R^{x}.

The acyl protecting group Q is advantageously an acetyl group.

The intermediate of the general formula XVI may conveniently be obtained, in Step A6, by reaction of the intermediate haloester (which is preferably a chloroester) of the general formula XV with a phosphine compound (preferably, triphenylphosphine, another triarylphosphine, or a trialkoxyphosphine), suitably at a temperature within the range of from 10 to 120°C, preferably at from 15 to 50°C, in an organic solvent (for example tetrahydrofuran or dioxan), and in the presence of a base, for example pyridine or lutidine.

The metal salt intermediate of the general formula XVII is preferably a silver salt intermediate. It may conveniently be obtained, in Step A7, from the phosphorane intermediate of the general formula XVI by reaction with a metal salt, preferably a silver salt, especially an inorganic silver salt, for example silver nitrate, suitably at a temperature within the range of from -20 to +30°C, preferably in the presence of a base, for example l,8-diazabicyclo[5.4.0]undec-7-ene [DBU] or triethylamine.

The metal salt intermediate of the general formula XVII may suitably be converted, in Steps A8 and A9, to the desired penem of the general formula IC by cyclisation, via the corresponding S-acyl azetidinone, which may suitably be obtained from the metal salt by reaction with an appropriate anhydride (for example formic acetic anhydride in the case when R³ = H) in the presence of an acylation catalyst (e.g. 4-dimethylaminopyridine) and a trialkylammonium halide in an aprotic solvent (e.g. acetonitrile, dioxane, or dichloromethane) at a temperature within the range of from -40°C to +40°C, preferably from 0 to 20°C. Cyclisation may then be effected simply by maintaining the reaction mixture, without any intermediate isolation, within the cited temperature range or by heating. In certain cases, cyclisation may be almost spontaneous.

### Route B

Route B constitutes a variation of Route A, providing an alternative (and preferred) route for converting the penam sulphoxide intermediate of the general formula XI to the azetidinone intermediate of the general formula XII instead of using Step A2. The conversion is effected via an intermediate of the general formula XIX, which may be obtained from the penam sulphoxide by reaction with an organic thiol compound (for example 2-mercapto-benzothiazole or 2-mercapto-pyridine), as described by R.G.Micetich et al, Heterocycles, vol.23, No.2, 325 (1985). (Alternatively, the reaction could be effected using compounds analogous to those of the formula XIX in which the -S-Het moiety is replaced by an -SO₂-R¹⁵ moiety in which R¹⁵ denotes optionally substituted phenyl, as described in JP 61 178961 A (Otsuka Kagaku Yakuhin)). The resulting intermediate of the general formula XIX may then be reacted with a trivalent phosphorus compound (for example triphenylphosphine) and acetic anhydride to give the azetidinone intermediate of the general formula XII. In this route, the carboxy-protecting group R^{x} may suitably be a p-methoxybenzyl group.

The azetidinone intermediate of the general formula XII may then be reacted according to Route A or C.

### Route C

Route C constitutes a further preferred variation of Route A, whereby the azetidinone intermediate of the general formula XII (obtained by Route A or B) is converted in two steps to the phosphorane intermediate of the general formula XVI, instead of the four steps of Route A, Steps A3 to A6. In this route, the carboxy-protecting group R^{x} may suitably be a p-methoxybenzyl group.

The azetidinone intermediate of the general formula XII may be subjected to ozonolysis, suitably at a temperature within the range of from -100°C to +20°C, preferably from -80°C to 0°C, in the presence of an aprotic solvent, for example ethyl acetate or dichloromethane, to give the intermediate of the general formula XX, according to Step C1, after a reductive work-up (using, for example, sodium bisulphite).

The intermediate of the general formula XX may then be reacted, according to Step C2, with a phosphine compound (preferably a triarylphosphine or a trialkylphosphite, for example triphenylphosphine or trimethylphosphite) suitably (if necessary) in conjunction with an air-sensitive trivalent phosphorus compound, for example a trialkylphosphite, tributylphosphine, or hexamethyl phosphorous triamide, (E.Perrone et al, Tetrahedron Letters, 25, 22, 2399 - 2402, 1984), at a temperature of from -20°C to +70°C, preferably from +10°C to +50°C. The phosphine compound may suitably be used in an amount of from 1 to 10, preferably from 4 to 5, moles per mole of the compound XX, in conjunction with approximately 1 mole of the air-sensitive trivalent phosphorus compound.

The phosphorane intermediate of the general formula XVI thus obtained may then be converted to the desired penem by Steps A7, A8 and A9 of Route A.

### Route D

Route D constitutes a shorter route than Routes A to C, and proceeds through novel intermediates of the general formulae XXI, XXII, XXIII and XVIII. Various of the reaction steps used in this route have been described in, for example, GB 2 043 639 A, GB 2 111 496 A, GB 2 116 167 A, GB 2 144 743 A, GB 2 144 126 A, and J.Am.Chem. Soc. 1985, 107, 6398-6399, but with organo substituents instead of the halo substituent(s). The reaction steps of the present route may be carried out analogously to those described in the above-cited documents.

The first step of Route D, namely the formation of the penam sulphoxide of the general formula XI is identical to the first step of Route A, except that the carboxy-protecting group R^{x} is suitably a p-methoxybenzyl group.

In the following reaction, Step D1, the penam sulphoxide of the general formula XI is reacted with an acetylene compound of the general formula

R³-C≡C-R¹⁴ XIV

in which
R³ and R¹⁴ are defined as above.

The reaction with the acetylene compound gives an intermediate of the general formula XXI in known manner, via a sulphenic acid obtained by thermolytic ring-opening of the sulphoxide. The choice of groups R³ and R¹⁴ can affect the manner in which the acetylene adds to the sulphenic acid, and it is desired to obtain a product of the general formula XXI rather than one of the general formula XXIA:

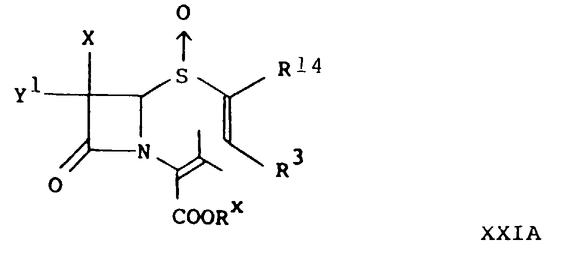

The choice of the group R¹⁴ is not critical in that it is subsequently eliminated in Step D3. It may, however, be critical in ensuring that the desired product of the general formula XXI is obtained. When the desired substituent R³ is a hydrogen atom, the group R¹⁴ may, for example, suitably be a hydrogen atom,an alkyl group, an aryl group, an organosilyl group, an acyl group, or an alkoxycarbonyl group (for example trimethylsilyl, trichloroethoxycarbonyl, methoxy-carbonyl, trifluorethoxycarbonyl, or ethoxycarbonyl). Other suitable groups R¹⁴ will be apparent to the person skilled in the art from the above-cited documents.

The reaction may suitably be effected by heating the penam sulphoxide and the acetylene compound at a temperature within the range of from 50 to 150°C, preferably from 80 to 120°C. In some cases, it may also be necessary to isomerise the resulting double bond in the nitrogen-bonded substituent, suitably by treatment with a base (for example, triethylamine) at from -50°C to +50°C, preferably 0 to 20°C.

Step D2 simply constitutes reduction of the azetidinone sulphoxide of the general formula XXI to the corresponding non-oxidised compound of the general formula XXII. This may suitably be carried out in known manner, for example by treatment with a trivalent phosphorus compound in an organic solvent (for example, phosphorus tribromide in dimethylformamide) at from -20°C to +20°C.

The intermediate of the general formula XXII thus obtained is then subjected to ozonolysis, according to Step D3, to eliminate the group R¹⁴ and form an oxalimide intermediate of the general formula XXIII. This may suitably be effected in the manner described hereinbefore in respect of Step Cl.

The oxalimide intermediate of the general formula XXIII thus obtained may then be reacted, without isolation or purification, according to Steps D4 and D5, to give the desired halo-penem of the general formula IC. Steps D4 and D5 may be carried out as described in GB 2 144 743 A, and suitably involve reaction of the oxalimide intermediate of the general formula XXIII with a trivalent organophosphorus compound (for example, trimethyl phosphite, triethyl phosphite, tetraethyl pyrophosphite, or methyl diphenyl phosphonite), suitably in an excess of up to 10 mole equivalents, in an inert solvent, at a temperature of from 0 to 150°C, to give the halo-penam of the general formula IC directly. Although the reaction proceeds via the phosphorane compound of the general formula XVIII, that compound need not be isolated and the two steps may be considered as a single reaction.

### Route E

Route E constitutes a variation on Route D, whereby a novel intermediate of the general formula XXIV may be obtained, according to Step El, by reaction of an intermediate of the general formula XIX (obtained according to Route B) with an appropriate anhydride (for example formic acetic anhydride in the case when R³ = H) in the presence of a trivalent phosphorus compound (preferably triphenylphosphine), at a temperature of from -50°C to +20°C, in an aprotic solvent (for example, dichloromethane), optionally in the presence of a base (for example, 4-dimethylaminopyridine).

The intermediate of the general formula XXIV thus obtained may then, without isolation or purification, be subjected to ozonolysis according to Step E2 (suitably as described for Step C1) to give an oxalimide intermediate of the general formula XXIII, for subsequent reaction according to Steps D4 and D5.

### Compound IC

The 6-halo- or 6,6-dihalo-penem of the general formula IC, obtained by any of the Routes A to E may be worked up, isolated and purified in any suitable conventional manner, including, for example, solvent extraction, washing with acid (mineral or organic, e.g. citric), base (e.g. sodium bicarbonate), water or brine, chromatography, and/or crystallisation.

Compounds of the formula IC may be inter-converted, that is to say that one compound of the formula IC may be converted to another compound of the formula IC. In particular, a 6a-halo-penem of the formula IC may be epimerised to a 6β-halo-penem of the formula IC, suitably under basic conditions, for example in the presence of DBU. Also a 6-monohalo-penem (Y¹ = H) of the formula IC may be halogenated to give a corresponding 6,6-dihalo-penem, in particular a 6-bromopenem may be converted to a 6,6-dibromopenem.

The carboxy-protecting group R^{x} in a compound of the formula IC may be converted to a free acid, carboxy ester or carboxy salt group R4 in conventional manner appropriate to the particular groups and compatible with the presence of the 6-halo group(s).

The compound of the general formula IC are useful intermediates in the preparation of 6-(substituted methylene)-penems of the general formula (A) and, to that end, they may subsequently be reacted according to the invention (Route F). In that case, the carboxy-protecting groups R^{x} may suitably be retained in the halo-penem of the general formula IC.

### Route F

A 6-halo-penem of the general formula IC may be reacted, according to Step F1, with a compound of the general formula XXV

R¹²-CHO XXV

in which R¹² is defined as above,
to give a halohydrin-penem of the general formula IB in which Z denotes a hydroxyl group.

The reaction may suitably be carried out in the presence of a base, preferably a non-nucleophilic base, and preferably a strong base. Suitable oases include, for example, lithium amide bases, for example lithium bistrimethylsilylamide, lithium dicyclohexylamide, lithium diisopropylamide, lithium 2,2,6,6-tetramethylpiperidide, lithium diphenylamide, and butyllithium.

Suitable solvents for the reaction are aprotic oryanic solvents (which may be polar or non-polar), for example tetrahydrofuran, toluene, dimethoxyethane, dimethylformamide, and mixtures of two or more such solvents.

The reaction may suitably be carried out at a temperature within the range of from -100°C to ambient temperature, preferably from -85°C to 0°C, especially from -85°C to -40°C.

The aldehyde of the general formula XXV and the base may be added to the halo-penem in either order. If it is desired to isolate the halohydrin-penem of the general formula ID in which Z denotes a hydroxy group, the reaction mixture may conveniently be quenched by adding a protic reagent, for example an acid, such as acetic acid or citric acid, or water.

Alternatively, if it is desired to produce a compound of the general formula ID 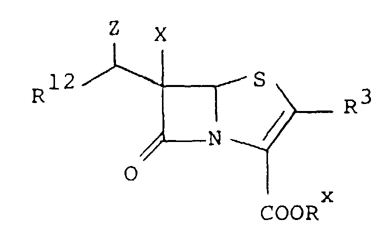 in which Z denotes a substituted hydroxy group, for example a group of the formula OR⁶, in which R⁶ is defined as above, an appropriate reagent to form the desired substituted hydroxy derivative may be added to the reaction mixture instead of the protic reagent. Suitable reagents are those of the general formula XXVI

R⁶-Q¹ XXVI

in which Q¹ denotes a leaving group, suitably having an electron-withdrawing effect, to make R⁶ electrophilic, for example an aryloxy group (e.g. nitrophenoxy), an acyloxy group, corresponding to the carboxylic, sulphonic and phosphoric acid acyl groups of formula IIIA, IIIB and IIIC hereinbefore) or a halogen atom. Suitable reagents of the general formula XXVI to give a penem of the general formula ID in which Z denotes an acetoxy group are, for example, acetyl chloride and acetic anhydride.

If it is desired to obtain a compound of the general formula ID in which Z denotes a halogen atom, an -S(O)ₙR⁵ group or an -Se(O)ₘR⁵ group, as defined above, the halohydrin of the general formula ID in which Z denotes a hydroxy group, may first be isolated as described above and then be reacted with an appropriate reagent in a manner known per se for alcohol reactions.

When using a 6,6-dihalopenem of the general formula IC, Step F1 may be carried out by metal/halogen exchange using a Grignard reagent or an alkyl-lithium compound as described in EP 0 150 984 A (Pfizer) (pages 24-26), isolated as described above, and then be reacted with an appropriate reagent in a manner known per se for alcohol reactions.

If desired the protected carboxy group -COOR^{x} of the compound of the general formula ID may be converted to a free acid, carboxy salt or carboxy ester group R⁴ in known manner. Preferably, however, the protected carboxy group is retained and the compound of the general formula ID is further reacted according to Step F2, without isolation or purification.

The compounds of the general formula ID may exist in four isomeric forms at the C-6 and C-8 positions, as discussed previously, and the sterochemistry may affect the ratio of isomers formed in the subsequent reaction.

A compound of the general formula ID, either as a separated isomer or as a mixture of isomers, may then be subjected to reductive elimination, according to Step F2, to eliminate the groups Z and X and to form two isomeric compounds of the general formula X. That reaction may be carried out in a manner Known per se for such elimination reactions. The elimination may for example, be carried out by reaction with a metal, for example zinc, magnesium, aluminium, or iron, in the presence of an acid (for example, acetic acid or a mineral acid), or by reaction with a triorganophosphorus compound, for example triphenylphosphine, suitably at a temperature within the range of from -20°C to +40°C, preferably from 0°C to 20°C. The reaction may be carried out in the presence of a polar or non-polar, protic or aprotic, organic solvent, for example dioxane, dimethoxyethane, or tetrahydrofuran.

The product of Step F2 is generally a mixture of isomers of the general formulae XA and XB. The desired compounds of the general formulae XA and XB may be isolated and purified in conventional manner for example by known crystallisation or chromatographic techniques. Moreover, the carboxy group -COOR^{x} may be deprotected, that is to say, converted to a free carboxy, carboxy salt or carboxy ester group -COOR⁴ in conventional manner, for example as described in our above-mentioned patent specifications describing the 6-(substituted methylene)-penems.

When it is desired to obtain a free acid or salt of the preferred penem isomer of the formula XA from an isomeric mixture of the penem esters of the formulae XA and XB, this may be effected by chromatographic separation of the esters followed by deprotection of the desired ester isomer to give the corresponding free acid or salt. In some cases, however, it has been found particularly convenient first to deprotect the isomeric mixture to give an isomeric mixture of the free acid or salt of formula XA and XB, followed by fractional recrystallisation to give the desired acid or salt isomer.

One important advantage of the Route F is that the stereo-chemistry at the C-5 position of the penam/penem ring system is maintained. The preferred stereo-chemistry at that position is as indicated in general formula IA and corresponds to that of naturally obtained penicillins. The routes described for the manufacture of 6-(substituted methylene)penems, via the novel 6-halo- and 6,6-dihalo-penems, thus have the advantage of retaining the natural stereochemistry and avoiding the need to separate C-5 isomers. They also have the advantages of using a readily available starting material, and of involving relatively few reaction steps compared with other penem preparation routes.

The compounds of the general formula X are suitably obtained in crystalline form (which may be non-hydrated, hydrated or solvated). The compounds are suitably obtained in a purity of at least 25%, advantageously 50%, preferably 60%, more preferably 75%, especially 80%, more especially 90%, very especially 95%, and most especially 98% by weight, although the purity of compounds used as intermediates is in general less critical than that of compounds used as pharmaceutical final products. Where the compounds of the invention include appropiate substituents they may exist in zwitterionic form.

The following examples illustrate the present invention. In the examples, the various reaction steps and compounds are identified in accordance with the lettering and numbering used in the reaction schemes setting out Routes A to F herein. Roman numerals refer to the general formulae; arabic numerals are used to identify individual compounds. Throughout the general formulae as referred to in the examples, X = Br, Y¹ = H, Q = CH₃CO-, M = Ag, P^{o} = PPh₃ (triphenylphosphoranylidene), R³ = H, unless otherwise specified.

In the following examples, compound ID has the formula: 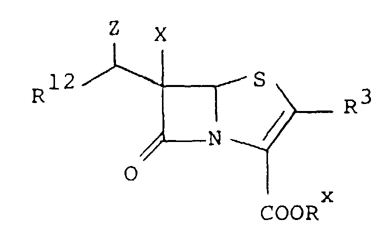 wherein R¹²,Z,X,R³ and R^{x} are as defined in the examples.

Examples 34-50, 52, 55, 57, 60-62, 72, 75, 78, 82, 87, 90, 92, 94, 96, 102 and 104 relate to processes of the invention. The remaining examples fall outside the present invention and are included for reference.

### EXAMPLE 1

### (3S,4R) 4-Acetylthio-3-bromo-1-(1-p-methoxybenzyloxycarbonyl-1-triphenylphosphoranylidenemethyl) azetidin-2-one (Intermediate XVI via Route A)

### EXAMPLE 1a

### Methyl 6-bromopenicillanate-1-oxide (Step A1)

A solution of m-chloroperbenzoic acid (645mg) in ethyl acetate (5ml) was added, dropwise over 5 minutes, to a stirred solution of methyl 6-bromopenicillanate (D.I. John et al, Tetrahedron, 1983, 39, 2477) (1.00g) in ethyl acetate (10ml) at ice-bath temperature. After being stirred at ice-bath temperature for 5 minutes, the mixture was washed with saturated NaHCO₃ solution (3ml) and brine (3 x 3ml). The dried (MgSO₄) organic layer was evaporated and the residue chromatographed on silica gel eluting with ethyl acetate/hexane mixtures to give the title sulphoxide (1; XI R^{x} = Me) (937mg) as a solid, νₘₐₓ (CHCl₃) 1800, 1750cm⁻¹.

### EXAMPLE 1b

### (3S,4R) 4-Acetylthio-3-bromo-1-(1-methoxycarbonyl-2-methylprop-1-enyl)azetidin-2-one (Step A2)

A mixture of the sulphoxide (1) (10.0g), acetic anhydride (15.2ml), and triisopropyl phosphite (8.3ml) were heated in refluxing benzene (200ml) for 4.5 hours. The mixture was evaporated and the residue re-evaporated from xylene (3 x 10ml). The crude product was dissolved in ethyl acetate (100ml) and treated with triethylamine (0.45ml). After 20 minutes at room temperature the mixture was washed with 5% citric acid (5ml), brine (5ml), saturated NaHCO₃ solution (5ml), and brine (3 x 5ml). The dried (MgSO₄) organic layer was evaporated and the residue chromatographed on silica gel eluting with ethyl acetate/hexane mixtures to give a mixture of the title compound and (3S, 4R) 4-acetylthio-3-bromo-1-(1-methoxycarbonyl-2-methylprop-2-enyl)azetidin-2-one as a viscous oil (5.60g). The mixture was redissolved in ethyl acetate (50ml) and treated with triethylamine (0.5ml). After 30 minutes at room temperature the mixture was washed with 5% citric acid (5ml), brine (5ml), saturated NaHCO₃ solution (5ml), and brine (3 x 5ml). The dried (MgSO₄) organic layer was evaporated to give the title compound (2; XII R^{x} = Me) (5.37g) as a viscous oil, νₘₐₓ (CHCl₃) 1780, 1725cm⁻¹; δₚₚₘ (CDCl₃) 1.97 (3H, s), 2.27 (3H, s), 2.38 (3H, s), 3.85 (3H, s), 4.91 (1H, d, J 2Hz), 5.79 (1H, d, J 2Hz).

### EXAMPLE 1C

### (3S,4R) 4-Acetylthio-3-bromoazetidin-2-one (Step A3)

Ozonised oxygen was bubbled through a solution of the azetidinone (2) (5.37g) in ethyl acetate (100ml) at -76°C until a permanent blue solution was obtained (t.l.c. indicated no starting azetidinone present). The excess ozone was removed by passage of argon and the mixture was allowed to attain room temperature. The mixture was evaporated to low volume (approximately 10ml), diluted with methanol (20ml), and treated with 2,6-lutidine (0.1ml). After 1 hour at room temperature the mixture was evaporated to low volume, diluted with ethyl acetate (50ml), and washed with 5% citric acid (5ml), brine (5ml), saturated NaHCO₃ solution (5ml), and brine (3 x 5ml). The dried (MgSO₄) organic layer was evaporated and the residue chromatographed on silica gel eluting with ethyl acetate/hexane mixtures to give the title azetidinone (3, VIII) (2.33g) as a gum, νₘₐₓ (CHCl₃) 3420, 1795, 1705cm⁻¹; δₚₚₘ (CDCl₃) 2.43 (3H, s), 4.86 (1H, dd, J 2 and 3Hz), 5.39 (1H, d, J 2Hz), 7.25-7.80 (1H, broad signal).

### EXAMPLE 1d

### (i) p-Methoxybenzyl glyoxylate hydrate (reagent for Step A4)

A mixture of fumaric acid (5.80g) anhydrous potassium carbonate (6.90g), and p-methoxybenzyl bromide [crude product obtained from the reaction of p-methoxybenzyl alcohol (13.8g) and phosphorous tribromide (3.45ml) in dry ether (50ml) containing pyridine (5 drops) followed by washing with NaHCO₃ solution, brine, drying (MgSO₄), and evaporation] were stirred in dry dimethylformamide (DMF) (100ml) at room temperature for 18 hours followed by 24 hours at 60°C. The mixture was diluted with ethyl acetate (500ml) and washed with brine (3 x 100ml). The dried (MgSO₄) organic layer was evaporated and the residue triturated with ether to give di-p-methoxybenzyl fumarate as a solid (3.41g), νₘₐₓ (CHCl₃) 1720cm⁻¹; δₚₚₘ (CDCl₃) 3.80 (6H, s), 5.20 (4H, s), 6.92 (d, J 9Hz) and 6.93 (s) together 6H, 7.37 (4H, d, J 9Hz).

Ozonised oxygen was bubbled through a solution of di-p-methoxybenzyl fumarate (3.06g) in dichloromethane (100ml) at -20°C until the solution was pale blue (t.l.c indicated no starting material present). The excess ozone was removed by passage of argon and the mixture was treated with dimethyl sulphide (0.76ml). The mixture was allowed to attain room temperature during 30 minutes and evaporated. The residue was dissolved in ethyl acetate (100ml) and washed with water. The dried (MgSO₄) organic layer was evaporated and the residue chromatographed on silica gel eluting with ethyl acetate/hexane mixtures to give the title compound (4) (2.59g) as a viscous oil.

### (ii) (3S,4R) 4-Acetylthio-3-bromo-1-(1-p-methoxybenzyloxycarbonyl-1-hydroxymethyl)azetidin-2-one (Step A4)

A mixture of the azetidinone (3) (224mg) and p-methoxybenzyl glyoxylate (4) (211mg) were heated in refluxing benzene (5ml) with provision for the azeotropic removal of water (Dean and Stark apparatus containing molecular sieves 4A) for 1 hour. The mixture was cooled to room temperature and treated with triethylamine (10mg). After 30 minutes at room temperature the mixture was evaporated to give the crude hydroxyester title compound (5; IVX R^{x} = pMB) as a gum, νₘₐₓ (CHCl₃) 3600-3100, 1790, 1745, 1720cm⁻¹.

### EXAMPLE 1e

### (3S,4R) 4-Acetylthio-3-bromo-1-(1-p-methoxybenzyloxycarbonyl-1-chloromethyl)azetidin-2-one (Step A5)

A solution of the crude hydroxyester (5) in dry tetrahydrofuran (THF) (5ml) was cooled to -10°C and treated with 2,6-lutidine (0.17ml) and thionyl chloride (0.11ml). After stirring for 10 minutes at -10°C the mixture was filtered and the residue was washed with dry THF (3ml). The combined filtrates were evaporated and the residue was re-evaporated from dry toluene (2 x 1ml) to give the crude chloroester title compound (6, XV R^{x} = pMB) as a gum, νₘₐₓ (CHCl₃) 1800, 1750, 1720cm⁻¹.

### EXAMPLE 1f

### (3S,4R) 4-Acethylthio-3-bromo-1-(1-p-methoxybenzyloxycarbonyl-1-triphenylphosphoranylidenemethyl)azetidin-2-one (Step A6; Compound XVI)

A mixture of the crude chloroester (6), triphenylphosphine (1.05g), and dioxan (3ml) were stirred at room temperature until homogenous. The mixture was evaporated to low volume (very viscous oil), treated with 2,6-lutidine (0.14ml), and stirred at 40°C under dry argon for 3 hours. The mixture was diluted with ethyl acetate (20ml) and washed with 5% citric acid (3ml), brine (3ml), saturated NaHCO₃ solution (3ml), and brine (3 x 3ml). The dried (MgSO₄) organic layer was evaporated and the residue chromatographed on silica gel eluting with ethyl acetate/hexane mixtures to give the title phosphorane (7; XVI R^{x} = pMB) (213mg) as a solid, νₘₐₓ (CHCl₃) 1775, 1695, 1615cm⁻¹.

### EXAMPLE 2

### (3S,4R) 4-Acetylthio-3-bromo-1-(1-p-methoxybenzyloxycarbonyl-1-triphenylphosphoranylidenemethyl)azetidin-2 -one (Intermediate XVI via Routes A or B and C)

### EXAMPLE 2a

### Dicyclohexylammonium 6-bromopenicillanate (Preparation of Starting Material VIII in salt form)

6-Aminopenicillanic acid (144g) in methanol (1333ml) and water (500ml) was stirred at 0°C and treated slowly with hydrobromic acid (48%, 500ml), the temperature being maintained below 5°C. After the addition was complete, sodium nitrite (68.7g) was added, portionwise over approximately 15 minutes, and the resulting solution was stirred without cooling for a further 1 hour. The mixture was poured into a mixture of water (1333ml) and chloroform (1333ml) and the organic layer separated. The aqueous layer was re-extracted with chloroform (2 x 833ml). The combined organic layers were washed with water (833ml) and brine (833ml), dried (MgSO₄), and evaporated to give a green oil. The oil was redissolved in ether (1000ml), diluted with petroleum ether (b.p. 40-60°C, 500ml), and cooled in an ice bath. To the stirred solution was added, in one portion, a solution of dicyclohexylamine (166ml) in ether (160ml). After being stirred overnight at 4°C, the solid mixture was filtered, washed with ether/ petroleum ether (1:1), and dried under vacuum to give the title salt (8) (218.4g) as a solid, δₚₚₘ (CDCl₃) 0.8-2.3 (26H, broad m), 2.6-3.3 (2H, broad m), 4.33 (1H, s), 4.75 (1H, d, J 2Hz), 5.35 (1H, d, J, 2Hz), 8.2-9.3 (broad signal, exch. D₂O).

### EXAMPLE 2b

### p-Methoxybenzyl 6-bromopenicillanate (Preparation of protected Starting Material IX)

A solution of phosphorus tribromide (11.7ml) in dry ether (25ml) was added, dropwise over 15 minutes, to a stirred mixture of 4-methoxybenzyl alcohol (51.6g) and pyridine (25 drops) in dry ether (500ml) at 10°C. After being stirred without cooling for 15 minutes, the mixture was washed with brine (100ml), saturated NaHCO₃ (100ml), and brine (100ml). The dried (MgSO₄) organic layer was evaporated to low volume, diluted with DMF (250ml), and added dropwise to a stirred solution of the dicyclohexylamine salt (8) (172.7g) in DMF (1000ml) at 10°C. After being stirred at room temperature for 18 hours, the mixture was poured into a mixture of water (2000ml) and ethyl acetate (2000ml). The organic layer was separated and the aqueous layer was re-extracted with ethyl acetate (2 x 1000ml). The combined organic layers were washed with 5% citric acid (1000ml), saturated NaHCO₃ solution (1000ml), and brine (1000ml). The dried (MgSO₄) organic layer was evaporated to give the title ester (9, IX R^{x} = pMB) (233g) as an oil which crystallised on standing, δₚₚₘ (CDCl₃) 1.31 (3H, s), 1.52 (3H, s), 3.74 (3H, s), 4.47 (1H, s), 4.70 (1H, d, J 2Hz), 5.08 (2H, s), 5.35 (1H, d J 2Hz), 6.80 (2H, d, J 8Hz), 7.25 (2H, d, J 8Hz).

### EXAMPLE 2c

### p-Methoxybenzyl 6-bromopenicillanate-1-oxide (Step A1)

A solution of m-chloroperbenzoic acid (78.5g) in dichloromethane (700ml) was added, over 30 minutes, to a stirred solution of the ester (9) (165.5g) in dichloromethane (1600ml) at 5°C. After 1 hour at 5°C the mixture was filtered and the filtrate was washed with saturated NaHCO₃ solution (2 x 500ml), water (500ml), and brine (500ml). The dried (MgSO₄) organic layer was evaporated and the residual oil was triturated with ethyl acetate to give the title sulphoxide (10; XI R^{x} = pMB) (122.8g) as a crystalline solid, δₚₚₘ (CDCl₃) 1.45 (3H, s), 2.00 (3H, s), 4.18 (3H, s), 4.90 (1H, s), 5.40 (2H, s), 5.52 (1H, s), 5.56 (1H, s), 7.25 (2H, d, J 8Hz), 7.68 (2H, d, J 8Hz).

### EXAMPLE 2d

### (3S,4R) 4-(Benzothiazol-2-yldithio)-3-bromo-1-(1-p-methoxybenzyloxycarbonyl-2-methylprop-1-enyl)azetidin -2-one (Step B1)

A mixture of the sulphoxide (10) (6.2g) and 2-mercaptobenzothiazole (2.5g) was heated in refluxing toluene (200ml) with provision for azeotropic removal of water (Dean and Stark apparatus) for 2 hours. The mixture was cooled to 5°C and treated with triethylamine (0.23ml). After being stirred at 5°C for 2 hours, the mixture was washed with 5% citric acid (50ml), water (50ml) and brine (50ml). The dried (MgSO₄) organic layer was evaporated and the residue chromatographed on silica gel eluting with ethyl acetate/hexane mixtures to give the title disulphide (11; XIX R^{x} = pMB, Het = benzothiazol-2-yl) (8.1g) as an oil, νₘₐₓ (CH₂Cl₂) 1785, 1720cm⁻¹; δₚₚₘ (CDCl₃) 1.92 (3H, s), 2.14 (3H, s), 3.77 (3H, s), 5.02 (1H, d, J 2Hz), 5.19 (2H, centre of m), 5.39 (1H, d, H, 2Hz), 6.79-8.10 (8H, m).

### EXAMPLE 2e

### (3S,4R) 4-Acetylthio-3-bromo-1-(1-p-methoxybenzyloxycarbonyl-2-methylprop-1-enyl)azetidin-2-one (Step B2)

Triphenylphosphine (3.9g) was added, portionwise over 20 minutes, to a stirred solution of the disulphide (11) (7.8g) in a mixture of acetic anhydride (66ml) and glacial acetic acid (22ml) under nitrogen at -20°C. After stirring at -20°C for a further 45 minutes the mixture was treated with pyridine (44ml) and allowed to attain room temperature. After stirring at room temperature for 3 hours the mixture was evaporated to an oil which was re-dissolved in ethyl acetate (50ml) and washed with 5% citric acid and brine. The dried (MgSO₄) organic layer was evaporated and the residue chromatographed on silica gel eluting with ethyl acetate/hexane mixtures to give the title azetidinone (12; XII R^{x} = pMB) (5.5g), νmax (CHCl₃) 1785, 1720cm⁻¹; δₚₚₘ (CDCl₃) 1.95 (3H, s), 2.24 (3H, s), 2.31 (3H, s), 3.80 (3H, s), 4.80 (1H, d, J 2Hz), 5.21 (2H, s), 5.71 (1H, d, J 2Hz), 6.92 (2H, d, J 9Hz), 7.42 (2H, d, J 9Hz).

### EXAMPLE 2f

### (3S,4R) 4-Acetylthio-3-bromo-1-(1-p-methoxybenzyloxycarbonyl-2-methylprop-1-enyl)azetidin-2-one (Step A2; alternative to Examples 2d and 2e)

A mixture of the sulphoxide (10) (416mg), acetic anhydride (0.47ml), and triisopropyl phosphite (0.27ml) were heated in refluxing benzene under dry argon for 8 hours. The mixture was evaporated and the residue re-evaporated from xylene (2 x 5ml). The crude product was dissolved in ethyl acetate (5ml) and was treated with triethylamine (10mg). After 20 minutes at room temperature the mixture was diluted with ethyl acetate (5ml) and washed with 5% citric acid (1ml), brine (1ml), saturated NaHCO₃ (1ml), and brine (3 x 1ml). The dried (MgSO₄) organic layer was evaporated and the residue chromatographed on silica gel eluting with ethyl acetate/hexane mixtures to give the title azetidinone (12; XII R^{x} = pMB) (221mg), as specified in Example 2e.

### EXAMPLE 2g

### (3S,4R) 4-Acetylthio-3-bromo-1-(p-methoxybenzyloxyoxalyl)azetidin-2-one (Step C1)

Ozonised oxygen was bubbled through a solution of the azetidinone (12) (165mg) in ethyl acetate (5ml) at -76°C until a permanent blue solution was obtained (t.l.c. indicated that no starting material remained). The excess ozone was removed by passage of argon. The mixture was diluted with ethyl acetate (5ml) and washed with 45% sodium bisulphite solution (1ml), brine (1ml), saturated NaHCO₃ solution (1ml) and brine (3 x 1ml). The dried (MgSO₄) organic layer was evaporated to give the title oxalimide (13, XX R^{x} = pMB) (139mg) as a gum, νₘₐₓ (CHCl₃) 1830, 1755, 1725cm⁻¹; δₚₚₘ (CDCl₃) 2.38 (3H, s), 3.82 (3H, s), 5.06 (1H, d, J 2½Hz), 5.32 (2H, s), 5.71 (1H, d, J 2½ Hz), 6.93 (2H, d, J 9Hz), 7.41 (2H, d, J 9Hz).

### EXAMPLE 2H

### (3A,4R) 4-Acetylthio-3-bromo-1-(1-p-methoxybenzyloxycarbonyl -1-triphenylphosphoranylidenemethyl)azetidin-2-one (Step C2; Compound XVI)

Samples of the oxalimide (13), which could be prepared as described in Example 2g, were treated as follows:
(i) A solution containing the oxalimide (13), (210mg), triphenylphosphine (530mg), and trimethyl phosphite (63mg) in dry toluene (5ml) was kept at room temperature for 18 hours. The mixture was diluted with toluene (5ml) and was washed with brine (3 x 1ml). The dried (MgSO₄) organic layer was evaporated and the residue chromatographed on silica gel eluting with ethyl acetate/hexane mixtures to give the title phosphorane (7) (117mg) as a solid.
(ii) A solution containing the oxalimide (13) (210mg), triphenylphosphine (132mg), and trimethyl phosphite (63mg) in dry toluene (5ml) was kept at room temperature for 18 hours and worked up as in (i) above to give the title phosphorane (7) (84mg) as a solid.
(iii) A solution containing the oxalimide (13) (210mg), triphenylphosphine (530mg), and triethyl phosphite (84mg) in dry toluene (5ml) and kept at room temperature for 18 hours and worked up as in (i) above to give the title phosphorane (7) (176mg) as a solid.
(iv) A solution containing the oxalimide (13) (210mg), triphenylphosphine (530mg), triisopropyl phosphite (105mg) in dry toluene (5ml) was kept at room temperature for 18 hours and worked up as in (i) above to give the phosphorane (7) (92mg) as a solid.

### EXAMPLE 3

### Silver (3S,4R) 3-bromo-1-(1-p-methoxybenzyloxycarbonyl-1-triphenylphosphoranylidenemethyl)azetidin-2 -one-4-thiolate (Step A7; Intermediate XVII)

A solution of 1,8-diazabicyclo[5.4.0]undec-7-ene (DBU) (55mg) in dry dichloromethane (0.5ml) was added, dropwise over 0.5 minute, to a stirred mixture of the phosphorane (7) (200mg), dry dichloromethane (1ml), and silver nitrate (2.22ml of a 0.15M solution in methanol) at ice-bath temperature. The mixture was stirred at ice-bath temperature for 15 minutes and evaporated. The residue was triturated with cold methanol and filtered. The residue was washed with a little cold methanol and dry ether and dried under vacuum to give the title silver salt (14; XVII R^{x} = pMB) (186mg) as a buff coloured solid, νₘₐₓ (Nujol) 1765, 1615cm⁻¹.

### EXAMPLE 4

### Silver (35,4R) 3-bromo-1-(1-p-methoxybenzyloxycarbonyl-1-triphenylphosphoranylidenemethyl)azetidin-2 -one-4-thiolate (Step A7; Intermediate XVII)

A solution of triethylamine (67mg) in methanol (1ml) was added, dropwise over 1 minute, to a stirred mixture of the phosphorane (7) (340mg), dry dichloromethane (4ml), and silver nitrate (4.46ml of a 0.15M solution in methanol) at ice-bath temperature. The mixture was stirred at ice-bath temperature for 40 minutes, evaporated to half volume, filtered, and worked up as for Example 3 to give the title silver salt (14; XVII R^{x} = pMB) (346mg) as a buff coloured solid.

### EXAMPLE 5

### p-Methoxybenzyl (5R,6S) 6-bromopenem-3-carboxylate (Steps A8 and A9; Compound IC)

A stirred, ice-bath cooled, solution of the silver salt (14) (182mg) in dry dichloromethane (3ml) was treated with formic acetic anhydride (0.20ml) and 4-dimethylaminopyridine (31mg). The ice bath was removed and the vigorously stirred mixture was treated with triethylamine hydrochloride (350mg). After 10 minutes the mixture was diluted with ethyl acetate (10ml) and filtered through Kieselguhr, the residue being washed with ethyl acetate (10ml). The combined filtrates were washed with 5% citric acid (3ml), brine (3ml), saturated NaHCO₃ solution (3ml), and brine (3 x 3ml). The dried (MgSO₄) organic layer was kept at room temperature for 30 minutes and evaporated. The residue was chromatographed on silica gel eluting with dichloromethane to give the title bromopenem ester (15, IC R³ = H, R^{x} = pMB) (41mg) as a solid, m.p. 102-104°C (needles ex ethyl acetate/hexane); [α]_{D}²⁰ +83°C (c 1 in CHCl₃); λₘₐₓ (EtOH) (εₘ 7,065), 280 (6,697), 274 (6,663) and 226nm (16,056); νₘₐₓ (CHCl₃) 1805, 1715cm⁻¹; δₚₚₘ (CDCl₃) 3.81 (3H, s), 5.15 (1H, dd, J 1.5 and 1.0Hz), 5.17 and 5.23 (2H, ABq, J 12.0Hz), 5.76 (1H, d, J 1.5Hz), 6.90 (2H, dd, J 6.7 and 2.0Hz), 7.25 (1H, d, J 1.0Hz), 7.35 (2H, dd, J 6.7 and 2.0Hz) (Found: C, 45.4; H, 3.2; N, 3.7; Br, 21.4; S, 8.5%; M⁺, 368.9673. C₁₄H₁₂NBrO₄S requires C, 45.4; H, 3.3; N, 3.8; Br 21.6; S, 8.7%; M, 368.9671).

### EXAMPLE 6

### (3S,4R) 3-Bromo-1-(p-methoxybenzyloxycarbonyl-2-methylprop-1-enyl)-4-[2-(2,2,2-trichloroethoxy carbonyl)ethenylsulphinyl]azetidin-2-one (Step D1; Intermediate XXI)

A solution containing sulphoxide (10; XI R^{x} = pMB) (4.2g), toluene (1ml), and 2,2,2-trichloroethyl propiolate (2.0g) was heated at 110°C (bath) for 2 hours and then dissolved in dichloromethane (30ml). Triethylamine (0.07ml) was then added, and the solution stirred for 10 minutes at 20°C and then washed successively with aqueous citric acid, aqueous sodium bicarbonate, and brine. Evaporation and recrystallisation of the residue from ether then gave the title compound (16, XXI R³ = H, R^{x} = pMB, R¹⁴ = trans CO₂CH₂CCl₃) (3.5g) as small white crystals, δₚₚₘ (CDCl₃) 2.1 and 2.3 (6H, each s), 3.8 (3H, s), 4.8 (2H, s), 5.0 (2H, m), 5.1 (2H, ABq), 6.8 and 7.3 (4H, ABq), 6.7 and 7.5 (2H, each d, J 15Hz).

### EXAMPLE 7

### (3S, 4R) 3-Bromo-1-(p-methoxybenzyloxycarbonyl-2-methylprop-1-enyl)-4-[2-(2,2,2-trifluoroethoxycarbonyl) ethenylsulphinyl]azetidin-2-one (Step D1; Intermediate XXI)

Using the method described in Example 6 the sulphoxide (10) (4.2 g) was treated with 2,2,2-trifluoroethyl propiolate (3.0g). The excess propiolate was removed by evaporation before dissolution of the reaction mixture in dichloromethane and treatment with triethylamine. Crystallisation from ether gave the title azetidinone (17; XXI R³ = H, R^{x} = pMB, R¹⁴ = trans CO₂CH₂CF₃) (1.0g) as white rhombs, δₚₚₘ (CDCl₃) 2.2 and 2.4 (6H, each s), 3.9 (3H, s), 4.7 (2H, q, J 8Hz), 5.1 (2H, s), 5.2 (2H, ABq), 6.9 and 7.4 (4H, ABq), 6.8 and 7.7 (2H, each d, J 15Hz).

### EXAMPLE 8

### (3S, 4R) 3-Bromo-1-(1-p-methoxybenzyloxycarbonyl-2- methylprop-1-enyl)-4-(2-ethoxycarbonylethenylsulphinyl)azetidin-2-one (Step D1; Intermediate XXI)

A mixture of the sulphoxide (10) (4.2g) and ethyl propiolate (2.0ml) were heated in refluxing toluene (30ml) for 30 minutes and evaporated. The residue was redissolved in dichloromethane (30ml) and treated with triethylamine (20 drops) as in Example 6 to give, after evaporation, the crude sulphoxide title compound (18; XXI R³ = H, R^{x} = pMB, R¹⁴ = CO₂C₂H₅).

### EXAMPLE 9

### (3S,4R) 3-Bromo-4(E)-(2-ethoxycarbonylethenylthio)-1-(1-p-methoxybenzyloxycarbonyl-2-methylprop-1-enyl) -azetidin-2-one (Step D2; Intermediate XXII)

The crude azetidinone (18) from Example 8 was redissolved in DMF (30ml), cooled to -10°C, and treated with phosphorus tribromide (0.95ml). After 20 minutes at -10°C the mixture was treated with ethyl acetate, toluene and saturated NaHCO₃ solution. The organic layer was separated and washed thoroughly with water. The dried (MgSO₄) organic layer was evaporated and the residue chromatographed on silica gel eluting with ethyl acetate/dichloromethane mixtures to give two fractions. The fraction eluted first contained (3S,4R) 3-bromo-4-(1-ethoxycarbonylethenylthio)-1-(1-p-methoxybenzyloxycarbonyl-2-methylprop-1-enyl)azetidin-2-one (0.33g), δₚₚₘ (CDCl₃) 1.4 (3H, t, J 7Hz), 2.0 and 2.3 (6H, each s), 3.8 (3H, s), 4.3 (2H, q, J 7Hz), 4.7 (1H, d, J 2Hz), 5.2 (3H, m), 5.7 and 6.5 (2H, each s), 6.9 and 7.4 (4H, ABq). The fraction eluted second contained the title azetidinone (19; XXII R³ = H, R^{x} = pMB, R¹⁴ = trans CO₂C₂H₅) (0.55g), δₚₚₘ (CDCl₃) 1.3 (3H, t, J 7HZ), 2.0 and 2.3 (6H, each s), 3.8 (3H, s), 4.2 (2H, q, J 7Hz), 4.7 (1H, d, J 2Hz), 5.2 (3H, m), 5.9 and 7.4 (2H, d, J 15Hz), 6.9 and 7.3 (4H, ABq).

### EXAMPLE 10

### (3S,4R) 3-Bromo-1-(1-p-methoxybenzyloxycarbonyl-2-methylprop-1-enyl)-4(E)-(2-methoxycarbonylethenylthio) azetidin-2-one (Steps D1 and D2, Intermediate XXII)

Reaction of the sulphoxide (10; XI R^{x} = pMB) (4.2g) with methyl propiolate (3.6ml) followed by triethylamine and finally phosphorus tribromide/DMF using the methods described in Examples 8 and 9 gave a crude mixture of the title azetidinone and (3S,4R) 3-bromo-1-(1-p-methoxybenzyloxycarbonyl-2-methylprop-1-enyl)-4-(1-methoxycarbonylethenylthio)azetidin-2-one in an approximately 4:1 ratio. Chromatography of the crude product afforded the title azetidinone (20; XXII R³ = H, R^{x} = pMB, R¹⁴ = trans CO₂CH₃) (2.56g) as a colourless gum, δₚₚₘ (CDCl₃) 2.0 and 2.4 (6H, each s), 3.8 (6H, s), 4.8 (1H, d, J 2Hz), 5.2 (3H, m), 5.9 and 7.4 (2H, each d, J 15Hz), 6.9 and 7.3 (4H, ABq).

### EXAMPLE 11

### (3S,4R) 3-Bromo-1-(1-p-methoxybenzyloxycarbonyl-2-methylprop-1-enyl)-4(E)-[2-(2,2,2-trichloroethoxy -carbonyl)ethenylthio]azetidin-2-one (Steps D1 and D2; Intermediate XXII)

Reaction of the sulphoxide (10) (4.2g) with 2,2,2-trichloroethyl propiolate (2.0g) followed by triethylamine and finally phosphorus tribromide/DMF using the methods described in Examples 8 and 9 gave the title azetidinone (21; XXII R³ = H, R^{x} = pMB, R¹⁴ = trans CO₂CH₂CCl₃) (2.67g) as a colourless gum, δₚₚₘ (CDCl₃) 2.0 and 2.3 (6H, each s), 3.8 (3H, s), 4.7 (3H, m), 5.1 (2H, ABq), 5.2 (1H, d, J 2Hz), 5.9 and 7.5 (2H, each d, J 15Hz), 6.8 and 7.2 (4H, ABq).

### EXAMPLE 12

### (3S,4R) 3-Bromo-1-(1-p-methoxybenzyloxycarbonyl-2-methylprop-1-enyl)-4(E)-(2-trimethylsilylethenylthio) azetidin-2-one (Steps D1 and D2; Intermediate XXII)

Reaction of the sulphoxide (10) (4.2g) with trimethylsilylacetylene (2.8ml) in refluxing toluene for 3 hours followed by treatment with triethylamine and phosphorus tribromide/DMF using the methods described in Examples 8 and 9 gave the title azetidinone (22; XXII R³ = H, R^{x} = pMB, R¹⁴ = trans SiMe₃) (3.65g) as a colourless gum, δₚₚₘ (CDCl₃) Me₃SI obscured by TMS signal, 1.9 and 2.2 (6H, each s), 3.7 (3H, s), 4.6 (1H, d, J 2Hz), 5.0 (2H, ABq), 5.1 (1H, d, J 2Hz), 5.8 and 6.3 (2H, ABq), 6.8 and 7.2 (4H, ABq).

### EXAMPLE 13

### (3S,4R) 3-Bromo-4-(formylthio)-1-(p-methoxybenzyloxyoxalyl)azetidin-2-one (Step D3; Intermediate XXIII)

Ozonised oxygen was passed into a solution of an azetidinone (19 to 22; XXII R³ = H, R^{x} = pMB, R¹⁴ optional) in ethyl acetate (ca. 0.05M) at -55 to -65°C (int.) until a strong blue colour persisted. Excess ozone was then swept out with oxygen, toluene added, and the resulting solution washed with aqueous sodium bisulphite adjusted to pH 7, and then with water. Drying and evaporation then gave the title oxalimide (23; XXIII R^{x} = pMB) as a colourless oil; νₘₐₓ (film) 1830, 1750, 1720, 1690 sh cm⁻¹; δₚₚₘ (CDCl₃) 3.8 (3H, s), 5.3 (2H, s), 5.0 and 5.8 (2H, each d, J 3Hz), 6.8 and 7.4 (4H, ABq), 10.1(1H, s).

### EXAMPLE 14

### p-Methoxybenzyl (5R, 6S) 6-bromopenem-3-carboxylate (Steps D3 to D5; Compound IC)

A solution of the azetidinone (21; XXII R³ = H, R^{x} = pMB, p¹⁴ = trans CO₂CH₂CCl₃) (600mg) in ethyl acetate (30ml) was ozonised and washed as in Example 13. The dried (MgSO₄) organic layer was evaporated until the toluene began to distil. The residual toluene solution was dried (MgSO₄) and treated with trimethyl phosphite (0.35ml) and the mixture was stirred for 30 minutes at 90°C under argon. The mixture was cooled to room temperature and applied to a column of silica gel (prepared from a slurry in hexane). Elution with ethyl acetate/hexane mixtures afforded the title bromopenem ester (15; IC R³ = H, R^{x} = pMB) (100mg).

### EXAMPLE 15

### p-Methoxybenzyl (5R,6S) 6-bromopenem-3-carboxylate (Steps D3 to D5; Compound IC)

Using the conditions described in Example 14, the azetidinone (20; XXII R³ = H, R^{x} = pMB, R¹⁴ = trans CO₂CH₃) (490mg) gave the title bromopenem ester (15; IC R³ = H, R^{x} = pMB) (80mg).

### EXAMPLE 16

### p-Methoxybenzyl (5R,6S) 6-bromopenem-3-carboxylate (Steps D3 to D5; Compound IC)

Using the conditions described in Example 14, the azetidinone (19; XXII R³ = H, R^{x} = pMB, R¹⁴ = trans CO₂C₂H₅) (500mg) gave the title bromopenem ester (15, IC R³ = H, R^{x} = pMB) (126mg).

### EXAMPLE 17

### p-Methoxybenzyl (5R,6S) 6-bromopenem-3-carboxylate (Steps D3 to D5; Compound IC)

Using the conditions described in Example 14, an unchromatographed sample of the azetidinone (20; XXII R³ = H, R^{x} = pMB, R¹⁴ = trans CO₂CH₃) (prepared from the sulphoxide (10) (420mg) using the method described in Example 10) gave the title bromopenem ester (15; IC R³ = H, R^{x} = pMB) (95mg).

### EXAMPLE 18

### p-Methoxybenzyl (5R,6S) 6-bromopenem-3-carboxylate (Steps D3 to D5; Compound IC)

A dried toluene solution of the oxalimide (23) prepared by ozonolysis and work up of the azetidinone (22; XXII R³ = H, R^{x} = pMB, R¹⁴ = trans SiMe₃) (500mg) according to the method described in Example 14 was added to a solution of trimethyl phosphite (0.60ml, 5 equivalents) in toluene (5ml) at 50°C. After 1 hour at 50°C, the mixture was worked up as in Example 14 to give the title bromopenem ester (15; IC R³ = H, R^{x} = pMB) (74mg).

### EXAMPLE 19

### p-Methoxybenzyl (5R,6S) 6-bromopenem-3-carboxylate (Steps D3 to D5; Compound IC)

Example 18 was repeated, with the variation that the mixture was heated at 90°C for 30 minutes to give the title bromopenem ester (15; IC R³ = H, R^{x} = pMB) (67mg).

### EXAMPLE 20

### p-Methoxybenzyl (5R,6S) 6-bromopenem-3-carboxylate (Steps D3 to D5; Compound IC)

A dried toluene solution of the oxalimide (23) prepared as in Example 18 was added to a solution of trimethyl phosphite (3 equivalents) in toluene at 20°C. The mixture was then heated at 50°C for 1 hour to give the title bromopenem ester (15; IC R³ = H, R^{x} = pMB) (48mg).

### EXAMPLE 21

### p-Methoxybenzyl (5R,6S) 6-bromopenem-3-carboxylate (Steps D3 to D5; Compound IC)

Example 20 was repeated, with the variation that 7 equivalents of the trimethyl phosphite were used. 52 mg of the title bromopenem ester (15; IC R³ = H, R^{x} = pMB) were obtained.

### EXAMPLE 22

### p-Methoxybenzyl (5R,6S) 6-bromopenem-3-carboxylate (Steps D3 to D5; Compound IC)

A dried toluene solution of the oxalimide (23) prepared as in Example 18 was added to a solution of triethyl phosphite (5 equivalents) in toluene at 0°C. The mixture was then heated at 50°C for 1 hour to give the title bromopenem ester (15; IC R³ = H, R^{x} = pMB (26mg).

### EXAMPLE 23

### p-Methoxybenzyl (5R,6S) 6-bromopenem-3-carboxylate (Steps D3 to D5; Compound IC)

A dried toluene solution of the oxalimide (23) prepared as in Example 18 was heated with tetraethyl pyrophosphite (5 equivalents) for 1 hour at 50°C to give the title bromopenem ester (15; IC R³ = H, R^{x} = pMB) (20mg).

### Example 24

### p-Methoxybenzyl (5R,6S) 6-bromopenem-3-carboxylate (Steps D3 to D5; Compound IC)

A dried toluene solution of the oxalimide (23) prepared as in Example 18 was treated with methyl diphenylphosphonite (0.36ml) at 20°C. After 1 hour at 20°C the mixture was filtered through a column of silica gel eluting with ethyl acetate. The eluate was evaporated and the residue was dissolved in toluene. The resulting solution was heated under argon at 90°C for 2 hours and evaporated. The residue was chromatographed on silica gel eluting with ethyl acetate/hexane mixtures to give the title bromopenem ester (15; IC R³ = H, R^{x} = pMB) (22mg).

### EXAMPLE 25

### p-Methoxybenzyl (5R,6S) 6-bromopenem-3-carboxylate (Compound IC)

### EXAMPLE 25a

### (3S,4R) 3-Bromo-4-formylthio-1-(1-p-methoxybenzyloxycarbonyl-2-methylprop-1-enyl)azetidin-2-one (Step E1; Compound XXIV)

The disulphide (11; XIX R^{x} = pMB) (1.00g) obtained as in Example 2d was dissolved in a solution of dichloromethane (40ml) containing acetic-formic anhydride (1.42ml) and cooled to -20°C, under an atmosphere of argon. Triphenylphosphine (0.464mg) was added portionwise to the stirred solution over a period of 15 minutes. Stirring was continued at -20°C for a further 20 minutes. 4-Dimethylaminopyridine (216mg) was added and the solution stirred at ice-bath temperature for one hour. The reaction solution was then diluted with dichloromethane and washed with 5% citric acid solution, water, saturated sodium hydrogen carbonate solution, water, brine, and dried over anhydrous magnesium sulphate. Removal of the solvent at reduced pressure afforded the title compound (24; XXIV R^{x} = pMB) as an oil, νₘₐₓ (CH₂Cl₂) 1790, 1725, 1685cm⁻¹. This material was used in the next stage of the synthesis without further purification.

### EXAMPLE 25b

### (3S,4R) 3-Bromo-4-formylthio-1-(p-methoxybenzyloxyoxalyl)azetidin-2-one (Step E2; Compound XXIII)

The crude (3S,4R) 3-bromo-4-formylthio-1-(1-p-methoxybenzyloxycarbonyl-2-methylprop-1-enyl)azetidin-2-one (24), prepared as described in Example 25a was dissolved in ethyl acetate (100ml) and cooled to -78°C. Ozonised oxygen was bubbled through the stirred solution for 2 hours. The reaction solution was then diluted with toluene (100ml) and washed with cold sodium bisulphite solution (adjusted to pH 7), water, brine and dried over anhydrous magnesium sulphate. The resulting solution contained the title oxalimide (23, XXIII R^{x} = pMB) and was then evaporated to small volume (approx. 20ml).

### EXAMPLE 25c

### p-Methoxybenzyl (5R,6S) 6-bromopenem-3-carboxylate (Steps D4 and D5; Compound IC)

Trimethyl phosphite (0.835ml) was added to the solution from Example 25b, which was then heated at 95°C, under an atmosphere of argon, for 30 min. After cooling the solution was applied directly to a column of silica gel gel (30g). Elution of the column with a gradient of 10-30% ethyl acetate/hexane afforded the title bromopenem ester (15; IC R³ = H, R^{x} = pMB) (159mg) as a colourless oil which crystallised from diethyl ether.

### EXAMPLE 26

### Sodium (5R,6S) 6-bromopenem-3-carboxylate (Compound IC)

Anhydrous aluminium chloride (90mg) was added, in one portion to a stirred solution of the brompenem ester (15; IC R³ = H, R^{x} = pMB) (100mg) in dry dichloromethane (0.4ml) and dry redistilled anisole (1.6ml) at -40°C. After stirring at -40°C for 20 minutes the mixture was poured into a mixture of methyl isobutyl ketone (MIBK) (5ml) and water (5ml). The pH of the vigorously stirred mixture was adjusted to 2.0 using 1M hydrochloric acid. The mixture was filtered through Kieselguhr, the residue being washed with MIBK (3ml). The organic layer was separated and the aqueous layer was re-extracted with MIBK (3ml). The combined organic layers were washed with water (3ml) and repartitioned with water (5ml). The pH of the vigorously stirred mixture was adjusted to 7.0 using 0.1M sodium hydroxide solution. The aqueous layer was separated and the organic layer was re-extracted with water (3ml). The combined aqueous layers were concentrated to low volume and chromatographed on Biogel P2 (Trade name) eluting with water. The appropriate fractions were freeze-dried to give the title sodium salt (25; IC R³ = H, R^{x} = Na) (52mg) as a pale pink solid, [α]_{D}¹⁹ +104.8° (c 1 in H₂O); λₘₐₓ (H₂O) 278nm (εₘ 4010); νₘₐₓ (KBr) 3700-2700, 1782, 1615, 1558cm⁻¹; δₚₚₘ (D₂O) 5.46 (1H, dd, J 1.3 and 0.8Hz), 5.88 (1H, d, J 1.3Hz), 7.12 (1H, d, J 0.8Hz).

### EXAMPLE 27

### p-Methoxybenzyl (5R) 6-bromo-6-[hydroxy(1-methyl-1,2,3-triazol-4-yl)methyl]penem-3-carboxylate (Step F1; Compound ID)

Lithium bis(trimethylsilyl)amide (0.35ml of a 1M solution in hexane) was added to a stirred solution of the bromopenem (15; IC R³ = H, R^{x} = pMB) (100mg) in dry THF (4ml) at -76°C under dry argon. After 3 minutes at -76°C the mixture was treated with a solution of 1-methyl-1,2,3-triazole-4-carboxaldehyde (Hüttel et al, Annalen, 1947, 558, 34) (39mg) in dry THF (1.5ml). The mixture was stirred for 1 minute, treated with 5% citric acid (lml), and allowed to reach room temperature. The mixture was diluted with ethyl acetate (15ml) and washed with brine (1ml), saturated NaHCO₃ solution (1ml), and brine (3 x 1ml). The dried (MgSO₄) organic layer was evaporated and the residue chromatographed on silica gel eluting with ethyl acetate/hexane mixtures to give two fractions. The less polar fraction contained a single isomer (Isomer A) of the title bromohydrin (26; 1B R³ = H, R^{x} = pMB, R¹² = 1-methyl-1,2,3-triazol-4-yl, ) (37mg), a solid, νₘₐₓ (CHCl₃) 3600-3100, 1795, 1710cm⁻¹; δₚₚₘ (CDCl₃) 3.2-3.7 (1H, broad signal), 3.80 (3H, s), 4.10 (3H, s), 5.16 and 5.24 (2H, ABq, J 12.0Hz), 5.72 br (1H, s), 6.14 (1H, s), 6.89 (2H, d, J 8.7Hz), 7.34 (s) and 7.35 (d, J 8.7Hz) together 3H, 7.69 (1H, d, J 0.4Hz). The more polar fraction contained a 2:1:1 mixture of three isomers (Isomers B, C and D) of the title bromohydrin (26) (19mg), a solid, νₘₐₓ (CHCl₃) 3600-3100, 1800, 1710cm⁻¹; δₚₚₘ (CDCl₃) 3.81 (3H, s), 4.08, 4.11, and 4.13 (3H, each s), 5.16-5.27 (2H, m), 5.38 (¼H, s), 5.48 (¼H, s), 5.53 (½H, s), 6.11 (½H, s), 6.35 (¼H, s), 6.39 (¼H, s), 6.87-6.93 (2H, m), 7.25-7.37 (3H, m), 7.70 (¼H, s), 7.79 (½H, s), 7.80 (¼H, s).

### EXAMPLE 28

### p-Methoxybenzyl (5R) 6-bromo-6-[hydroxy(1-methyl-1,2,3-triazol-4-yl)methyl]penem-3-carboxylate (Step F1; Compound IB)

A solution of lithium bis(trimethylsilyl)amide (0.75ml of a 0.93M solution in hexane) was diluted with dry THF (lml) and added, in one portion, to a vigorously stirred solution of the bromopenem (15; IC R³ = H, R^{x} = pMB) (250mg) in dry THF (10ml) at -76°C under dry argon. After 15-20 seconds the vigorously stirred mixture was treated, in one portion, with a solution of 1-methyl-1,2,3-triazole-4-carboxaldehyde (90mg) in dry THF (2ml). After a further 15-20 seconds, the mixture was treated with a solution of glacial acetic acid (0.2ml) and water (0.2ml) in THF (lml). The mixture was diluted with ethyl acetate (20ml) and washed with urine (2ml), saturated NaHCO₃ (2ml), and brine (3 x 2ml). The dried (MgSO₄) organic layer was evaporated and chromatographed to give the less polar Isomer A (131mg) and a 1:1:1 mixture of Isomers B, C, and D (90mg) of the title bromohydrin (26) (see Example 27).

### EXAMPLE 29

### p-Methoxybenzyl (5R) 6-[acetoxy(1-methyl-1,2,3-triazol-4-yl)methyl]-6-bromopenem-3-carboxylate (Compound ID)

A mixture of bromohydrins (26) (an approximately 1:1:1 mixture of Isomers B, C, and D) (202mg) was stirred with acetic anhydride (48µl), triethylamine (71µl) and 4-dimethylaminopyridine (4mg) in dichloromethane (25ml) at room temperature for 1 hour. The solution was diluted with dichloromethane and washed with dilute citric acid, water and brine. The dried (MgSO₄) organic layer was evaporated and the residue chromatographed on silica gel eluting with methyl acetate/ hexane mixtures to give the title acetate (27; ID R³ = H, R^{x} = pMB, R¹² = 1-methyl-1,2,3-triazol-4-yl, Z = CH₃CO₂) (146mg), a mixture of isomers B, C, and D, as a colourless oil, νₘₐₓ (CH₂Cl₂) 1805, 1755, 1715cm⁻¹.

### EXAMPLE 30

### p-Methoxybenzyl (5R) 6-(1-methyl-1,2,3-triazol-4-yl-methylene)penem-3-carboxylate (Step F2)

A stirred, ice bath cooled, solution of the bromohydrin (26) (Isomer A) (21Gmy) in dry THF (4ml) was treated with acetic anhydride (55mg), triethylamine (54mg) and 4-dimethylaminopyridine (5mg). The ice bath was removed and the mixture was stirred for 1 hour [t.l.c. showed the formation of the less polar acetate (27, Isomer A)]. The stirred mixture was re-cooled in an ice bath and treated with glacial acetic acid (0.2ml) and zinc dust (216mg). The ice bath was removed and the mixture was stirred vigorously for 20 minutes. The mixture was filtered through Kieselguhr and the residue washed with ethyl acetate (2 x 4ml) The combined filtrates were diluted with ethyl acetate (10ml) and washed with brine (1ml), saturated NaHCO₃ solution (1ml), and brine (3 x 1ml). The dried (MgSO₄) organic layer was evaporated and the residue chromatographed on silica gel eluting with ethyl acetate/hexane mixtures to give two fractions. The less polar fraction contained the (E)-isomer of the title penem ester (28; XB R³ = H, R^{x}= pMB, R¹² = 1-methyl-1,2,3-triazol-4-yl) (19mg), a solid, m.p. 181-183°C (clusters of needles ex ethyl acetate/hexane); [α]_{D}²⁰ -244° (c 1 in CHCl₃); λₘₐₓ (EtOH) 296 (εₘ 19,400) and 224nm (18,500); νₘₐₓ (CHCl₃) 1775, 1715, 1685 sh cm⁻¹; δₚₚₘ (CDCl₃) 3.81 (3H, s), 4.14 (3H, s), 5.19 and 5.24 (2H, ABq, J 12.6Hz), 6.42 (1H, d, J 0.7Hz), 6.90 (3H, s and d, J 8.7Hz), 7.33 (s) and 7.36 (d, J 8.7Hz) together 3H, 8.74 (1H, s). (Found C, 56.5; H, 4.0; N, 14.6; S, 8.2%. C₁₈H₁₆N₄O₄S requires C, 56.2; H, 4.2; N, 14.6; S, 8.3%). The more polar fraction contained the (Z)-isomer of the title penem ester (29; XA symbols as for 28) (100mg), a solid, m.p. 183-184°C (clusters of small needles ex ethyl acetate/hexane); [α]_{D}²⁰ +399° (c 1 in CHCl₃); λₘₐₓ (EtOH) 285 (εₘ 25,300) and 226nm (18,040); νₘₐₓ (CHCl₃) 1780, 1710cm⁻¹; δₚₚₘ (CDCl₃) 3.81 (3H, s), 4.14 (3H, s), 5.17 and 5.25 (2H, ABq, J 12.0Hz), 6.62 (1H, d, J 1.1Hz), 6.89 (2H, d, J 8.7Hz), 7.03 (1H, d, J 1.1Hz), 7.27 (1H, s), 7.36 (2H, d, J 8.7Hz), 7.70 (1H, s). (Found: C, 56.5; H, 4.2; N, 14.6; S, 8.1%. C₁₈H₁₆N₄O₄S requires C, 56.2; H, 4.2; N, 14.6; S, 8.3%).

### EXAMPLE 31

### p-Methoxybenzyl (5R) 6-(1-methyl-1,2,3-triazol-4-ylmethylene)penem-3-carboxylate (Step F2)

Using the method described in Example 30 the mixture of isomers of the bromohydrins (26) (B:C:D, approximately 1:1:1) (90mg) gave the (E)-isomer of the title penem ester (28) (13mg) and the (Z)-isomer of the title penem ester (29) (34mg).

### EXAMPLE 32

### p-Methoxybenzyl (5R) 6-(1-methyl-1,2,3-triazol-4-yl-methylene)penem-3-carboxylate (Step F2)

A stirred solution of the bromohydrin (26) (Isomer A) (90mg) in dry THF (2ml) was cooled in an ice bath and treated with triethylamine (23mg) and methane sulphonyl chloride (26mg). The ice bath was removed and the mixture was stirred for 30 minutes [t.l.c. showed only partial conversion to the less polar mesylate (30; ID R³ = H, R^{x} = pMB, R¹² = 1-methyl-1,2,3-triazol-4-yl, SO₂Me)]. The mixture was treated with a further two portions of triethylamine (2 x 9mg) and methane sulphonyl chloride (2 x 11mg). After stirring at room temperature for a further 45 minutes [t.l.c. showed complete conversion to the less polar mesylate (30)] the mixture was diluted with ethyl acetate (10ml) and washed with 5% citric acid (1ml), brine (1ml), saturated NaHCO₃ solution (1ml) and brine (3 x 1ml). The dried (MgSO₄) organic layer was evaporated to give the crude mesylate (30) as a solid. The crude mesylate was redissolved in THF (2ml) and glacial acetic acid (0.2ml), cooled in an ice bath, and treated with zinc dust (90mg). The ice bath was removed and the mixture was stirred vigorously for 15 minutes. Work up as in Example 30 gave the (E) isomer of the title penem ester (28) (1mg) and the (Z)-isomer of the title penem ester (29) (61mg).

### EXAMPLE 33

### Sodium (5R) 6-(Z)-(1-methyl-1,2,3-triazol-4-yl-methylene)penem-3-carboxylate

Anhydrous aluminium chloride (96mg) was added, in one portion, to a stirred solution of the penem ester (29) (110mg) in dry dichloromethane (0.6ml) and dry redistilled anisole (2.4ml) at -40°C. After stirring at -40°C for 20 minutes the mixture was poured into a mixture of methyl isobutyl ketone (MIBK) (5ml) and water (5ml). The pH of the vigorously stirred mixture was adjusted to 2.0 using 1M hydrochloric acid. The mixture was filtered through Keiselguhr the residue being washed with MIBK (5ml). The organic layer was separated and the aqueous layer was re-extracted with MIBK (2 x 3ml). The combined organic layers were washed with water (3ml) and repartitioned with water (5ml). The pH of the vigorously stirred mixture was adjusted to 7.0 using 0.1M sodium hydroxide solution. The aqueous layer was separated and the organic layer was re-extracted with water (3ml). The combined aqueous layers were concentrated to low volume and chromatographed on Biogel P2 (Trade name) eluting with water. The appropriate fractions were freeze-dried to give the title sodium salt (31; XA R³ = H, R^{x} = Na, R¹² = 1-methyl-1,2,3-triazol-4-yl) (39mg) as a yellow solid, δₚₚₘ (D₂O) 4.09 (3H, s), 6.54 (1H, d, J 0.7Hz), 7.00 (1H, s), 7.14 (1H, s), 8.11 (1H, s).

### EXAMPLES 34 - 48

### p-Methoxybenzyl (5R) 6-(1-methyl-1,2,3-triazol-4-ylmethylene)penem-3-carboxylate (Steps F1 and F2)

### Example 34

A solution of lithium bis(trimethylsilyl)amide (0.75ml of a 0.93M solution in hexane) was diluted with dry THF (1ml) and added, in one portion, to a vigorously stirred solution of the bromopenem (15; IC R³ = H, R^{x} = pMB) (250mg) in dry THF (8ml) at -76°C under dry argon. After 15-20 seconds the vigorously stirred mixture was treated, in one portion, with a solution of 1-methyl-1,2,3-triazole-4-carboxaldehyde (90mg) in dry THF (2ml). After a further 15-20 seconds the resulting solution, containing a mixture of the lithium salts of the bromohydrins (ID, R³ = H, R^{x} = pMB, R¹² = 1-methyl-1,2,3-triazol-4-yl, Z = OLi), was treated, in one portion with a solution of acetic anhydride (0.20ml) in dry THF (1ml). The cooling bath was removed and the mixture was stirred for 10 minutes. The resulting solution, containing a mixture of the acetates (ID, R³ = H, R^{x} = pMB, R¹² = 1-methyl-1,2,3-triazol-4-yl, Z =OCOCH₃), was treated with glacial acetic acid (0.20ml) and zinc dust (250mg). After stirring without cooling for 15 minutes the mixture was filtered through Kieselguhr and the residue was washed with ethyl acetate (2 x 5ml). Work-up as for Example 30 then gave the (E)-isomer (28) (39mg) and the (Z)-isomer (29) (113mg) of the title penem (data consistent with Example 30).

### Example 35

A repeat of Example 34 in which the initial reaction temperature was -40°C gave the (E)-isomer (28) (13%) and the (Z)-isomer (29) (36%) of the title penem.

### Example 36

A repeat of Example 34 carried out using dry 1,2-dimethoxyethane as solvent gave the (E)-isomer (28) (14%) and the (Z)-isomer (29) (40%) of the title penem.

### Example 37

A repeat of Example 34 in which the lithium bis(trimethylsilyl)amide was added to the bromopenem in a mixture of dry THF and dry dimethylformamide (DMF) 10:1) gave the (E)-isomer (28) (18%) and the (Z)-isomer (29) (50%) of the title penem.

### Example 38

A repeat of Example 37 in which the dry DMF was replaced by, 1,3-dimethyl-3,4,5,6-tetrahydro-2-(1H)pyrimidinone gave the (E)-isomer (28) (13%) and the (Z)-isomer (29) (42%) of the title penem.

### Example 39

A repeat of Example 37 in which the dry THF was replaced by dry toluene gave the (E)-isomer (28) (14%) and the (Z)-isomer (29) (38%) of the title penem.

### Example 40

A repeat of Example 34 in which the initial reaction temperature was -90°C to -95°C and the aldehyde was added as a solution in dry DMF gave the (E)-isomer (28) (16%) and the (Z)-isomer (29) (40%) of the title penem.

### Example 41

A repeat of Example 34 in which the aldehyde was added as a solution in dry DMF gave the (E)-isomer (28) (15%) and the (Z)-isomer (29) (40%) of the title penem.

### Example 42

A repeat of Example 41 in which the lithium bis(trimethylsilyl)amide was replaced by lithium 2,2,6,6-tetramethylpiperidide gave the (E)-isomer (28) (12%) and the (Z)-isomer (29) (31%) of the title penem.

### Example 43

A repeat of Example 41 in which the lithium bis(trimethylsilyl)amide was replaced by lithium diisopropylamide gave the (E)-isomer (28) (15%) and the (Z)-isomer (29) (41%) of the title penem.

### Example 44

A repeat of Example 34 in which the lithium bis(trimethylsilyl)amide was mixed with N,N,N',N'-tetramethylethylenediamine (1 equivalent) in dry THF prior to addition to the bromopenem (15) gave the (E)-isomer (28) (13%) and the (Z)-isomer (29) (39%) of the title penem.

### Example 45

A repeat of Example 34 in which the lithium bis(trimethylsilyl)amide was replaced by sodium bis (trimethylsilyl)amide gave the (E)-isomer (28) (3%) and the (Z)-isomer (29) (11%) of the title penem.

### Example 46

A repeat of Example 34 in which the lithium bis(trimethylsilyl)amide was replaced by lithium diphenylamide gave the (E)-isomer (28) (19%) and the (Z)-isomer (29) (46%) of the title penem.

### Example 47

A repeat of Example 46 in which the initial reaction temperature was -40°C gave the (E)-isomer (28) (18%) and the (Z)-isomer (29) (45%) of the title penem.

### Example 48

A repeat of Example 46 in which the initial reaction temperature was -20°C gave the (E)-isomer (28) (17%) and the (Z)-isomer (29) (42%) of the title penem.

### EXAMPLE 49

### p-Methoxybenzyl (5R) 6-(1-methyl-1,2,3-triazol-4-ylmethylene)penem-3-carboxylate (Steps F1 and F2)

A solution of lithium bis(trimethylsilyl)amide (1.50ml of a 0.93M solution in hexane) was diluted with dry THF (2ml) and added, in one portion, to a stirred solution of the bromopenem (15; IC, R³ = H, R^{x} = pMB) (500mg) in dry THF (16ml) at -76°C for 15-20 seconds the stirred mixture was treated, in one portion, with a solution of 1-methyl-1,2,3-triazole-4-carboxaldehyde (180mg) in dry THF (4ml). After a further 15-20 seconds the resulting solution, containing a mixture of the lithium salts of the bromohydrins (ID, R³ = H, R^{x} = pMB, R¹² = 1-methyl-1,2,3-triazol-4-yl, Z = OLi) was treated, in one portion with a solution of redistilled trifluoroacetic anhydride (0.50ml) in dry THF (2ml). The cooling bath was removed and the mixture was stirred for 5 minutes. The resulting solution containing a mixture of the trifluoroacetates (ID, R³ = H, R^{x} = pMB, R¹² = 1-methyl-1,2,3-triazol-4-yl, Z = OCOCF₃) was treated with glacial acetic acid (0.5ml) and zinc dust (500mg). The mixture was stirred vigorously and treated with further portions of glacial acetic acid (0.5ml) and zinc dust (500mg). After a further 15 minutes the mixture was worked up as for Example 34 to give the (E)-isomer (28)(69mg) and the (Z)-isomer (29) (131mg) of the title penem.

### EXAMPLE 50

### p-Methoxybenzyl (5R) 6-(1-methyl-1,2,3-triazol-4-ylmethylene)penem-3-carboxylate (Steps F1 and F2)

A solution of diphenylamine (0.23g, 1.36 mmol) in THF (25ml) at -10°C under argon was treated with n-butyllithium (0.93ml x 1.5M in hexane, 1.40 mmol). After 10 min at 20°C and 5 min at -70°C, there were added in fairly rapid succession:
(i) bromopenem (15; IC, R³ = H, R^{x} = pMB) (0.50g, 1.35 mmol) in THF (5ml),
(ii) 1-methyl-1,2,3-triazole-4-carboxaldehyde (0.18g, 1.62 mmol) in THF (5ml),
(iii) acetic anhydride (0.40ml, 4.30 mmol) in THF (4ml).

The cooling bath was then removed and after 10 min, ethyl acetate and aqueous sodium bicarbonate were added. The separated organic layer was washed twice with brine, dried (MgSO₄) and evaporated. The resulting oil was dissolved in DMF (20ml) and ammonium chloride (0.22g, 4.00 mmol), N,N,N', N'-tetramethylethylenediamine dihydrochloride (0.19g, 1.00 mmol) and zinc powder (0.26g, 4.00 mmol) were added. After 1h stirring at 20°C, ethyl acetate and 0.2M aqueous citric acid were added. The separated organic layer was washed with brine, with aqueous sodium bicarbonate, and twice more with brine, and then dried (MgSO₄) and evaporated. The residue was chromatographed (silica, 50% to 90% ethyl acetate in hexane) to give first diphenylamine, then the E-isomer of the title compound (28) (0.042g, 8%) and then and Z-isomer (29) (0.33g, 64%), identical to samples obtained previously.

### EXAMPLE 51

### p-Methoxybenzyl (5R,6S) 6-bromo-2-methylpenem-3-carboxylate (Step A9; Compound IC)

A solution of the phosphorane (7) (5.0g) in toluene (50ml) was heated at 90 - 100°C under nitrogen for 4 hours. The mixture was concentrated and chromatographed on silica gel eluting with ethyl acetate/hexane mixtures to give the title penem (32; IC, R³ = CH₃, R^{x} = pMB) (1.15g) as a solid, mp 131 - 132°C (rods ex ethyl acetate/hexane), [α_{D}²⁰ 86.7°(c1.0, CHCl₃), λₘₐₓ (EtOH)205 (ε 10850), 274 (ε 5250), 280 (ε 5350), 307 (ε 5300), νₘₐₓ (CHCl₃) 1800, 1710cm⁻¹, δₚₚₘ (CDCl₃) 2.35 (3H,s), 3.81 (3H,s), 5.06(1H, d, J1.4Hz), 5.17 and 5.23 (2H, ABq, J 12.0Hz), 5.60 (1H, d, J 1.4Hz), 6.86-6.92 (2H, m), 7.34-7.40 (2H, m), (Found: C, 46.6; H, 3.7; N, 3.6; S, 8.3; Br, 20.9%. C₁₅H₁₄NBrO₄S requires: C, 46.9; H, 3.7; N, 3.6; S, 8.3; Br, 20.8%), M⁺ m/e 383.

### EXAMPLE 52

### p-Methoxybenzyl (5R) 2-methyl-6-(1-methyl-1,2,3-triazol-4-ylmethylene)penem-3-carboxylate (Steps F1 and F2)

A solution of n-butyllithium (1.71ml of 1.49M in hexane) was added to a stirred solution of diphenylamine (430mg) in dry THF (2ml) of -20°C under dry argon. The stirred mixture was allowed to attain room temperature during 10 minutes and was diluted to 5.0ml using dry THF. An aliquot (1.00ml) of the lithium diphenylamide solution prepared above was added, one portion, to a stirred solution of the bromopenem (32; IC, R³ = CH₃, R^{x} = pMB) (192mg) in dry THF (4ml) at -76°C under dry argon. After 2 minutes at -76°C the stirred mixture was treated, in one portion, with a solution of 1-methyl-1,2,3-triazole-4-carboxaldehyde (67mg) in dry THF (1.5ml). After a further 15-20 seconds the resulting solution, containing a mixture of the lithium salts of the bromohydrins (ID; R³ = CH₃, R^{x} = pMB, R¹² = 1-methyl-1,2,3-triazol-4-yl, Z = OLi), was treated, in one portion, with a solution of acetic anhydride (0.2ml) in dry THF (0.5ml). The cooling bath was removed and the mixture was stirred for 5 minutes. The resulting solution, containing a mixture of the acetates (ID, R³ = CH₃, R^{x} = pMB, R¹² = 1-methyl-1,2,3-triazol-4-yl, Z = OCOCH₃), was treated with glacial acetic acid (0.2ml) and zinc dust (192mg). The mixture was stirred without cooling for 20 minutes and worked up as for Example 30 to give two fractions. The less polar fraction contained the (E)-isomer of the title penem (33; XB, R³ = CH₃, R^{x} = pMB, R¹² = 1-methyl-1,2,3-triazol-4-yl) (49mg), a solid, m.p. 178-179°C (micro-needles ex ethyl acetate/hexane); [α]_{D}²⁰ -346.9°(cl in CHCl₃); λₘₐₓ(EtOH) 224(εₘ 16,600) and 294nm (19000); νₘₐₓ(CHCl₃) 1770, 1705cm⁻¹; δₚₚₘ (CDCl₃) 2.35 (3H,s), 3.81(3H,s), 4.14 (3H,s), 5.23 (2H,s), 6.21 (1H,d,J0.7Hz), 6.84(1H,s), 6.90 (2H,d, J8.7Hz), 7.38 (2H, d, J8.7Hz), 8.74(1H,s). (Found: C, 57.5; H, 4.6; N, 14.0; S, 7.7; M⁺, 398.1043. C₁₉H₁₈N₄O₄S requires C, 57.3; H, 4.6; N, 14.1; S, 8.0%; M, 398.1049). The more polar fraction contained the (Z)-isomer of the title penem (34; XA, symbols as for 33) (109mg), a solid, m.p. 116-117°C (nuggets ex ethyl acetate/hexane; [α]_{D}²⁹ + 365.7° (c1.0 in CHCl₃); λₘₐₓ (2% CHCl₃/EtOH) 282 nm (εₘ 23,050); νₘₐₓ (CHCl₃) 1775, 1700 cm⁻¹; δₚₚₘ (CDCl₃) 2.33 (3H,s), 3.80 (3H,s),4.12 (3H,s), 5.17 and 5.27 (2H, ABq, J12.2Hz), 6.42 (1H,d, J1.1Hz), 6.89 (2H,d,J8.7Hz), 6.99 (1H,d,J1.1Hz), 7.40 (2H,d, J8.7Hz). (Found: C, 57.5; H, 4.5; N, 14.1; S, 7.5; M⁺, 398.1064. C₁₉H₁₈N₄O₄S requires C, 57.3; H, 4.6; N, 14.1; S, 8.0%; M, 398.1050).

### EXAMPLE 53

### Sodium (5R) 2-methyl-(Z)-6-(1-methyl-1,2,3-triazol-4-ylmethylene)penem-3-carboxylate (Deprotection of XA)

A solution of the penem ester (34) (199mg) in dry dichloromethane (2ml) was added, dropwise over 2 minutes, to a stirred solution of anhydrous aluminium chloride (166mg) in a mixture of dry redistilled anisole (1.6ml) and dry dichloromethane (0.4ml) at -40°C. After stirring at -40°C for 10 minutes the mixture was treated with an aqueous solution of disodium hydrogen orthophosphate (17ml of 0.5M). The mixture was stirred without cooling for 10 minutes, filtered through Kieselguhr, and the residue was washed with water. The combined filtrates were partitioned with MIBK (50ml) and the pH of the vigorously stirred mixture was adjusted to 2.0 using 1M sulphuric acid. The organic layers were washed with water (2 x 5ml), repartitioned with water (20ml), and the pH of the vigorously stirred mixture was carefully adjusted to 7.0 using 0.1M sodium hydroxide solution. The aqueous layer was separated, evaporated to low volume, and chromatographed on Diaion HP20SS (Trade mark) eluting with water. The appropriate fractions were concentrated and freeze-dried to give the title sodium salt (35; XA, R³ = CH₃, R^{x} = Na, R¹² = 1-methyl-1,2,3-triazol-4-yl) (100mg) as a pale yellow solid, [α]_{D}²⁰ + 524° (c0.25 in H₂O); λₘₐₓ (H₂O) 280 (εₘ 20,400) and 370 nm (1,900); νₘₐₓ (KBr) 3700-2700, 1750, 1689, 1600, 1577cm⁻¹; δₚₚₘ (D₂O) 2.24 (3H,s), 4.11 (3H,s), 6.41 (1H,d,J0.7Hz), 7.17 (1H, d, J0.7Hz), 8.12 (1H,s).

### EXAMPLE 54

### Sodium (5R) 2-methyl-(E)-6-(1-methyl-1,2,3-triazol-4-ylmethylene)penem-3-carboxylate (Deprotection of XB)

A solution of the penem ester (33) (100mg) in dry dichloromethane (1ml) was added, dropwise over 2 minutes, to a stirred solution of anhydrous aluminium chloride (83mg) in a mixture of dry redistilled anisole (1.6ml) and dry dichloromethane (0.4ml) at -40°C. After stirring at -40°C for 10 minutes the mixture was treated with an aqueous solution of disodium hydrogen orthophosphate (8.5ml of 0.5M) and worked up as for Example 53 to give the title sodium salt (36; XB,R³ = CH₃, R^{x} = Na, R¹² = 1-methyl-1,2,3-triazol-4-yl) (42mg) as a pale yellow solid, [α]_{D}²⁰ -506° (c1.0 in H₂O); λₘₐₓ (H₂O) 283 (εₘ 16,400) and 366 nm (2,700); νₘₐₓ (KBr)3700-2700, 1746, 1686, 1604, 1585cm⁻¹; δₚₚₘ (D₂O) 2.24 (3H,s), 4.13 (3H,s), 6.27(1H,s), 6.87 (1H,s), 8.67(1H,s).

### EXAMPLE 55

### p-Methoxybenzyl (5R) 6-1-methylbenzotriazol-6-ylmethylene)penem-3-carboxylate (Steps F1 and F2)

Diphenylamine (0.23g) was dissolved in dry tetrahydrofuran (THF) (25ml) and the solution cooled to -20°C under an atmosphere of argon. A solution of n-butyl lithium in hexane (0.93ml of a 1.48M solution) was added with stirring. The cooling bath removed and the mixture stirred for 10 minutes. The lithium diphenylamide solution at -70°C was then treated with p-methoxybenzyl (5R,6S) 6-bromopenem-3-carboxylate (15) (0.5g) dissolved in dry THF (5ml), 1-methylbenzotriazole-6-carboxaldehyde (0.25g) in dry THF (5ml) followed by acetic anhydride (0.4ml) in rapid succession with thorough mixing. The cooling bath was removed and the reaction mixture stirred to ambient temperature during 0.5 hour. Finely divided zinc dust (0.5g) and glacial acetic acid (0.4ml) were then added and the reaction continued for a further 1.0 hour. The reaction was diluted with ethyl acetate (70ml) and the zinc removed by filtration on a celite pad. The filtrate was washed successively with saturated brine solution (1 x 30ml), saturated sodium bicarbonate solution (2 x 30ml), saturated brine solution (1 x 30ml), dried (MgSO₄) and evaporated. The residue was subjected to a silica column (10g) eluting with hexane: ethyl acetate 1:1 and the title penem (37; XA + XB, R³ = H, R^{x} = pMB, R¹² = 1-methylbenzotriazol-6-yl) isolated as an inseparable mixture of isomers (Z:E, 1:1) (0.24g).νₘₐₓ (CHCl₃) 1785, 1715 and 1615cm⁻¹; δₚₚₘ (CDCl₃) 3.82 (3H,s), 4.54 (1.5H,s), 4.56 (1.5H,s), 5.15-5.30 (2H,m), 6.43 (0.5H,s), 6.68 (0.5H,s), 6.76 (0.5H,s) 6.85-7.0 (2.5H,m), 7.21-7.47 (m), 7.85-7.95(m), 8.2-8.3 (m) and 8.85 (d, J 2.0Hz) together 6H; (Found, M⁺434).

### EXAMPLE 56

### Sodium (5R) 6-(1-methylbenzotriazol-6-ylmethylene) penem-3-carboxylate (Deprotection of XA + XB)

p-Methoxybenzyl (5R) 6-(1-methylbenzotriazol-6-ylmethylene)penem-3-carboxylate (37) (180mg) was deprotected and converted to the sodium salt using the aluminium trichloride procedure described in Example 53. The product (38; XA + XB, R³ = H, R^{x} = Na, R¹² = 1-methylbenzotriazol-6-yl) was isolated as a mixture of isomers (Z:E, 1:1) (30mg) after HP20 column chromatography and lyophilisation. νₘₐₓ (KBr) 1760 and 1610cm⁻¹; δ (D₂O) 4.45 (3H, 2xs), 6.38 (0.5H,s) 6.55 (0.5H,s), 6.71 (0.5H,s), 6.86 (0.5H,s), 7.02 (0.5H,s), 7.11 (0.5,d,J 9Hz), 7.18(0.5H,s), 7.57 (0.5H,s), 7.64 (0.5H,d,J 9Hz), 7.72 (0.5H,d,J 9Hz), 7.77(0.5H,d,J 9Hz), 7.91(0.5H,s).

### EXAMPLE 57

### (5R) p-Methoxybenzyl-(Z)-6-(1-acetyl-5-ethoxycarbonyl-2-pyrazolin-3-ylmethylene)penem -3-carboxylate (Steps F1 + F2)

n-Butyl lithium (0.925ml of a 1.49M solution in hexane) was added to a solution of diphenylamine (233mg) in dry tetrahydrofuran (25ml) at -20°C under argon. The cooling bath was removed for 10 minutes and the temperature was then dropped to -76°C. During rapid stirring the following ingredients were added sequentially, (5R,6S) p-methoxybenzyl 6-bromopenem-3-carboxylate (15) (0.5g) in dry tetrahydrofuran (5ml), 1-acetyl-5-ethoxycarbonyl-3-formyl-2-pyrazoline (A.F. Noels, J.N. Braham, A.J. Hubert and Ph. Teyssie., Tetrahedron 34, 3495, 1978) (0.48g) in dry tetrahydrofuran (3ml), and finally neat acetic anhydride (0.4ml). The cooling bath was removed and stirring continued for 10 minutes before being treated with glacial acetic acid (0.4ml) and finely powdered zinc (0.5g). The resulting suspension was stirred for 30 minutes at ambient temperature, then diluted with ethyl acetate and filtered through celite before being washed with saturated aqueous sodium hydrogen carbonate. Evaporation of solvent and chromatography of the residue upon silica eluting with n-hexane/ethyl acetate (1:1) gave the title ester (39; XA, R₃ = H, R^{x} = pMB, R¹² = 1-acetyl-5-ethoxycarbonyl-2-pyrazolin-3-yl) as a gum (0.204g, 30% yield), as a mixture (1.6:1) diastereoisomers (5-position of pyrazoline ring). λₘₐₓ (EtOH) 225 (ε19 100) and 316nm (ε 22,000). νₘₐₓ (KBr) 1784, 1744, 1711 and 1670cm⁻¹, δ(CDCl₃) 1.22 - 1.33 (m, 2x3H, isomers A and B), 2.38 and 2.39 each (3H,s, isomers A and B), 3.02 and 3.37 (2H, ABX, J 7.5, 12.8 and 18.1Hz, isomer B), 3.04 and 3.31 (2H, ABX, J 6.5, 12.8 and 17.9 Hz, isomer A), 3.82 and 3.83 each (3H, s, isomers A and B), 3.19 - 4.3 (2x2H, m, isomers A and B), 4.93 (1H, dd, J 6.5 and 12.8 Hz, isomer A), 4.94 (1H, dd, J 7.5 and 12.8 Hz, isomer B), 5.18 and 5.24 (2x2H, ABq, J 12 Hz, isomers A and B), 6.37 (1H, d, J 1Hz, isomer B), 6.38 (1H, d, J 1Hz, isomer A), 6.67 (1H, s, isomer B), 6.74 (1H, s, isomer A), 6.87-6,94 (2x2H,m, aromatics, isomers A and B), 7.26 (2x1H, 2, isomers A and B), 7.32-7.39 (2x2H,m, aromatics, isomers A and B).

### EXAMPLE 58

### (5R) Sodium (Z)-6-(1-acetyl-5-ethoxycarbonyl-2-pyrazolin-3-ylmethylene)penem-3-carboxylate (Deprotection of XA)

A solution of (5R) p-methoxybenzyl (Z)-6-(1-acetyl-5-ethoxycarbonyl-2-pyrazolin-3-ylmethylene)penem-3-carboxylate (39) (200mg) in dry dichloromethane (2ml) was added dropwise to a stirred solution of anhydrous aluminium chloride (100mg) in a mixture of redistilled anisole (5ml) and dichloromethane (1ml) at -40°C under an atmosphere of argon. After stirring at -40°C for 10 minutes the mixture was treated with an aqueous solution of disodium hydrogen orthophosphate (9ml of a 0.5M solution). The mixture was allowed to reach room temperature and the pH of the solution was adjusted to 7.0. The reaction mixture was then filtered through Celite. The aqueous solution was separated, concentrated at reduced pressure and chromatographed over Diaion HP 20SS (Trade mark), eluting with a gradient of 0% to 10% acetone/water. The column fractions were monitored by u.v. spectroscopy. The appropriate fractions were combined and freeze-dried to yield the title sodium salt (40; XA, R³ = H, R^{x} = Na, R¹² as for 39) (49mg) as a pale yellow solid, λₘₐₓ (H₂O) 306nm (ε 18688), 210nm (ε 11640); νₘₐₓ (KBr) 2800-3700, 1750, 1674, 1604, 1551cm⁻¹; δ(D₂O) 1.27 (2x3H,t, isomers A and B), 2.40 (2x3H,s, isomers A and B), 3.16 (1H, ABX, J 6.2 and 18.3 Hz, isomer A), 3.29 (1H, ABX, J 6.2 and 18.2Hz, isomer B), 3.51 (1H, ABX, J 12.4 and 18.2Hz, isomer B), 3.61 (1H, ABX, J 12.6 and 18.4Hz, isomer A), 4.25 (2x2H,q, isomers A and B), 4.96-5.09 (2x1H,m, isomers A and B), 6.56 (2x1H,s, isomers A and B), 6.88 (1H, broad s, isomer A), 6.90 (1H, broad s, isomer B), 7.10 (1H,s, isomer A), 7.11 (1H,s, isomer B).

### EXAMPLE 59

### p-Methoxybenzyl (5R,6S) 6-bromopenem-3-carboxylate (Steps A7 to A9; Compound IC)

A solution of the phosphorane (7)(69.8g) in acetonitrile (1600ml) was stirred and treated in quick succession with 4-dimethylaminopyridine (16.7g) and a solution of silver nitrate (23.2g) in acetonitrile (75ml). After stirring at ambient temperature for 0.5h, the solution was cooled to 5°C and treated in quick succession with 4-dimethylaminopyridine (12.9g), formic-acetic anhydride (84ml) and sodium iodide (158g). The cooling bath was removed and stirring continued for 0.5h. The mixture was poured into stirred ethyl acetate (1600ml) and water (1600ml) and filtered through Celite. The filtrate was separated and the organic phase washed with 5% citric acid (500ml), saturated sodium bicarbonate (2x500ml) and brine (500ml). After drying (MgSO₄), the solution was concentrated and the residue chromatographed over silica gel (700g). Elution with 25% EtoAc/petroleum ether (60-80) gave the title compound (15; IC, R³ = H, R^{x} = pMB) as a white solid (25.1g), after crystallisation from ethyl acetate/petroleum ether. (Spectroscopic data consistent with material prepared in Example 5).

### EXAMPLES 60-62

### p-Methoxybenzyl (5R) 6-(1-methyl 1,2,3-triazol-4-ylmethylene)penem-3-carboxylate (Steps F1 and F2)

### Example 60

The procedure of Example 50 was repeated with the change that the acetic anhydride used was replaced by isobutyl chloroformate (0.56ml). This gave a crude product containing three parts of (Z)-isomer (29) to one part of (E)-isomer (28).

### Example 61

The procedure of Example 50 was repeated with the change that the acetic anhydride was replaced by pivaloyl chloride (0.53ml). This gave a mixture containing four parts of the (Z)-isomer (29) to one part of the (E)-isomer (28).

### Example 62

The procedure of Example 32 was repeated with the change that the reductive elimination of the intermediate crude mesylate was performed according to the method of Example 50. This gave a crude product containing five parts of the (Z)-isomer (29) to two parts of the (E)-isomer (28).

### EXAMPLE 63

### p-Methoxybenzyl (5R) 6-(1-methyl-1,2,3-triazol-4-ylmethylene)penem-3-carboxylate (Step F2)

A mixture of bromohydrins (26) (approximately a 4:1:1:1 mixture of isomers A,B,C, and D)(0.25g) in dichloromethane (10ml) was treated with pyridine (0.20ml) and benzoyl chloride (0.24ml) for 16 hours at 20°C. The solution was washed with brine, 5% aqueous citric acid, brine, saturated aqueous sodium bicarbonate, and brine, dried (MgSO₄), evaporated, and the residue purified by chromatography on silica gel, eluting with hexane/ethyl acetate mixtures, to give the corresponding benzoates (0.23g) as a colourless oil; νₘₐₓ (film) 1805, 1720 cm⁻¹.

This material (0.08g) was dissolved in DMF (2ml) and zinc (0.04g) and ammonium chloride (0.03g) were added. After one hour stirring at 20°C ethyl acetate was added, and the solution washed with 5% aqueous citric acid, brine, saturated aqueous sodium bicarbonate, and brine, dried (MgSO₄), and evaporated, to give a crude product containing five parts of the (Z)-isomer (29) to one part of the (E)-isomer (28).

### EXAMPLE 64

### (5R) p-Methoxybenzyl 6,6-dibromopenem-3-carboxylate (Compound IC)

To a solution of diphenylamine (0.5g) in THF (10ml) at 20°C was added n-butyllithium (1.0ml of 2.5M solution in hexane). The solution was cooled to -70°C and a solution of (5R,6S) p-methoxybenzyl 6-bromopenem-3-carboxylate (1.0g) in THF (3ml) was added, followed by a solution of bromine (0.13ml) in hexane (2ml). After 20 minutes stirring without cooling, ethyl acetate and saturated aqueous sodium bicarbonate were added. The organic part was dried (MgSO₄), and evaporated, and the residue crystallised twice from ether, then purified by chromatography on silica gel eluting with dichloromethane to give the title compound (41; IC, X = Y = Br, R³ = H, R^{x} = pMB) (0.6g) as white needles; m.p. 148°-150°C, [α]_{D}²⁰ + 45.3° (C 1.0, CHCl₃); λₘₐₓ (EtOH) 280nm (εₘ 8,000); νₘₐₓ (KBr) 1791, 1697cm⁻¹; δₚₚₘ (CDCl₃) 3.81 (3H,s), 5.20 (2H, ABq), 6.24 (1H,s) 6.89 (2H,d,J8Hz), 7.30 (2H,d,J8Hz), 7.34(1H,s). (Found C37.1, H2.5, N3.1%, C₁₄H₁₁Br₂NO₄S requires C37.4, H2.5, N3.1%).

### Example 65

### (5R) 6,6-Dibromopenem-3-carboxylic acid (Compound IC)

(5R) p-Methoxybenzyl 6,6-dibromopenem-3-carboxylate (41) (0.23g) in dichloromethane (4ml) was added over 5 minutes to a solution of aluminium chloride (0.13g) in dichloromethane (1ml)/anisole (2ml), at -40°C. After 20 minutes at -20°C, aqueous disodium hydrogen phosphate (20ml of 0.5M) was added and the mixture filtered through Celite. The organic phase was separated washed with ether then taken to pH2 and extracted with methyl isobutyl ketone. This organic extract was washed with water, dried (MgSO₄), and evaporated, and the residue collected with petrol to give the title acid (42; IC, X = Y¹ = Br, R³ = R^{x} = H) (0.06g) as a white powder; m.p. 135-150°C (decomp.); νₘₐₓ (KBr) 1795, 1663cm⁻¹; δₚₚₘ (acetone -d₆) 6.54 (1H,s), 7.71 (1H,s).

### EXAMPLE 66

### (5R) p-Methoxybenzyl 6-[acetoxy(1-methyl-1,2,3-triazol-4-yl)methyl]-6-bromopenem-3-carboxylate (Step F1, Compound ID)

A solution of (5R) p-methoxybenzyl 6,6-dibromopenem-3-carboxylate (41) (0.14g) in THF (5ml) at -70°C was treated consecutively with methylmagnesium bromide (0.15ml of 3M solution in ether), 1-methyl-1,2,3-triazole-4- carboxylaldhyde (0.05g) in THF (2ml), and acetic anhydride (0.10ml) in THF (1ml). After 20 minutes without cooling, ethyl acetate was added, and the solution washed with saturated aqueous sodium bicarbonate and brine, dried (MgSO₄), and evaporated. The residue was purified by chromatography on silica gel, eluting with mixtures of ethyl acetate/hexane, to give a mixture of the four isomers of the title compound (27) (0.03g); δₚₚₘ 2.04, 2.18, 2.21 (3H, each s), 3.83 (3H,s), 4.12 (3H,s), 5.26 (2H,m), 6.19, 6.40, 6.50, 6.64 (1H, each s), 6.9-7.9 (7H, m).

### Example 67

### (5R,6S) p-Methoxybenzyl 6-chloropenem-3-carboxylate (Compound IC via Route D)

### EXAMPLE 67a

### p-Methoxybenzyl 6-chloropenicillanate (Preparation of Starting Material IX)

A solution of phosphorous tribromide (0.75ml) in dry ether (2ml) was added to a solution of p-methoxybenzyl alcohol (3.3g) and pyridine (2 drops) in dry ether (30ml) at 0°C. After 20 minutes stirring without cooling, the solution was washed with brine, saturated aqueous sodium bicarbonate, and more brine, dried (MgSO₄), and evaporated.

The resulting residue in DMF (2ml) was added to a solution of benzylammonium 6-chloropenicillanate [see N.P. Gensmantel et al, J.C.S.Perkin II, 1725 (1980)] (6.7g) in DMF (30ml). After 2 hours at 20°C, the solution was diluted with ethyl acetate and washed with brine, saturated aqueous sodium bicarbonate, and brine twice, dried (MgSO₄) and evaporated. The residue was chromatographed on silica gel eluting with ethyl acetate/hexane mixtutes to give the title ester (43, IX, X = Cl, Y¹ = H, R^{x} = pMB) as a semi-solid (2.0g); δₚₚₘ (CDCl₃) 1.4 (3H, s), 1.6 (3H, s), 3.8 (3H, s), 4.4 (1H, s), 4.7 (1H, s), 5.1 (2H s), 5.3 (1H, s), 6.9 (2H,d,J8Hz), 7.3 (2H, d, J8Hz).

### EXAMPLE 67b

### p-Methoxybenzyl 6-chloropenicillanate-1-oxide (Step A1)

m-Chloroperbenzoic acid (1.1g) was added to a solution of p-methoxybenzyl 6-chloropenicillanate (43) (2.0g) in ethyl acetate (10ml) at 0°C. After 15 minutes the solution was diluted with ethyl acetate and washed with saturated aqueous sodium bicarbonate, 10% aqueous sodium bisulphite, saturated aqueous sodium bicarbonate, and brine twice, dried (MgSO₄), and evaporated. The residue was crystallised from diethyl ether to give the title sulphoxide (44; XI, symbols as for 43) (1.3g); m.p. 102°-104°C; δₚₚₘ (CDCl₃) 1.1 (3H, s), 1.7 (3H, s), 3.9 (3H, s), 4.6 (3H, s), 5.2 (2H, m), 5.3 (2H, ABq), 7.1 (2H, d, J8Hz), 7.5 (2H, d, J8Hz).

### EXAMPLE 67c

### (3S,4R) 3-chloro-1-(1-p-methoxybenzyloxycarbonyl-2-methylprop-1-enyl)-4(E)-[2-(2,2,2-trichloroethoxy carbonyl)ethenylthio]azetidin-2-one (Steps D1 and D2; Intermediate XXII)

Reaction of p-methoxybenzyl 6-chloropenicillanate-1-oxide (44) (1.9g) with 2,2,2-trichloroethyl propiolate (1.0g), followed by triethylamine, and finally phosphorous tribromide/DMF using the method described in Example 11, gave (3S,4R) 3-chloro-1-(1-p-methoxybenzyloxycarbonyl-2-methylprop-1-enyl)-4(E)-[2-(2,2,2-trichloroethoxycarbonyl)ethenylthio]azetidin-2-one (45; XXII, X = Cl, Y¹ = R³ = H, R^{x} = pMB, R¹⁴ = trans CO₂CH₂CCl₃) as a yellow gum (1.4g), δₚₚₘ (CDCl₃)2.0 (3H, s), 2.3 (3H, s), 3.8 (3H, s), 4.8 (3H, m), 5.2 (3H, m), 6.0 (1H, d, J 15Hz), 6.9 (2H, d, J8Hz), 7.3 (2H, d, J8Hz), 7.6(1H, d, J15Hz).

### EXAMPLE 67d

### (5R,6S) p-Methoxybenzyl 6-chloropenem-3-carboxylate (Steps D3 to D5; Compound IC)

Using the conditions described in Example 14, the azetidinone (45)(1.3g) was converted to the title penem (46; IC, X = Cl, Y¹ = R³ = H, R^{x} = pMB) (0.15g) as needles from ether; m.pt. 74°-76°C; λₘₐₓ (EtOH) 321 nm (εₘ 7,200); νₘₐₓ (KBr) 1789, 1705cm⁻¹; δₚₚₘ (CDCl₃) 3.82 (3H, s), 5.13 (1H, dd, J 1.5 and 1.0Hz), 5.21 (2H, ABq), 5.70 (1H, d, J 1.5Hz), 6.88 (2H, d, JHz), 7.35 (2H, d, J8Hz), 7.21 (1H, d, J 1.0Hz).

### EXAMPLE 68

### (5R,6S) p-Methoxybenzyl 6-chloropenem-3-carboxylate (Compound IC via Route B and C)

### EXAMPLE 68a

### (3S,4R) 4-(benzothiazol-2-yldithio)-3-chloro-1-(1-p-methoxybenzyloxycarbonyl-2-methylprop-1 -enyl)azetidin-2-one (Step B1)

Using the method described in Example 2d, p-methoxybenzyl 6-chloropenicillanate-1-oxide (44) (1.1g), was converted to (3S,4R) 4-(benzothiazol-2-yldithio)-3-chloro-1-(1-p-methoxybenzyloxycarbonyl-2-methylprop-1-enyl)azetidin-2-one (47; XIX, R^{x} = pMB, Het = benzothiazol-2-yl, Y¹ = H, X = Cl) (1.6g) as a yellow oil νₘₐₓ (film) 1795, 1725cm⁻¹; δₚₚₘ (CDCl₃) 1.9 (3H, s), 2.1 (3H, s), 3.8 (3H, s), 5.0 (1H, d, J1.1Hz), 5.2(2H, ABq), 5.3(1H, d, J 1.1Hz), 6.8-8.1 (8H, m).

### EXAMPLE 68b

### (3S,4R) 4-acetylthio-3-chloro-1-(1-p-methoxybenzyloxycarbonyl-2-methylprop-1-enyl)azetidin-2-one (Step B2)

Using the method of Example 2e, the azetidinone (47) (1.5g) was converted to (3S,4R) 4-acetylthio-3-chloro-1-(1-p-methoxybenzyloxycarbonyl-2-methylprop-1-enyl)azetidin-2-one (48; XII, R^{x} = pMB, Q = acetyl, Y¹ = H, X = Cl) (0.4g) as a colourless oil; νₘₐₓ (film) 1790, 1720cm⁻¹; δₚₚₘ (CDCl₃) 2.0 (3H, s), 5.7 (1H, d, J2Hz), 7.0 (2H, d, J8Hz), 7.5 (2H, d, J8Hz).

### EXAMPLE 68c

### (3S,4R) 4-acetylthio-3-chloro-1-(1-p-methoxybenzyloxycarbonyl-1-triphenylphosphoranylidenemethyl)azetidin -2-one (Steps C1 and C2; Compound XVI)

A solution of the thiolacetate (48) (0.4g) in ethyl acetate was ozonised at -65°C for one hour. Excess ozone was purged with argon, ethyl acetate and toluene were added, and the solution was washed with aqueous sodium bisulphite and brine, dried (MgSO₄), and evaporated until toluene began to distil. Triphenylphosphine (1.0g) and triethyl phosphite (0.2ml) were added and the solution stood for 18 hours at 20°C. Chromatography silica gel eluting with ethyl acetate/hexane mixtures then gave (3S,4R) 4-acetylthio-3-chloro-1-(1-p-methoxybenzyloxycarbonyl-1-triphenylphosphoranylidenemethyl)azetidin-2-one (49; XVI, R^{x} = pMB, X = Cl) (0.25g) as a yellow oil, νₘₐₓ (film) 1780, 1700, 1630cm⁻¹,

### EXAMPLE 68d

### (3S,4R) 3-chloro-1-(1-p-methoxybenzyloxycarbonyl-1-triphenyl-phosphoranylidenemethyl)azetidin-2-one -4-thiolate (Step A7; Intermediate XVII)

The method of Example 4 was used to convert the phosphorane (49)(2.4g) to silver (3S,4R) 3-chloro-1-(1-p-methoxybenzyloxycarbonyl-1-triphenylphosphoranylidenemethyl)azetidin-2-one-4-thiolate (50; XVII, R^{x} = pMB, X = Cl) (1.6g) as a brown powder, νₘₐₓ (film) 1770, 1610cm⁻¹.

### EXAMPLE 68c

### (5R,6S) p-Methoxybenzyl 6-chloropenem-3-carboxylate (Compound IC; Steps A8 and A9)

The silver salt (50) (1.6g) was dissolved in acetonitrile (30ml) at 0°C and treated with formic acetic anhydride (2ml), 4-dimethylaminopyridine (0.3g) and sodium iodide (3.5g). After 20 minutes at 0°C, ethyl acetate was added and the mixture filtered. The filtrate was washed with 5% aqueous citric acid, brine, saturated aqueous sodium bicarbonate, and brine twice, and dried (MgSO₄). After one hour at 20°C the solution was evaporated and the residue purified by chromatography on silica gel, eluting with dichloromethane, to give penem (46) (0.2g) identical to that obtained by Example 67d.

### EXAMPLE 69

### (5R) p-Methoxybenzyl 6-chloro-6-[acetoxy(1-methyl-1,2,3-triazol-4-yl)methyl]-penem-3-carboxylate (Step F1; Compound ID)

A solution of (5R,6S) p-methoxybenzyl 6-chloropenem-3-carboxylate (46) (0.16g) in THF (4ml) at -70°C was treated in rapid succession with lithium hexamethyldisilazide (0.60ml of 0.9M solution in hexane), 1-methyl-1,2,3-triazole-4-carboxaldehyde (0.07g) in THF (3ml), and acetic anhydride (0.20ml). After 10 minutes without cooling, ethyl acetate was added and the solution washed with saturated aqueous sodium bicarbonate and brine, dried (MgSO₄), and evaporated. The residue was purified by chromatography on silica gel, eluting with ethyl acetate/hexane mixtures, to give a mixture of all four isomers of the title compound (51; ID, X = Cl, R³ = H, R^{x} = pMB, Z = CH₃COO-, R¹² = 1-methyl-1,2,3-triazol-4-yl) (0.10g) as a off-white foam; νₘₐₓ (film) 1800, 1750, 1710cm⁻¹; δₚₚₘ (CDCl₃) 2.05, 2.15 2.19 (3H, each s), 3.8 (3H, s), 4.1 (3H, s), 5.2 (2H, m), 6.0, 6.2, 6.4, 6.6 (1H, each s), 6.9 (2H, d, J8Hz), 7.5(2H, d, J8HZ), 7.2, 7.3, 7.6, 7.8 (3H, each s).

### EXAMPLE 70

### (3S,4R) 4-Benzoylthio-3-bromo-1-(1-p-methoxybenzyloxycarbonyl-1-triphenylphosphoranylidenemethyl)azetidin-2 -one (Steps A7 and A8; Intermediate XVIII)

A solution of azetidinone (7) (1.1g) (see Example 2h) in acetonitrile (20ml) at 20°C was treated with silver nitrate (0.3g) and 4-dimethylaminopyridine (0.2g). After 40 minutes at 20°C the solution was cooled to 0°C and 4-dimethylaminopyridine (0.2g), benzoyl chloride (0.2ml) and sodium iodide (0.6g) were added. After 10 minutes at 0°C, the mixture was diluted with ethyl acetate, filtered through celite, washed with 5% aqueous citric acid, brine, saturated aqueous sodium bicarbonate twice and brine three times, dried (MgSO₄), and evaporated. The residue was purified by chromatography on silica gel, eluting with ethyl acetate/hexane mixtures to give the title phosphorane as a gum (52; XVIII, X = Br, R³ = phenyl) (0.3g); νₘₐₓ (film) 1780, 1670, 1630cm⁻¹.

### EXAMPLE 71

### p-Methoxybenzyl (5R,6S) 6-bromo-2-phenylpenem-3-carboxylate (Step A9; Compound IC)

A solution of (3S,4R) 4-benzoylthio-3-bromo-1-(1-p-methoxybenzyloxycarbonyl-1-triphenylphosphoranylidenemethyl)azetidin-2-one (52) (0.3g) in toluene (5ml) was heated to reflux for 4h then cooled and chromatographed on silica gel, eluting with dichloromethane, to give the title compound (53; IC, R^{x} = pMB, R³ = phenyl) as needles from ether/petrol (0.1g); m.p. 102°-104°C; νₘₐₓ (film) 1800, 1710cm⁻¹; δₚₚₘ (CDCl₃) 3.8 (3H, s), 5.1 (2H, s), 5.2(1H, d, J2Hz), 5.8 (1H, d, J2Hz), 6.9 (2H, d, J8Hz), 7.4 (2H, d, J8Hz), 7.4 (5H, s).

### EXAMPLE 72

### p-Methoxybenzyl (5R) (Z)-6-(1-methyl-1,2,3-triazol-4-yl methylene)-2-phenylpenem-3-carboxylate (Steps F1 and F2)

Using the method described in Example 50, p-methoxybenzyl (5R,6S) 6-bromo-2-phenylpenem-3-carboxylate (53) (0.13g) gave the title compound (54, XA, R^{x} = pMB, R³ = phenyl, R¹² = 1-methyl-1,2,3-triazol-4-yl) (0.03g) as yellow needles, m.p. 159°-161° )Et₂O); νₘₐₓ (KBr) 1763, 1702cm⁻¹; δₘₐₓ (CDCl₃) 3.81 (3H, s), 4.14 (3H, s), 5.19 (2H, ABq), 6.56 (1H, s), 6.80 (2H, d, J8Hz), 7.15 (1H, s), 7.3-7.5 (5H, m), 7.67 (1H, s).

### EXAMPLE 73

### Sodium (5R) (Z)-6-(1-methyl-1,2,3-triazol-4-yl-methylene)-2-phenylpenem-3-carboxylate (Deprotection of XA)

A solution of p-methoxybenzyl (5R) (Z)-6-(1-methyl-1,2,3-triazol-4-ylmethylene)-2-phenylpenem-3-carboxylate (54) (20mg) in dichloromethane (0.2ml) was added to a solution of aluminium chloride (13mg) in anisole (0.2ml/dichloromethane (0.1ml) at -40°C. After 10 minutes at -40°C, aqueous disodium hydrogen phosphate (2ml of 0.5M) was added. The mixture was filtered through Celite, washing with water. The aqueous part of the filtrate was washed twice with ether then adjusted to pH2. The precipiated penem free acid was collected and then stirred with water while the pH was taken to 7. Filtration and freeze-drying gave the title penem (55; XA, R^{x} = Na, R³ = phenyl, R¹² as for 54) (8mg) as a yellow powder; λₘₐₓ (H₂O) 280nm (εₘ 20,600); νₘₐₓ (KBr) 1764, 1592cm⁻¹; δₚₚₘ (D₂O) 4.14 (3H, s), 6.62 (1H, d, J1Hz), 7.24 (1H, d, J1Hz), 7.40 (5H, s), 8.12 (1H, s).

### EXAMPLE 74

### p-Methoxybenzyl (5R,6S) 6-bromo-2-(1,5-dimethylpyrazol -3-yl)penem-3-carboxylate (Steps A7 to A9: Compound IC)

Using the methods of Examples 70 and 71, azetidinone (7) (7.5g) was converted to the title penem (56, IC, R^{x} = pMB, R³ = 1,5-dimethyl-pyrazol-3-yl) (0.07g) as white rhombs; m.p. 135-136°C(ether); νₘₐₓ(KBr) 1800, 1714cm⁻¹; δₚₚₘ (CDCl₃) 2.26 (3H, s), 3.78 (3H, s), 3.82 (3H, s), 5.10 (1H, d, J1Hz), 5.24 (2H, s), 5.56 (1H, d, J1Hz), 6.70 (2H, d, J8Hz, 7.40 (2H, d J8Hz), 6.95 (1H, s).

### EXAMPLE 75

### p-Methoxybenzyl (5R) 2-(1,5-dimethylpyrazol-3-yl)-(Z)-6-(1-methyl-1,2,3-triazol-4-ylmethylene)penem-3 -carboxylate (Steps F1 and F2)

The procedure of Example 50 was used to convert p-methoxybenzyl (5R,6S) 6-bromo-2-(1,5-dimethylpyrazol-3-yl)penem-3-carboxylate (56) (55mg) to the title penem (57; XA, R^{x} = pMB, R³ as for 56, R¹² as for 54) (22mg), obtained as yellow rhombs from ether, m.pt. 202-205°C; δₚₚₘ (CDCl₃) 2.3 (3H, s), 3.7 (3H, s), 3.8 (3H, s), 5.3 (2H, s), 6.3 (1H, s), 6.9 (3H, s), 7.0 (1H, s). 7.4 (2H, s), 7.6 (1H, s).

### EXAMPLE 76

### Sodium (5R) 2-(1,5-dimethylpyrazol-3-yl)-(Z)-6-(1-methyl-1,2,3-triazol-4-ylmethylene)penem-3-carboxylate

Using the procedure of Example 73, p-methoxybenzyl (5R) 2-(1,5-dimethylpyrazol-3-yl)-(Z)-6-(1-methyl-1,2,3-triazol-4-ylmethylene)penem-3-carboxylate (57) (20mg) was converted to the title compound (58; XA, R^{x} = Na, R³ and R¹² as for 57) (12mg), obtained as a yellow powder, λₘₐₓ 280 nm (εₘ 18,900); νₘₐₓ (KBr) 1763, 1611cm⁻¹; δₚₚₘ (D₂O) 2.28 (3H, s), 3.60 (3H, s), 4.12 (3H, s), 6.47 (1H, s), 6.61 (1H, s), 7.21 (1H, s), 8.13 (1H, s).

### EXAMPLE 77

### p-Methoxybenzyl (5R,6S) 6-bromo-2-(2-furyl)penem-3-carboxylate (Steps A7 to A9; Compound IC)

Using the methods described in Examples 70 and 71, azetidinone (7) (15g) was converted to the title penem (59; IC, R^{x} = pMB, R³ = 2-furyl) (1.3g), obtained as white rhombs from ether; m.p. 90-93°C; δₘₐₓ (KBr) 1813, 1713cm⁻¹; νₚₚₘ (CDCl₃) 3.88 (3H, s), 5.14 (1H, d, J1Hz), 5.26 (2H, s), 5.62 (1H, d, J1Hz), 6.53 (1H, dd, J1Hz and 4Hz), 6.90 (2H, d, J8Hz), 7.38 (2H, d, J8Hz), 7.52 (1H, d, J1Hz), 7.73 (1H, d,J4Hz).

### EXAMPLE 78

### p-Methoxybenzyl (5R) 2-(2-furyl)-(Z)-6-(1-methyl-1,2,3-triazol-4-yl-methylene)penem-3-carboxylate (Steps F1 and F2)

The procedure of Example 50 was used to convert p-methoxybenzyl (5R,6S) 6-bromo-2-(2-furyl)penem-3-carboxylate (59) (0.44g) to the title compound (60; XA, R^{x} = pMB, R³ = 2-furyl, R¹² as for 54) (0.20g), obtained as yellow rhombs from ether; m.p. 186-190°C; λₘₐₓ (EtOH) 286nm (εₘ 27,100); νₘₐₓ (KBr) 1777, 1703 cm⁻¹; δₚₚₘ (CDCl₃) 3.81 (3H, s), 4.17 (3H,s), 5.29 (2H, s), 6.25 (1H, s), 6.54 (1H, dd, J1Hz and 4Hz), 6.88 (2H, d, J8Hz), 6.89 (1H, s), 7.41 (2H, d, J8Hz), 7.52 (1H, d, J1Hz), 7.69(1H, d, J4Hz), 8.76 (1H, s).

### EXAMPLE 79

### Sodium (5R) 2-(2-furyl)-(Z)-6-(1-methyl-1,2,3-triazol-4-ylmethylene)penem-3-carboxylate

Using the procedure of Example 73, p-methoxybenzyl (5R) 2-(2-furyl)-(Z)-6-(1-methyl-1,2,3-triazol-4-ylmethylene)penem-3-carboxylate (60) (0.10g) was converted to the title compound (61; XA, R^{x} = Na, R³ and R¹² as for 60) (0.06g) as a yellow powder; [α]_{D}²⁰ + 292°(CO.5, H₂O); λₘₐₓ(H₂O) 284 nm (εₘ 20,700); νₘₐₓ (KBr) 1749, 1609cm⁻¹; δₚₚₘ (D₂O) 4.08 (3H, s), 6.40 (1H, s), 6.54 (1H, m), 7.05 (1H, d, J4Hz), 7.14(1H, s), 7.52 (1H, s), 8.10 (1H, s).

### EXAMPLE 80

### Sodium (5R,6S) 6-bromo-2-(2-furyl)penem-3-carboxylate (Deprotection of IC)

A solution of p-methoxybenzyl (5R,6S) 6-bromo-2-(2-furyl)penem-3-carboxylate (59) (0.22g) in dichloromethane (4ml) was added to a solution of aluminium chloride (0.13g) in dichloromethane (1ml)/anisole (2ml) at -40°C. After 5 minutes 0.5M aqueous disodium hydrogen phosphate (20ml) was added and the mixture filtered through celite washing with water. The aqueous fraction of the filtrate was washed twice with ether, then taken to pH3 and extracted with ethyl acetate, which was then stirred with water as the pH was taken to 7. The resulting aqueous solution was chromatographed on Biogel, eluting with water, to give the title compound (62; IC, R^{x} = Na, R³ = 2-furyl) (0.05g), as a pale brown powder after freeze-drying; λₘₐₓ (H₂O) 340nm (εₘ 8,700); νₘₐₓ (KBr) 1782, 1595cm⁻¹; δₚₚₘ(D₂O) 5.42 (1H, d, J1Hz), 5.79 (1H, d, J1Hz), 6.57 (1H, dd, J1Hz and 4Hz), 7.15 (1H, d, J4Hz), 7.55 (1H, d, J1Hz).

### EXAMPLE 81

### p-Methoxybenzyl (5R,6S) 6-bromo-2-(3-pyridyl)penem-3-carboxylate (Steps A7 to A9; Compound IC)

Using the methods described in Examples 70 and 71, but avoiding any acidic washes, azetidinone (7)(7.5g) was converted to the title penem (63; IC, R^{x} = pMB, R³ = 3-pyridyl) (0.3g), obtained as white needles from ether; m.p. 144-146°C; νₘₐₓ (KBr) 1811, 1713cm⁻¹; δₚₚₘ (CDCl₃) 3.81 (3H, s), 5.08 (2H, s), 5.22 (1H, d, J1Hz), 5.79 (1H, d, J1Hz), 6.82 (2H, d, J8Hz), 7.18 (2H, d, J8Hz), 7.25 (1H, m), 7.72 (1H, m), 8.6 (2H, m).

### EXAMPLE 82

### p-Methoxybenzyl (5R) (Z)-6-(1-methyl-1,2,3-triazol-4-ylmethylene)-2-(3-pyridyl)penem-3-carboxylate (Steps F1 and F2)

The procedure of Example 50 was used, but avoiding acidic washes, to convert p-methoxybenzyl 6-bromo-2-(3-pyridyl)penem-3-carboxylate (63) (0.22g) to the title compound (64; XA, R^{x} = pMB, R³ = 3-pyridyl, R¹² as for 54) (0.08g), obtained as yellow rhombs from ether; m.p. 190°C-194°C; νₘₐₓ (KBr) 1772, 1690cm⁻¹; δₚₚₘ (CDCl₃) 3.78 (3H, s), 4.15 (3H, s), 5.21 (2H, ABq), 6.55 (1H, s), 6.81 (2H, d, J8Hz), 7.06 (1H, s), 7.20 (2H, d, J8Hz), 7.2 (1H, m), 7.4 (2H, m), 8.5 (1H, m), 8.6 (1H, m).

### EXAMPLE 83

### Sodium (5R) (Z)-6-(1-methyl-1,2,3-triazol-4-ylmethylene)-2-(3-pyridyl)penem-3-carboxylate

The procedure of Example 58 was used to convert p-methoxybenzyl (5R) (Z)-6-(1-methyl-1,2,3-triazol-4-ylmethylene)-2-(3-pyridyl)penem-3-carboxylate (64) (60mg) to the title compound (65; XA, R^{x} = Na, R³ and R¹² as for 64) (30mg), isolated as a yellow powder; λₘₐₓ (H₂O) 280 nm (εₘ 18,600); νₘₐₓ (KBr) 1758, 1609cm⁻¹; δₚₚₘ (D₂O) 4.05 (3H, s), 6.57 (1H, s), 7.16 (1H, s), 7.4 (1H, bs), 7.70 (1H, d, J8Hz), 8.06 (1H, s), 8.8 (2H, bs).

### EXAMPLE 84

### p-Methoxybenzyl (5R,6S) 6-bromo-2-(2-methyltetrazol-5-yl)-penem-3-carboxylate (Steps A7 to A9; Compound IC)

Using the methods described in Examples 70 and 71, azetidinone (7)(4.5g) was converted to the title penem (66; IC, R^{x} = pMB, R³ = 2-methyl-tetrazol-5-yl) (0.5g), isolated as white rhombs from ethyl acetate/petrol; m.p. 128-130°C; νₘₐₓ (film) 1810, 1725cm⁻¹: δₚₚₘ (CDCl₃) 3.81 (3H, s), 4.28 (3H, s), 5.15 (2H, s), 5.26 (1H, d, J1Hz), 5.82 (1H, d, J1Hz) 6.86 (2H, d, J8Hz), 7.25 (2H, d, J8Hz).

### EXAMPLE 85

### (3S,4R) 3-Bromo-4-(t-butyldiphenylsilyloxyacetylthio)-1-(1-p-methoxybenzyloxycarbonyl-1 -triphenylphosphoranylidenemethyl)azetidin-2-one (Step A8; Intermediate XVII)

The silver thiolate (14)(2.56g) from Example 3 was dissolved in dry dichloromethane (100ml) and cooled to 5°C , with stirring. Pyridine (0.67ml) was added, followed by a solution of t-butyldiphenylsilyloxyacetyl chloride (2.97g)(EP 0 120 613 A, Beecham) in dry dichloromethane (20ml). The reaction mixture was stirred at 5°C for 30min. The resulting suspension was then filtered through Kieselguhr. The filtrate was washed with 1N HCl, water, saturated sodium hydrogen carbonate solution, and brine, dried (MgSO₄), filtered and the solvent evaporated at reduced pressure. The residual oil was chromatographed over silica gel (100g). Elution with a gradient of 10-50% ethyl acetate/hexane afforded the title phosphorane (67; XVIII, R^{x} = pMB, R³ = t-butyldiphenylsilyloxymethyl) as a white foam (1.59g), νₘₐₓ (CH₂Cl₂) 1779, 1788, 1705, 1622cm⁻¹.

### EXAMPLE 86

### p-Methoxybenzyl (5R,6S) 6-Bromo-2-(t-butyldiphenylsilyloxymethyl)penem-3-carboxylate (Step A9; Compound IC)

The phosphorane (67)(1.59g) was dissolved in dry toluene (250ml) and heated to reflux under an atmosphere of argon for 2 hours. After cooling, the solvent was evaporated at reduced pressure and the residue chromatographed over silica gel (100g). Elution with 10% ethyl acetate/hexane afforded the title compound (68; IC, R^{x} = pMB, R³ as for 67) as a yellow oil (0.38g). The product was furthe purified by silica gel column chromatography (10g), with dichloromethane as eluant. The pure product was (0.275g), νₘₐₓ (CH₂Cl₂) 1805, 1712, 1618, 1592cm⁻¹, δ_{H} (CDCl₃) 1.07 (9H, s), 3.79 (3H, s), 4.86 (2H, s), 5.07 (2H, ABq, J12Hz), 5.10 (1H, d, J1.4Hz), 5.59 (1H, d, J1.4Hz), 6.80(2H, d, J8.7Hz), 7.21(2H, d, J8.8Hz), 7.3-7.7(10H, m).

### EXAMPLE 87

### p-Methoxybenzyl (5R) 2-(t-butyldiphenylsilyloxymethyl)-6-(1-methyl-1,2,3-triazol-4-ylmethylene)penem-3 -carboxylate (Steps F1 and F2)

The bromopenem (68)(0.25g) was reacted with lithium diphenylamide, 1-methyl-1,2,3-triazole-4-carboxaldehyde, acetic anhydride and zinc/acetic acid, following the procedure described in Example 55. Silica gel column chromatography of the reaction mixture, on elution with 50% ethyl acetate/hexane, afforded the (E)-isomer of the title compound (69; XB, R^{x} = pMB, R³ as for 67, R¹² as for 54) as a yellow oil (0.053g), νₘₐₓ (CH₂Cl₂) 1772, 1705cm⁻¹, δ_{H} (CDCl₃) 1.07 (9H, s), 3.80 (3H, s), 4.17 (3H, s), 4.86 (2H, AA'q, J17Hz), 5.09 (2H, s), 6.22 (1H, d, J0.5Hz), 6.80 (2H, d, J 8.8Hz), 6.88 (1H, d, J 0.5Hz), 7.22(2H, d, J8.8Hz), 7.3-7.3 (10H, m), 8.76 (1H, s). Continued elution gave the (Z)-isomer of the title compound (70; XA, symbols as for 69) as a yellow oil (0.99g), [α]_{D}²⁰ + 243° (C1.0, CHCl₃), νₘₐₓ (CH₂Cl₂) 1780, 1702, 1618, 1580cm⁻¹, δ_{H} (CDCl₃)1.03 (9H, s), 3.79 (3H, s), 4.17 (3H, s), 4.81 and 4.91 (2H, ABq, J17Hz), 5.03 and 5.12(2H, ABq, J12Hz), 6.48 (1H, d, J1Hz), 6.79 (2H, d, J8.6Hz) 7.02 (1H, d, J1Hz), 7.21 (1H, d, J8.6Hz), 7.3-7.7 (10H, m), 7.69 (1H, s).

### EXAMPLE 88

### p-Methoxybenzyl (5R) 2-(hydroxymethyl)-(z)-6-(1-methyl-1,2,3-triazol-4-ylmethylene)penem-3-carboxylate (Conversion of R³)

The penem (70) (0.093g) was dissolved in dry THF (4ml) and tetra-n-butyl ammonium fluoride (0.179ml of a 1M solution in THF) were added to the stirred solution and stirring was continued at 5°C for 2 hours. The reaction solution was then partitioned between ethyl acetate and water. The organic solution was separated and washed with saturated sodium hydrogen carbonate solution, water and brine and dried (MgSO₄). The solvent was then evaporated at reduced pressure and the residue chromatographed over silica gel (10gm). Elution with a gradient of 50-100% ethyl acetate/hexane afforded the title compound (71; XA, R³ = -CH₂OH, R^{x} and R¹² as for 69) 0.039g), [α]_{D}²⁰ + 417° (C 1.0, CH₂Cl₂), νₘₐₓ (CH₂Cl₂) 1780, 1698, 1615cm⁻¹, δ_{H} CD₂Cl₃) 3.4-3.7 (1H, broad res.) 3.81 (3H, s), 4.13 (3H, s), 4.53 and 4.65 (2H, broad ABq, J 15.5Hz), 5.22 and 5.28 (2H, ABq, J 12.2Hz), 6.46 (1H, d, J1Hz), 6.90 (2H, d, J8.7Hz), 7.01 (1H, d, J1Hz), 7.41 (2H, d, J8.7Hz), 7.69 (1H, s).

### EXAMPLE 89

### Sodium (5R) 2-(Hydroxymethyl)-(Z)-6-(1-methyl-1,2,3-triazol-4-ylmethylene)penem-3-carboxylate

Deprotection of the ester (71)(0.039g) by the procedure described in Example 58 afforded the title compound (72; XA, R^{x} = Na, R³ and R¹² as for 71) as a pale yellow solid after HP-20 column chromatography and lyophilisation, (0.012g),λₘₐₓ (H₂O) 368 (ε 1,126), 281nm (ε 16,031), νₘₐₓ (KBr) 2800-3700 (broad), 1763, 1601cm⁻¹; δ_{H} (D₂O) 4.10 (3H, s), 4.51 and 4.73 (2H, ABq, J15Hz), 6.44 (1H, s), 7.19 (1H, s), 8.12 (1H, s).

### EXAMPLE 90

### p-Methoxybenzyl (5R) (Z)-6-(2-pyridylmethylene)penem-3-carboxylate (Steps F1 and F2)

Pyridine-2-carboxaldehyde (130µl) was reacted with (5R,6S) p-methoxybenzyl 6-bromopenem-3-carboxylate (15) (500mg), by the procedure described in Example 55, and the crude product subjected to chromatography (silica gel eluted with ethyl acetate:n-hexane, 1:1) to give the title penem (73; XA, R^{x} = pMB, R³ H, R¹² = 2-pyridyl) (140mg) as a single isomer. νₘₐₓ(CHCl₃)1780, 1725(Sh.), 1710cm⁻¹; δₚₚₘ (CDCl₃) 3.82 (3H, s), 5.18 (1H, d, J12Hz), 5.27 (1H, d, J12Hz), 6.65 (1H, s with fine coupling), 6.92 (2H, d, J10Hz), 7.05 (1H, s), 7.08 (1H, m), 7.26 (2H, m), 7.39 (3H, m), 7.74 (1H, t with fine coupling, J7.7Hz), 8.65 (1H, d, J4Hz).

### EXAMPLE 91

### Sodium (5R) (Z)-6-(2-pyridylmethylene)penem-3-carboxylate

The penem ester (73)(140mg) was reacted with ethylaluminium dichloride (1.8M solution in toluene, 514µl) diluted in a mixture of dry anisole (1.5ml) and dry methylene dichloride (0.5ml), by the methodology and procedure described in Example 58, to yield the title compound (74; XA, R^{x} = Na, R³ = H, R¹² = 2-pyridyl) as a yellow freeze-dried solid (34mg), λₘₐₓ (H₂O) 260nm (εₘ 13,000), 297nm (εₘ 19,000); νₘₐₓ (KBr) 1750 and 1600cm⁻¹; δₚₚₘ (D₂O) 6.63 (1H, s with fine coupling), 6.97 (1H, d, J 8Hz), 7.14 (1H, s), 7.36 (1H, m), 7.50 (1H, d, J 8Hz), 7.84 (1H, t with fine coupling, J7.8Hz), 8.60 (1H, d, J4Hz).

### EXAMPLE 92

### p-Methoxybenzyl (5R) 6-(2-trifluoromethylbenzylidene) penem-3-carboxylate (Steps F1 and F2)

p-Methoxybenzyl (5R,6S) 6-bromopenem-3-carboxylate (15) (1.0g) was reacted with lithium diphenylamide, 2-trifluoromethylbenzaldehyde, acetic anhydride and zinc/acetic acid, following the procedure described in Example 55. Silica gel column chromatography (20g) of the reaction mixture gave, after elution with 50% ethyl acetate/hexane, the (E)-isomer of the title compound (75; XB, R^{x} = pMB, R¹² = CF₃C₆H₄) as a yellow solid (0.345g), νₘₐₓ (CH₂Cl₂) 1778, 1710, 1610cm⁻¹; δ_{H} (CDCl₃) 3.82 (3H, s), 5.22 (2H, s) 6.43(1H, d, J0.6Hz), 6.91 (2H, d, J8Hz) 7.01 (1H, broad s), 7.36 (2H, d, J8Hz) 7.38 (1H, s), 7.43-7.85 (3H, m), 8.44 (1H, d, J8Hz). Continued elution gave the (Z)-isomer of the title compound (76; XA, symbols as for 75) as a yellow oil (0.104g),νₘₐₓ (CH₂Cl₂) 1792, 1718, 1618cm⁻¹, δ_{H}(CDCl₃) 3.82 (3H, s), 5.18 and 5.27 (2H, ABq, J12Hz), 6.50 (1H, s), 6.90 (2H, d, J8.6Hz), 7.27 (1H, s), 7.3-7.7 (7H, m), 7.78 (1H, d, J8Hz).

### EXAMPLE 93

### Sodium (5R) (Z)-6-(2-trifluoromethylbenzylidine)penem-3-carboxylate

Deprotection of the (Z)-penem ester (76) (0.095g) by the procedure outlined in Example 58, afforded the title compound (77; XA, R^{x} = Na, R¹² = CF₃C₆H₄) as a pale yellow solid (0.027g) after HP-20 column chromatography and lyophilisation, λₘₐₓ(H₂O) 283nm (ε 17,743), νₘₐₓ (KBr) 2600-3600 (Broad), 1762, 1611cm⁻¹, δ_{H} (D₂O) 6.69 (1H, s), 7.02 (1H, s), 7.4-7.75 (4H, m), 7.82 (2H, d, J7.5Hz).

### EXAMPLE 94

### p-Methoxybenzyl (5R) 6-(p-methoxybenzylidene) penem-3-carboxylate (Steps F1 and F2)

p-Methoxybenzyl (5R, 6S) 6-bromopenem-3-carboxylate (15) (1.0g) was reacted with lithium diphenylamide, p-methoxybenzaldehyde and acetic anhydride, following the procedure of Example 50, to yield the mixture of acylated bromohydrins (78)(ID, R^{x}=pMB, R¹²=p-methoxyphenyl, Z=OCOCH₃). The acylated bromohydrins were then reacted with zinc, ammonium chloride and tetramethylethylene diamine dihydrochloride in DMF, according to the procedure of Example 50. Silica gel column chromatography, eluting with a gradient of 25-50% ethyl acetate/hexane afforded the title compound (79) as a mixture of (E) and (Z)-isomers (XA + XB, R^{x} = pMB, R¹² = CH₃OC₆H₄) as a yellow foam (0.120g), νₘₐₓ 1775, 1705, 1600cm⁻¹ δ_{H}(CHCl₃) 3.81 (3H, s), 3.87 (3H, s), 5.1-5.4 (2H, m), 6.39 (s), 6.55 (s), 6.59 (d, J1Hz), 6.85-7.0(m), 7.11(s), 7.2-7.45(m), 7.93(d, J9Hz).

### EXAMPLE 95

### Sodium (5R) 6-(p-methoxybenzylidene)penem-3-carboxylate

The mixture of (E) and (Z)-penems (79) were deprotected, according to the procedure of Example 58, to yield an aqueous solution containing the title compound (80; XA + XB, R^{x} = Na, R¹² = CH₃OC₆H₄) λₘₐₓ (H₂O) 310nm.

### EXAMPLE 96

### Sodium (5R) (Z)-6-(1-methyl-1,2,3-triazol-4-ylmethylene)penem-3-carboxylate (Steps F1 and F2)

The procedure of Example 50 was followed up to but not including the final chromatography therein. The crude product was dissolved in dichloromethane (2ml) and then hexane (100ml) added, and the clear colourless supernatant poured off the precipitated yellow gum. The procedure was repeated to give a crude product which h.p.l.c. showed to contain five parts of the (Z)-penem (29) to one part of the (E)-penem (28).

This mixture was then subjected to the procedure of Example 73 to give (31) (0.5g) which h.p.l.c. showed to contain less than 1% of the corresponding (E)-isomer.

### EXAMPLE 97

### Sodium (5R) (Z)-6-(1-methyl-1,2,3-triazol-4-ylmethylene) penem-3-carboxylate

The (Z) penem ester (29(1.0g) from Example 50 was dissolved in dry dichloromethane (14ml) and added dropwise over a period of 10min to a stirred solution of ethylaluminium dichloride (3.6ml of a 1.8M solution in toluene) in dry anisole (11ml) and dry dichloromethane (2.7ml) at -30°C, under an atmosphere of argon. Stirring was continued for an additional 20 min between -30°C and -20°C. 1M Disodium hydrogen phosphate solution (31.5ml) was then added and the resulting mixture stirred for 10 min without cooling. Celite was added and the resulting suspension filtered through Celite, washing well with water. The filtrate was separated and the aqueous solution washed with ether, filtered through coarse grade glass fibre filter paper and acidified to pH2.0 using 2.5M sulphuric acid. The resulting precipitate was collected by filtration, washing well with water. The solid was then suspended in water and the pH adjusted to 7.0 by the addition of 0.1M sodium hydroxide solution. The resulting solution was freeze-dried to yield the title compound (31) as a yellow solid (0.560g). This sample was identical to that prepared in Example 33.

### EXAMPLE 98

### p-Methoxybenzyl (5R,6R) 6-bromopenem-3-carboxylate (Compound IC)

1,8-Diazabicyclo [5.4.0] undec-7-ene (5 drops) was added to a solution of p-methoxybenzyl (5R,6S) 6-bromopenem-3-carboxylate (15) (5g) in dichloromethane (100ml). After 1h at 20°C the solution was washed with 0.05M aqueous sulphuric acid, dried (MgSO₄), and evaporated to give a solid containing 10% of the title compound (81, IC, R^{x} = pMB, R³ = H) and 90% of the (6S) epimer; δ_{H} (CDCl₃) 3.84 (3H, s), 5.26 (2.9H, m), 5.70 (0.1H, d, J4HHz), 5.81 (0.9H, d, J2Hz), 6.13 (0.1H, d, J4Hz), 6.96 (2H, d, J9Hz), 7.5 (3H, m).

### EXAMPLE 99

### Sodium (5R,6R) 6-bromopenem-3-carboxylate (Compound IC)

1,8-Diazabicyclo [5.4.0]undec-7-ene (0.17ml) in dichloromethane (1ml) was added slowly to a suspension of (5R, 6S) 6-bromopenem-3-carboxylic acid (0.25g) in dichloromethane (5ml) at 20°C. After 10 min the black solution was poured into 0.01M aqueous sulphuric acid and the mixture was extracted three times with methyl isobutyl ketone. The combined extracts were washed with water, dried (MgSO₄), and evaporated. The resulting residue was suspended in water, the pH taken to 7, and the solution then filtered and freeze-dried to give a beige powder containing 10% of the title compound (82; IC, R^{x} = Na, R³ = H) plus 90% of the (6S) epimer; [α]_{D}²⁰ + 109° (c 1.0, H₂O), δ_{H}(D₂O) 5.44 (S, 0.9H), 5.81 (d, 0.1H, J3.5Hz), 5.88 (s, 0.9H), 6.13(d, 0.1H, J 3.5Hz), 7.09 (s, 1H).

### EXAMPLE 100

### 1-Phenoxyethyl (5R, 6S) 6-bromopenem-3-carboxylate (Compound IC via Route D)

### Example 100a

### 1-Phenoxyethyl 6-bromopenicillanate (Preparation of Starting Material IX)

A solution of dicyclohexylammonium 6-bromopenicillanate (9.1g) and 1-chloroethyl phenyl ether (3.1g) in THF (50ml) and acetonitrile (5ml) was stirred at 20°C for 1 hour. Saturated aqueous sodium bicarbonate and ethyl acetate were then added, and the separated organic phase was washed with water, dried (MgSO₄), and evaporated. The resulting residue was purified by chromatography on silica gel, eluting with ethyl acetate/dichloromethane mixtures, to give the title ester (83; IX) as a colourless oil; νₘₐₓ (film) 1790, 1745cm⁻¹.

### Example 100b

### 1-Phenoxyethyl 6-bromopenicillanate-1-oxide (Step A1)

Application of the procedure of Example 2c to 1-phenoxy-ethyl 6-bromopenicillanate (83) (1.4g), gave the title sulphoxide (84) a colourless oil (1.0g); δ_{H} (CDCl₃) 1.0 (3H, d, J5Hz), 1.5-1.8 (6H, m), 4.5 (1H, bs), 5.0 (1H, bs), 5.1 (1H, bs), 6.6-7.6 (6H, m).

### Example 100c

### (3S,4R) 3-Bromo-4-(E)-(2-methoxycarbonylethenylthio)-1-[1-(1-phenoxyethoxycarbonyl)-2-methylprop-1-enyl] -azetidin-2-one (Steps D1 and D2; Intermediate XXII)

Reaction of 1-phenoxyethyl 6-bromopenicillanate-1-oxide (84) (1.2g) with methyl propiolate (1.0ml) according to the method of Example 10 gave the title compound (85)(0.5g) as a colourless gum; δ_{H} (CDCl₃) 1.65, 1.7 (3H, 2d, each J5Hz), 2.1-2.3 (6H, 4s), 3.7 (3H, s), 4.7 (1H, 2d, each J2Hz), 5.0 (0.5H, d, J2Hz), 5.4 (0.5H, d, J2Hz), 5.8, 6.0, 7.3, 7.6 (2H, 4d, each J15Hz), 6.65, 6.75 (1H, 2d, each J5Hz), 6.9-7.4 (5H, m).

### EXAMPLE 100d

### 1-Phenyoxyethyl (5R,6S) 6-bromopenem-3-carboxylate (Steps D3 to D5; Compound IC)

Using the method of Example 14, ester (85) (0.5g) was converted to the title compound (86)(0.1g), obtained as a colourless oil, δ_{H} (CDCl₃) 1.66 (3H, d, J5Hz). 5.18 (1H, m), 5.79 (1H, d, J1Hz), 6.6 - 7.5 (7H, m).

### EXAMPLE 101

### 2,2,2-Trichloroethyl (5R,6S) 6-bromopenem-3-carboxylate (Compound IC)

### EXAMPLE 101a

### 2,2,2-Trichloroethyl (5R,6S) 6-bromopenicillanate (Preparation of Starting Material IX)

2,2,2-Trichloro 6-diazopenicillanate (18g) (prepared by the method of J.C. Sheehan et al, J.Org.Chem., 39, 1444, 1974) was dissolved in acetone (500ml) and cooled to 5°C with stirring. A solution of sodium bromide (20.6g) in water (60ml), containing 48% hydrogen bromide solution (12.66ml), was added rapidly to the above solution. After stirring at 5°C for 30min, sodium hydrogen carbonate (10.08g) was added and the solution stirred at room temperature for 10min. The solution was decanted and evaporated to small volume. The residue was partitioned between ethyl acetate and water. The organic layer was washed with brine, dried (MgSO₄) and evaporated. The residue was chromatographed over silica gel, eluting with a gradient of 10-25% ethyl acetate/hexane. The title ester (87) was obtained as a white solid after crystallisation from ethyl acetate/hexane (14.6g), νₘₐₓ (CH₂Cl₂) 1792, 1760cm⁻¹, δ_{H} (CDCl₃) 1.56 (3H, s), 1.68 (3H, s), 4.72 (1H, s), 4.82 (2H, s), 4.87 (1H, d, J2Hz), 5.49 (1H, d, J 2Hz).

### EXAMPLE 101b

### (3S, 4R) 3-Bromo-4-formylthio-1-[1-[2,2,2-trichloroethyloxycarbonyl)-2-methylprop-1-enyl]azetidin-2-one

A solution of the ester (87)(1.0g) in acetonitrile (5ml) was added to a cooled solution (5°C) of silver nitrate (0.413g) and 1,5-diazabicyclo [4.3.0]non-5-ene (0.317g) in acetonitrile (25ml), under an atmosphere of argon. The solution was stored at 5°C for 88 hours. Acetic formic anhydride (1.94ml), 4-dimethylaminopyridine (0.297g) and sodium iodide (3.645g) were then added to the solution, which was then filtered through Kieselguhr, washing well with ethyl acetate. The organic solution was washed with 5% citric acid solution, water, sodium hydrogen carbonate solution, water and brine and dried (MgSO₄). The solvent was evaporated at reduced pressure to yield the title formyltnio-derivative (88) as a pale yellow semi solid (0.95g), νₘₐₓ (CH₂Cl₂) 1792, 1740, 1685, 1630cm.

### EXAMPLE 101c

### 2,2,2-Trichloroethyl (5R,6S)6-bromopenem-3-carboxylate (Steps E2, D4 and D5; Compound IC)

The ester (88)(0.95g) from was converted to the title compound (89), by the procedure described in Examples 25b and 25c. The product was obtained as a white solid (0.20g) after silica gel column chromatography, νₘₐₓ (CH₂Cl₂) 1812, 1738cm⁻¹, δ_{H} (CDCl₃) 4.82 (2H, ABq, J12Hz), 5.17 (1H, d, J1.5Hz), 5.78(1H, d, J1.5Hz), 7.42 (1H, s).

### EXAMPLE 102

### (5R) p-Methoxybenzyl 6-methylenepenem-3-carboxylate (Steps F1 and F2)

(5R,6S) p-Methoxybenzyl 6-bromopenem-3-carboxylate (15) (0.5g) was lithiated according to the procedure in Example 50. The reaction was then quenched sequentially with excess gaseous formaldehyde and acetic anhydride (0.8ml) respectively. (5R) p-Methoxybenzyl 6-acetoxymethyl-6-bromopenem-3-carboxylate (90; ID) was isolated after work-up according to Example 50 and chromatographed on silica eluting with n-hexane/ethyl acetate (2:1). The bromoacetate thus obtained (0.16g) was reacted with zinc/acetic acid according to the procedure in Example 30. The title penem (91; X, R¹² = H, R³ = H, R^{x} = pMB) was isolated by chromatography on silica, eluting with n-hexane/ethyl acetate 2:1, and crystallized from chloroform/diethyl ether (0.037g), m.p. 93-94°C; [α]_{D} -115°(cl. 0, CHCl₃); νₘₐₓ (KBr) 1764 and 1718cm⁻¹; δ(CDCl₃) 3.82 (3H, s,), 5.16 and 5.24 (2H, ABq, J12Hz), 5.50 (1H, d, J2Hz), 6.02 (1H, dd, J1.5 and 2Hz), 6.30 (1H, br s), 728 (1H, s) and 6.90 with 7.36 (4H, AA'BB').

### EXAMPLE 103

### (5R) Sodium 6-Methylenepenem-3-carboxylate

(5R) p-Methoxybenzyl 6-methylenepenem-3-carboxylate (91)(0.2g) was deesterified and purified according to the procedure in Example 58. The title salt (92) was thus isolated in 55% yield. νₘₐₓ (KBr) 1762 and 1617cm⁻¹; δ(D₂O) 5.63 (1H, d, J2.1Hz), 5.98 (1H, dd, J1.1 and 2.1Hz), 6.38 (1H, br, s) and 7.08 (1H, s).

### EXAMPLE 104

### p-Methoxybenzyl (5R, 6S) 6-bromo-2-imidazol-1-ylpenem-3-carboxylate (Steps F1 and F2)

Using the procedure of Examples 70 and 71 phosphorane (7)(6.7g) and 1,1'-thiocarbonyldiimidazole (1.8g) were converted to the title compound (93)(0.3g) which was obtained as colourless plates from ether; m.p. 113°-116°C; [α]_{D}²⁰ + 127° (cl.0,CHCl₃); νₘₐₓ (film) 1795, 1705cm⁻¹; δ_{H} (CDCl₃) 3.81 (3H, s), 5.18 (2H, s), 5.24 (1H, d, J 1Hz), 5.70 (1H, d, J 1Hz), 6.88 (2H, d, J 7Hz), 7.12 (1H, bs), 7.19 (1H, t, J 1Hz), 7.28 (2H, d, J=7Hz), 7.82 (1H, bs).

### EXAMPLE 105

### Sodium (5R) 2-imidazol-1-yl-6-(Z)-(1-methyl-1,2,3-triazol-4-ylmethylene)penem-3-carboxylate

Using the procedure of Examples 82 and 58, p-methoxybenzyl (5R,6S) 6-bromo-2-imidazol-1-ylpenem-3-carboxylate (93) (0.22g) was converted to the title compound (94)(6mg); νₘₐₓ (KBr) 1758, 1616cm⁻¹; δ_{H} (D₂O) 4.10 (3H, s), 6.60 (1H, s), 7.0 (1H, bs), 7.30 (2H, bs), 7.9 (1H, bs), 8.15 (1H, s).

## Claims

1. A process for the preparation of a compound of the formula (X): 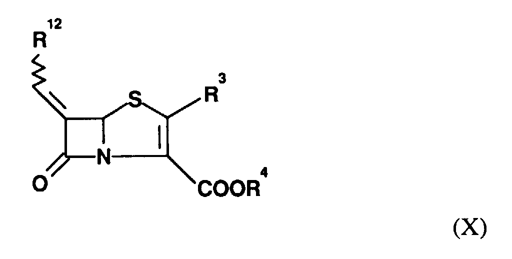 wherein:
R³ denotes a hydrogen atom or a group -R^{9A} or -SR^{9A} where R^{9A} denotes an unsubstituted or substituted (C₁₋₁₀) hydrocarbon or heterocyclyl group, or a group -OR^{9B} or -R^{9C} in which R^{9B} denotes an unsubstititued or substituted phenyl, naphthyl, thienyl, pyridyl, quinolyl or isoquinolyl, and R^{9C} denotes a nitrogen-containing heterocyclyl ring bonded through a ring-nitrogen atom;
R⁴ denotes a hydrogen atom, a carboxy-salt-forming ion, or a carboxy-ester-forming group;
R¹² denotes a hydrogen atom, an unsubstituted or substituted (C₁₋₁₀) hydrocarbon group, or an unsubstituted or substituted heterocyclyl group;
wherein the term "heterocyclyl" denotes an optionally substituted, 5 or 6 membered hetero-aromatic ring, or a fused bicyclic hetero-aromatic ring system having 5 or 6 atoms in each ring, or a non-aromatic heterocyclic ring having 3 to 7 ring atoms, and from 1 to 3 of the ring atoms being heteroatoms selected from nitrogen, oxygen and sulphur;
characterised in that (a) a penicillin or 6-aminopenicillanic acid is converted into a compound of formula (IC): 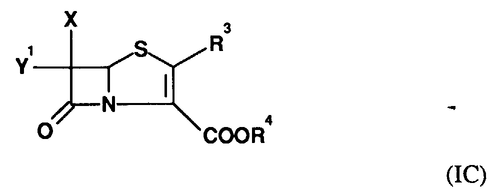 wherein R³ and R⁴ are as defined in formula (X), X denotes a halogen atom, and Y¹ denotes a hydrogen atom or a halogen atom, (b) the compound of formula (IC) is reacted with a compound of formula (XXV):
R¹² - CHO (XXV)
wherein R¹² is as defined above, to form a compound of formula (IB) 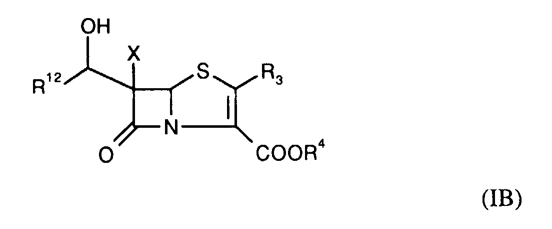 wherein R³, R⁴, R¹² and X are as defined above, and (c) the compound of formula (IB) is subjected to reductive elimination to eliminate X and hydroxy.

2. A process according to claim 1 characterised in that a 6-halo or 6, 6-dihalopenicillanic acid derivative of formula (IX): 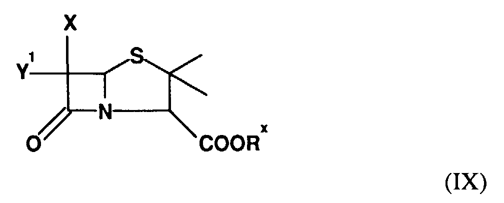 wherein X and Y¹ are as defined in claim 1, and R^{x} is a carboxy protecting group is converted into a compound of formula (IC), which is then converted into a compound of formula (X) as set out in claim 1.

3. A process according to claim 1 or 2 characterised in that X denotes a bromine atom and Y¹ denotes a hydrogen or bromine atom.

4. A process according to any one of the preceding claims characterised in that R³ is hydrogen.

5. A process according to any preceding claim characterised in that the reductive elimination reaction is effected by reaction of the compound of formula (IB) with a metal or triorganophosphorus compound.

6. A process according to any preceding claim characterised in that the reaction between compound (IC) and (XXV) is carried out in the presence of a base.

7. A process according to any one of the preceding claims characterised in that the compound (IC) has a stereochemistry:

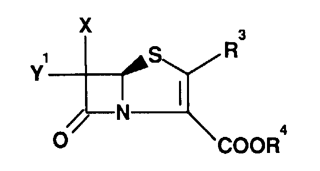

8. A compound of formula (IC), in which X and Y¹ are both bromine, R³ is hydrogen and R⁴ is hydrogen, ie 6, 6-dibromopenem-3-carboxylate, or a salt or ester thereof.

## Patentansprüche

1. Verfahren zur Herstellung einer Verbindung der Formel (X): 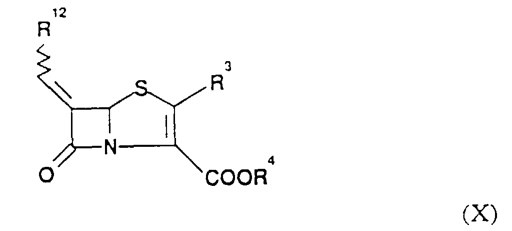
in der R³ ein Wasserstoffatom oder einen Rest -R^{9A} oder -SR^{9A}, wobei R^{9A} einen unsubstituierten oder substituierten (C₁₋₁₀)-Kohlenwasserstoff- oder Heterocyclenrest bezeichnet, oder einen Rest -OR^{9B} oder -R^{9C} bezeichnet, wobei R^{9B} eine unsubstituierte oder substituierte Phenyl-, Naphthyl-, Thienyl-, Pyridyl-, Chinolyl- oder Isochinolylgruppe bezeichnet und R^{9C} einen stickstoffhaltigen Heterocyclenring bezeichnet, der durch ein Ringstickstoffatom gebunden ist,
R⁴ ein Wasserstoffatom, ein ein Carboxysalz bildendes Ion oder einen einen Carboxyester bildenden Rest bezeichnet,
R¹² ein Wasserstoffatom, einen unsubstituierten oder substituierten (C₁₋₁₀)-Kohlenwasserstoffrest oder einen unsubstituierten oder substituierten Heterocyclenrest bezeichnet,
wobei der Begriff "Heterocyclenrest" einen gegebenenfalls substituierten, 5- oder 6-gliedrigen heteroaromatischen Ring oder ein kondensiertes bicyclisches hetero-aromatisches Ringsystem mit 5 oder 6 Atomen in jedem Ring oder einen nicht aromatischen heterocyclischen Ring mit 3 bis 7 Ringatomen bezeichnet und 1 bis 3 der Ringatome Heteroatome, ausgewählt aus Stickstoff, Sauerstoff und Schwefel sind,
dadurch gekennzeichnet, daß (a) ein Penicillin oder 6-Aminopenicillansäure in eine Verbindung der Formel (IC): 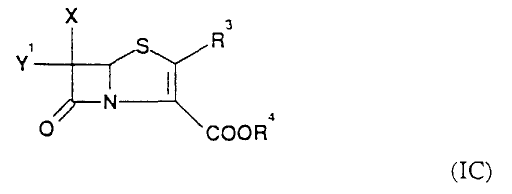 umgewandelt wird, in der R³ und R⁴ die in Formel (X) angegebene Bedeutung haben, X ein Halogenatom bezeichnet und Y¹ ein Wasserstoffatom oder ein Halogenatom bezeichnet, (b) die Verbindung der Formel (IC) mit einer Verbindung der Formel (XXV) umgesetzt wird:
R¹² - CHO (XXV)
in der R¹² die vorstehend angegebene Bedeutung hat, wobei eine Verbindung der Formel (IB) gebildet wird 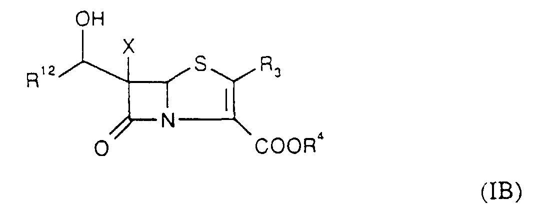 in der R³, R⁴, R¹² und X die vorstehend angegebene Bedeutung haben und (c) die Verbindung der Formel (IB) einer reduktiven Abspaltung unterzogen wird, um X und eine Hydroxylgruppe abzuspalten.

2. Verfahren nach Anspruch 1, dadurch gekennzeichnet, daß ein 6-Halogen- oder 6,6-Dihalogenpenicillansäurederivat der Formel (IX): 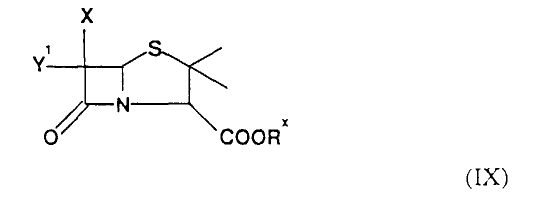 in der X und Y¹ die in Anspruch 1 angegebene Bedeutung haben und R^{x} eine Carboxylschutzgruppe ist, in eine Verbindung der Formel (IC) umgewandelt wird, die dann wie in Anspruch 1 dargestellt in eine Verbindung der Formel (X) umgewandelt wird.

3. Verfahren nach Anspruch 1 oder 2, dadurch gekennzeichnet, daß X ein Bromatom bezeichnet und Y¹ ein Wasserstoff- oder Bromatom bezeichnet.

4. Verfahren nach einem der vorstehenden Ansprüche, dadurch gekennzeichnet, daß R³ ein Wasserstoffatom ist.

5. Verfahren nach einem der vorstehenden Ansprüche, dadurch gekennzeichnet, daß die reduktive Abspaltungsreaktion unter Umsetzung der Verbindung der Formel (IB) mit einer Metall- oder Triorganophosphorverbindung durchgeführt wird.

6. Verfahren nach einem der vorstehenden Ansprüche, dadurch gekennzeichnet, daß die Reaktion zwischen der Verbindung (IC) und (XXV) in Gegenwart einer Base durchgeführt wird.

7. Verfahren nach einem der vorstehenden Ansprüche, dadurch gekennzeichnet, daß die Verbindung (IC) folgende Stereochemie aufweist:

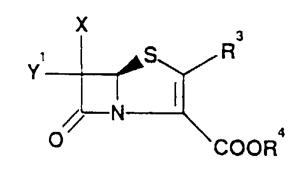

8. Verbindung der Formel (IC), in der X und Y¹ beide Bromatome sind, R³ ein Wasserstoffatom ist und R⁴ ein Wasserstoffatom ist, d.h. 6,6-Dibrompenem-3-carboxylat oder ein Salz oder Ester davon.

## Revendications

1. Procédé pour la préparation d'un composé de formule (X): 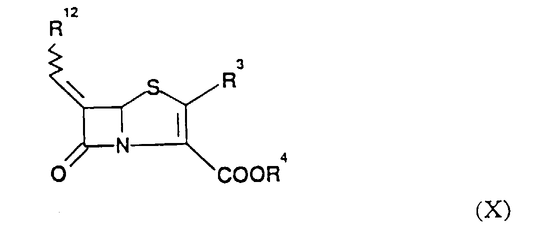 dans laquelle :
R³ représente un atome d'hydrogène ou un groupe -R^{9A} ou -SR^{9A} dans lequel R^{9A} représente un groupe hydrocarboné en C₁₋₁₀ ou hétérocyclyle non substitué ou substitué ou un groupe -OR^{9B} ou -R^{9C} dans lequel R^{9B} représente un groupe phényle, naphtyle, thiényle, pyridyle, quinolyle ou isoquinolyle non substitué ou substitué et R^{9C} représente un noyau hétérocyclyle contenant de l'azote, lié par l'intermédiaire d'un atome d'azote du noyau ;
R⁴ représente un atome d'hydrogène, un ion formant un sel de la fonction carboxy ou un groupe formant un ester de la fonction carboxy ;
R¹² représente un atome d'hydrogène, un groupe hydrocarboné en C₁₋₁₀ non substitué ou substitué ou un groupe hétérocyclyle non substitué ou substitué ;
où le terme "hétérocyclyle" représente un noyau hétéro aromatique à 5 ou 6 chaînons éventuellement substitué ou un système à noyau hétéro aromatique bicyclique condensé ayant 5 ou 6 atomes de carbone dans chaque noyau ou un noyau hétérocyclique non aromatique ayant 3 à 7 atomes de noyau, et de 1 à 3 des atomes du noyau étant des hétéroatomes choisis parmi les atomes d'azote, d'oxygène et de soufre ;
caractérisé en ce que (a) une pénicilline ou un acide 6-aminopénicillanique est converti en un groupe de formule (IC) : 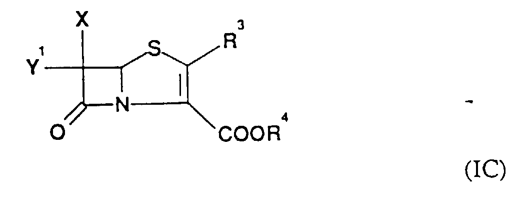 dans laquelle R³ et R⁴ sont tels que définis dans la formule (X), X représente un atome d'halogène et Y¹ représente un atome d'hydrogène ou un atome d'halogène, (b) le composé de formule (IC) est traité avec un composé de formule (XXV):
R¹²-CHO (XXV)
dans laquelle R¹² est tel que défini plus haut pour former un composé de formule (IB) : 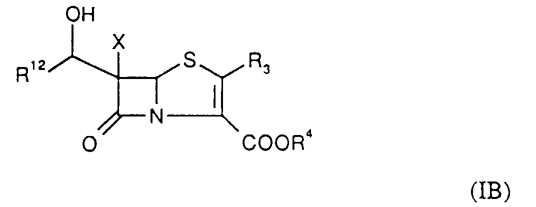 dans laquelle R³, R⁴, R¹² et X sont tels que défini plus haut, et (c) le composé de formule (IB) est soumis à une élimination réductrice pour éliminer X et le groupe hydroxy.

2. Procédé suivant la revendication 1, caractérisé en ce qu'un dérivé d'acide 6-halo ou 6,6-dihalopénicillanique de formule (IX) : 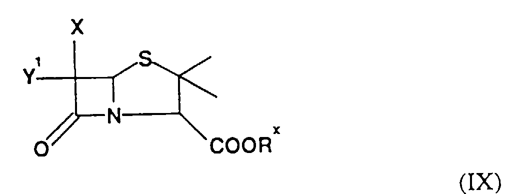 dans laquelle X et Y¹ sont tels que définis dans la revendication 1 et R^{x} est un groupe protégeant la fonction carboxy est converti en un composé de formule (IC), lequel est ensuite converti en un composé de formule (X) tel que défini dans la revendication 1.

3. Procédé suivant les revendications 1 et 2, caractérisé en ce que X représente un atome de brome et Y¹ représente un atome d'hydrogène ou de brome.

4. Procédé suivant l'une quelconque des revendications précédentes, caractérisé en ce que R³ est un atome d'hydrogène.

5. Procédé suivant l'une quelconque des revendications précédentes, caractérisé en ce que la réaction d'élimination réductrice est effectuée par réaction du composé de formule (IB) avec un métal ou un composé de triorganophosphore.

6. Procédé suivant l'une quelconque des revendications précédentes, caractérisé en ce que la réaction entre le composé (IC) et le composé (XXV) est conduite en présence d'une base.

7. Procédé suivant l'une quelconque des revendications précédentes, caractérisé en ce que le composé (IC) a une stéréochimie :

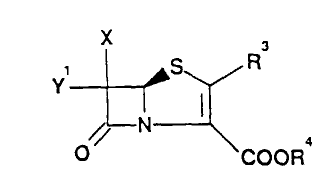

8. Composé de formule (IC), dans laquelle X et Y¹ sont tous deux un atome de brome, R³ est un atome d'hydrogène et R⁴ est un atome d'hydrogène, c'est-à-dire un 6,6-dibromopénèm-3-carboxylate ou un sel ou un ester de celui-ci.
